# EUROPEAN PATENT APPLICATION

(11) **EP 4 814 443 A1**
(43) Date of publication of application: **30.09.2026**
(21) Application number: 24896744.0
(22) Date of filing: 29.11.2024
(51) Int. Cl.: G01C 21/20, G01C 25/00, G01C 21/04

(54) **ROTATING MAGNET UNIT, FIELD EMITTER, AND CONTROL METHOD FOR FIELD EMITTER**

(30) Priority: 01.12.2023 CN 202311648640; 01.12.2023 CN 202311645150; 01.12.2023 CN 202311655768
(71) Applicant: Wuhan United Imaging Surgical Co., Ltd., Wuhan, Hubei 430073 (CN)
(72) Inventor: YANG, Daitianyi, Wuhan, Hubei 430073 (CN)
(74) Representative: Wang, Bo
(86) International application number: PCT/CN2024/135775
(87) International publication number: WO 2025/113664

(57) **Abstract**

An embodiment of this specification provides a rotating magnet unit, comprising: a drive assembly, a magnet, and a self-check assembly; the drive assembly is connected to the magnet and is used to drive the magnet to rotate; the self-check assembly is used to detect a magnetic field signal generated during the rotation of the magnet.

## Description

This application claims the priority to Chinese Patent Application No. 202311655768.8, titled "Field Emitter, and Its Self-Calibration Method, Interference Detection Method and Anomaly Detection Method," filed on December 01, 2023, and Chinese Patent Application No. 202311648640.9, titled "Rotating Magnet Unit and Its Self-Inspection Method, Field Emitter and Its Self-Inspection Method" filed on December 01, 2023, and Chinese Patent Application No. 202311645150.3 , titled "Field Emitter, and Its Drive Control Method, Apparatus and Storage Medium " filed on December 01, 2023, the entire contents of each of which are hereby incorporated by reference.

### TECHNICAL FIELD

The present disclosure relates to the field of electromagnetic navigation, and in particular, to rotating magnet unit, field emitter, control method for field emitter.

### BACKGROUND

An electro-magnetic transient simulator (EMTS) is one of the mainstream technical solutions for surgical navigation systems. The most typical basic principle of EMTS is to generate a time-varying magnetic field through a field emitter, detect the time-varying magnetic field through a magnetic sensor, and then perform pose calculation of the magnetic sensor. The field emitter is one of the core components in this system. There are two most common ways to generate a time-varying magnetic field: one is to use an electromagnetic coil to pass an alternating current to generate a time-varying magnetic field, and the other is to generate a time-varying magnetic field by rotating a magnet. Compared with electromagnetic coils, magnets of the same volume can generate a stronger magnetic field, have lower system power consumption, and do not suffer from heating issues, thus offering unique advantages.

### SUMMARY

One or more embodiments of this specification provide a rotating magnet unit, comprising: a drive assembly, a magnet, and a self-check assembly; the drive assembly is connected to the magnet and is used to drive the magnet to rotate; the self-check assembly is used to detect the magnetic field signal generated during the rotation of the magnet.

In some embodiments, the self-check assembly comprises three ring coils whose normal vectors are pairwise orthogonal; the three ring coils are respectively located on three sides of the magnet; the normal vector of one of the ring coils coincides with the rotation axis of the magnet.

In some embodiments, the self-check assembly comprises a magnetic sensor; the magnetic sensor is located on a side of the drive assembly away from the magnet.

In some embodiments, the rotation axis of the magnet: is not parallel to the magnetic moment direction of the magnet; or, is not parallel to the magnetic moment direction of the magnet and passes through the center of mass of the magnet; or, is perpendicular to the magnetic moment direction of the magnet; or, is perpendicular to the magnetic moment direction of the magnet and passes through the center of mass of the magnet.

In some embodiments, the drive assembly comprises a motor, a reduction mechanism, and an absolute position encoder; the output shaft of the motor is connected to the magnet via the reduction mechanism, and the absolute position encoder is used to collect the angular position information of the magnet.

In some embodiments, the drive assembly comprises a transmission device, and the motor drives the magnet to rotate through the transmission device.

In some embodiments, the rotating magnet unit further comprises: a mounting housing; the drive assembly, the magnet, and the self-check assembly are all installed inside the mounting housing; the mounting housing is provided with a functional interface, which connects the drive assembly and the self-check assembly.

In some embodiments, the rotating magnet unit further comprises: a locking assembly; the locking assembly is used to lock the angular position of the magnet.

One or more embodiments of this specification provide a field emitter, comprising at least one rotating magnet unit, at least one of the rotating magnet units being the rotating magnet unit according to any of the above embodiments.

One or more embodiments of this specification provide an electro-magnetic transient simulatorelectro-magnetic transient simulator, comprising a processor, a receiving device, and the field emitter according to any of the above embodiments, the receiving device being used to detect the time-varying magnetic field generated by the field emitter, the processor being used to control the operation of the field emitter and determine the real-time pose of the receiving device in the time-varying magnetic field based on the magnetic field detection data of the receiving device.

One or more embodiments of this specification provide a self-check method for a rotating magnet unit, the self-check method being applied to the rotating magnet unit according to any of the above embodiments; the self-check method for the rotating magnet unit comprises: controlling the drive assembly to drive the magnet to rotate at a preset rotational speed; acquiring a current time-varying magnetic field signal of the magnet; wherein the current time-varying magnetic field signal is obtained by the self-check assembly detecting the magnetic field signal generated when the magnet rotates; determining a signal difference between the current time-varying magnetic field signal of the magnet and a reference time-varying magnetic field signal, and determining a self-check result of the rotating magnet unit based on the signal difference.

In some embodiments, the reference time-varying magnetic field signal is obtained by the self-check assembly detecting the magnetic field signal generated when the magnet rotates at the preset rotational speed in an initial state of the rotating magnet unit.

In some embodiments, determining the signal difference between the current time-varying magnetic field signal of the magnet and the reference time-varying magnetic field signal, and determining the self-check result of the rotating magnet unit based on the signal difference comprises: determining a current magnetic field strength based on the current time-varying magnetic field signal, and a proportional relationship among components in the current time-varying magnetic field signal; determining a strength difference between the current magnetic field strength and a reference magnetic field strength, the reference magnetic field strength being determined based on the reference time-varying magnetic field signal; determining a proportional difference between the proportional relationship among components in the current time-varying magnetic field signal and the proportional relationship among components in the reference time-varying magnetic field signal; determining the self-check result of the rotating magnet unit based on the strength difference and the proportional difference.

One or more embodiments of this specification provide a self-check system for a rotating magnet unit, the system being applied to the rotating magnet unit according to any of the above embodiments; the system comprises: a drive module, used to control the drive assembly to drive the magnet to rotate at a preset rotational speed; a first signal acquisition module, used to acquire a current time-varying magnetic field signal of the magnet; wherein the current time-varying magnetic field signal is obtained by the self-check assembly detecting the magnetic field signal generated when the magnet rotates; a first self-check result determination module, used to determine a signal difference between the current time-varying magnetic field signal of the magnet and a reference time-varying magnetic field signal, and determine a self-check result of the rotating magnet unit based on the signal difference.

One or more embodiments of this specification provide a self-check device for a rotating magnet unit, comprising a processor, the processor being used to execute the self-check method for the rotating magnet unit according to any of the above embodiments.

One or more embodiments of this specification provide a computer-readable storage medium, the storage medium storing computer instructions, when a computer reads the computer instructions in the storage medium, the computer executes the self-check method for the rotating magnet unit according to any of the above embodiments.

One or more embodiments of this specification provide a self-check method for a field emitter, the self-check method being applied to the above field emitter; the self-check method for the field emitter comprises: determining a target rotating magnet unit to be self-checked in the field emitter, and locking the angular position of the magnet of a non-target rotating magnet unit; controlling the drive assembly to drive the magnet of the target rotating magnet unit to rotate at a preset rotational speed; acquiring a current time-varying magnetic field signal of the magnet; wherein the current time-varying magnetic field signal is obtained by the self-check assembly detecting the magnetic field signal generated when the magnet rotates; determining a signal difference between the current time-varying magnetic field signal of the magnet and a reference time-varying magnetic field signal, and determining a self-check result of the rotating magnet unit based on the signal difference.

One or more embodiments of this specification provide a self-check system for a field emitter, the system being applied to the above field emitter; the system comprises: a locking module, used to determine a target rotating magnet unit to be self-checked in the field emitter, and lock the angular position of the magnet of a non-target rotating magnet unit; a rotation control module, used to control the drive assembly to drive the magnet of the target rotating magnet unit to rotate at a preset rotational speed; a second signal acquisition module, used to acquire a current time-varying magnetic field signal of the magnet; wherein the current time-varying magnetic field signal is obtained by the self-check assembly detecting the magnetic field signal generated when the magnet rotates; a second self-check result determination module, used to determine a signal difference between the current time-varying magnetic field signal of the magnet and a reference time-varying magnetic field signal, and determine a self-check result of the rotating magnet unit based on the signal difference.

One or more embodiments of this specification provide a self-check device for a rotating magnet unit, comprising a processor, the processor being used to execute the self-check method for the rotating magnet unit as described above.

One or more embodiments of this specification provide a computer-readable storage medium, the storage medium storing computer instructions, when a computer reads the computer instructions in the storage medium, the computer executes the self-check method for the rotating magnet unit as described.

One or more embodiments of this specification provide an electromagnetic navigation method, the electromagnetic navigation method being applied to the above electro-magnetic transient simulatorelectro-magnetic transient simulator; the electromagnetic navigation method comprises: performing self-check on the field emitter to obtain a self-check result for each rotating magnet unit in the field emitter, comprising: determining a target rotating magnet unit to be self-checked in the field emitter, and locking the angular position of the magnet of a non-target rotating magnet unit; controlling the drive assembly to drive the magnet of the target rotating magnet unit to rotate at a preset rotational speed; acquiring a current time-varying magnetic field signal of the magnet; wherein the current time-varying magnetic field signal is obtained by the self-check assembly detecting the magnetic field signal generated when the magnet rotates; determining a signal difference between the current time-varying magnetic field signal of the magnet and a reference time-varying magnetic field signal, and determining a self-check result of the rotating magnet unit based on the signal difference; for any of the rotating magnet units, determining a target magnetic moment strength of the rotating magnet unit based on the self-check result of the rotating magnet unit.

In some embodiments, the electromagnetic navigation method further includes: acquiring magnetic field data of a time-varying magnetic field generated by the field emitter; wherein the magnetic field data of the time-varying magnetic field is obtained by the receiving device detecting the time-varying magnetic field; determining a real-time pose of the receiving device in the time-varying magnetic field based on the magnetic field data of the time-varying magnetic field and a time-varying characteristic of the time-varying magnetic field.

In some embodiments, the time-varying characteristic of the time-varying magnetic field includes a magnetic moment time-varying characteristic of each of the rotating magnet units in the field emitter in a field emitter coordinate system, and the magnetic moment time-varying characteristic includes a magnetic moment direction time-varying characteristic and a magnetic moment strength.

In some embodiments, determining the real-time pose of the receiving device in the time-varying magnetic field based on the magnetic field data of the time-varying magnetic field and the time-varying characteristic of the time-varying magnetic field includes: calculating the pose of the receiving device in the time-varying magnetic field in real time based on the magnetic field data of the time-varying magnetic field within a real-time time window and the time-varying characteristic of the time-varying magnetic field; wherein a window width of the real-time time window is determined based on a real-time requirement for calculating the pose of the receiving device.

In some embodiments, the method further includes: acquiring real-time detection results obtained by the self-checking components of each of the rotating magnet units detecting the time-varying magnetic field generated by the field emitter; determining a result difference between the real-time detection results of the self-checking components of each of the rotating magnet units and respective reference detection results; determining a real-time operating status of the field emitter based on the result difference.

One or more embodiments of this specification provide an electromagnetic navigation device, applied to the electro-magnetic transient simulatorelectro-magnetic transient simulator; the electromagnetic navigation device includes: a self-checking module, configured to: perform a self-check on the field emitter to obtain a self-check result for each rotating magnet unit in the field emitter, including: determining a target rotating magnet unit to be self-checked in the field emitter, and locking an angular position of a magnet of a non-target rotating magnet unit; controlling the drive assembly to drive the magnet of the target rotating magnet unit to rotate at a preset rotational speed; acquiring a current time-varying magnetic field signal of the magnet; wherein the current time-varying magnetic field signal is obtained by the self-checking component detecting a magnetic field signal generated by the magnet during rotation; determining a signal difference between the current time-varying magnetic field signal of the magnet and a reference time-varying magnetic field signal, and determining the self-check result of the rotating magnet unit based on the signal difference; a correction module, configured to: for any rotating magnet unit, determine a target magnetic moment strength of the rotating magnet unit based on the self-check result of the rotating magnet unit.

One or more embodiments of this specification provide an electromagnetic navigation device, including a processor configured to execute the electromagnetic navigation method according to any one of the above embodiments.

One or more embodiments of this specification provide a computer-readable storage medium, the storage medium storing computer instructions, and when a computer reads the computer instructions in the storage medium, the computer executes the electromagnetic navigation method according to any one of the above embodiments.

One or more embodiments of this specification provide a control method for a field emitter, applied to a field emitter, the field emitter including at least one rotating magnet unit, at least one of the rotating magnet units including a drive assembly and a magnet; the method includes: for each rotating magnet unit in the field emitter, determining an interaction torque on the magnet of the rotating magnet unit from magnets of other rotating magnet units; using the interaction torque on the magnet of the rotating magnet unit as a feedforward input for its own drive, determining a drive current corresponding to the rotating magnet unit; controlling the drive assembly to drive the rotating magnet unit to rotate based on the drive current, generating a time-varying magnetic field.

In some embodiments, determining the interaction torque on the magnet of the rotating magnet unit from magnets of other rotating magnet units includes: determining one of the rotating magnet units in the field emitter as a first target rotating magnet unit; determining a time-varying characteristic of a combined magnetic field at the magnet of the first target rotating magnet unit, the combined magnetic field being jointly generated by rotating magnet units in the field emitter other than the first target rotating magnet unit; determining a time-varying characteristic of a target magnetic moment of the magnet of the first target rotating magnet unit; determining a time-varying characteristic of the interaction torque on the magnet of the first target rotating magnet unit based on the time-varying characteristic of the combined magnetic field and the time-varying characteristic of the target magnetic moment.

One or more embodiments of this specification provide a control system for a field emitter, applied to a field emitter, the field emitter including at least one rotating magnet unit, at least one of the rotating magnet units including a drive assembly and a magnet; the system includes: a torque determination module, configured to: for each rotating magnet unit in the field emitter, determine an interaction torque on the magnet of the rotating magnet unit from magnets of other rotating magnet units; a torque processing module, configured to: use the interaction torque on the magnet of the rotating magnet unit as a feedforward input for its own drive, determine a drive current corresponding to the rotating magnet unit; control the drive assembly to drive the rotating magnet unit to rotate based on the drive current, generating a time-varying magnetic field.

One or more embodiments of this specification provide a control device for a field emitter, including a processor configured to execute the control method for a field emitter according to any one of the above embodiments.

One or more embodiments of this specification provide a computer-readable storage medium, the storage medium storing computer instructions, and when a computer reads the computer instructions in the storage medium, the computer executes the control method for a field emitter according to any one of the above embodiments.

One or more embodiments of this specification provide a control method for a field emitter, applied to a field emitter, the field emitter including at least one rotating magnet unit, at least one of the rotating magnet units including a drive assembly and a magnet; the method includes: controlling the magnets of the rotating magnet units having the same initial magnetic moment direction to generate different rotational speeds.

In some embodiments, the method further includes: controlling the magnets of the rotating magnet units having different initial magnetic moment directions to generate the same rotational speed.

One or more embodiments of this specification provide a control system for a field emitter, applied to a field emitter, the field emitter including at least one rotating magnet unit, at least one of the rotating magnet units including a drive assembly and a magnet; the system includes a rotational speed control module, configured to: control the magnets of the rotating magnet units having the same initial magnetic moment direction to generate different rotational speeds.

One or more embodiments of this specification provide a control device for a field emitter, including a processor configured to execute the control method for a field emitter according to any one of the above embodiments.

One or more embodiments of this specification provide a computer-readable storage medium, the storage medium storing computer instructions, and when a computer reads the computer instructions in the storage medium, the computer executes the control method for a field emitter according to any one of the above embodiments.

One or more embodiments of this specification provide a field emitter, the field emitter including: a field emitter unit group and a magnetic detection assembly; the field emitter unit group includes at least one rotating magnet unit; the magnetic detection assembly is configured to detect a calibration magnetic field generated by the rotating magnet unit.

In some embodiments, the magnetic detection assembly further includes a circuit board, and the magnetic detection assembly includes a plurality of magnetic sensors; the plurality of magnetic sensors are uniformly mounted on the circuit board.

In some embodiments, the field emitter further includes: a mounting housing; the field emitter unit group and the magnetic detection assembly are both mounted inside the mounting housing; or, the field emitter unit group is mounted inside the mounting housing, and the magnetic detection assembly is mounted outside the mounting housing.

In some embodiments, the field emitter further includes: a magnetic source component; a spatial pose of the magnetic source component in the field emitter is fixed; when the magnetic source component is operating, the magnetic detection assembly is further configured to detect a detection magnetic field generated by the magnetic source component.

In some embodiments, the magnetic source component includes a plurality of coils, and the plurality of coils are uniformly distributed on a periphery of the field emitter unit group.

One or more embodiments of this specification provide a self-calibration method for a field emitter, the self-calibration method being applied to the field emitter according to any one of the above embodiments; the self-calibration method includes: acquiring measured calibration magnetic field data obtained by the magnetic detection assembly detecting a target calibration magnetic field, wherein the target calibration magnetic field is a calibration magnetic field generated by a target rotating magnet unit, and the target rotating magnet unit is a rotating magnet unit to be calibrated in the field emitter unit group; acquiring magnet angle information of the target rotating magnet unit; determining a target calibration parameter of the target rotating magnet unit based on the measured calibration magnetic field data and the magnet angle information.

In some embodiments, determining the target calibration parameters of the target rotating magnet unit based on the measured working magnetic field data and the magnet angle information includes: determining model calibration magnetic field data of the target calibration magnetic field at the magnetic detection assembly based on the magnet angle information, model parameters of the target rotating magnet unit, and the spatial pose of the magnetic detection assembly; optimizing the model parameters of the target rotating magnet unit with the optimization objective of minimizing the difference between the measured calibration magnetic field data and the model calibration magnetic field data to obtain the target calibration parameters of the target rotating magnet unit.

In some embodiments, the optimization objective of minimizing the difference between the measured working magnetic field data and the model calibration magnetic field data includes: taking minimizing the difference between the modulus value of the measured calibration magnetic field data and the modulus value of the model calibration magnetic field data as the optimization objective.

In some embodiments, the measured calibration magnetic field data includes a sequence of measured calibration magnetic field values, and the model calibration magnetic field data includes a sequence of model calibration magnetic field values; the optimization objective of minimizing the difference between the measured calibration magnetic field data and the model calibration magnetic field data includes: determining the sequence mean of the measured calibration magnetic field value sequence and the sequence mean of the model calibration magnetic field value sequence; subtracting the sequence mean of the measured calibration magnetic field value sequence from each magnetic field value in the measured calibration magnetic field value sequence to obtain a first magnetic field value sequence; subtracting the sequence mean of the model calibration magnetic field value sequence from each magnetic field value in the model calibration magnetic field value sequence to obtain a second magnetic field value sequence; taking minimizing the difference between the first magnetic field value sequence and the second magnetic field value sequence as the optimization objective.

One or more embodiments of this specification provide a self-calibration system for a field emitter, the system being applied to the field emitter according to any of the preceding embodiments; the self-calibration system includes: a magnetic field data acquisition module, configured to acquire measured calibration magnetic field data obtained by the magnetic detection assembly detecting a target calibration magnetic field, wherein the target calibration magnetic field is a calibration magnetic field generated by a target rotating magnet unit, and the target rotating magnet unit is a rotating magnet unit to be calibrated in the field emitter unit group; an angle information acquisition module, configured to acquire magnet angle information of the target rotating magnet unit; a target calibration parameter determination module, configured to determine target calibration parameters of the target rotating magnet unit based on the measured calibration magnetic field data and the magnet angle information.

One or more embodiments of this specification provide a self-calibration device for a field emitter, comprising a processor configured to execute the self-calibration method for a field emitter according to any of the preceding embodiments.

One or more embodiments of this specification provide a computer-readable storage medium, the storage medium storing computer instructions, and when a computer reads the computer instructions from the storage medium, the computer executes the self-calibration method for a field emitter according to any of the preceding embodiments.

One or more embodiments of this specification provide a field emitter, the field emitter comprising: a field emitter unit group and a magnetic source component; the field emitter unit group includes at least one rotating magnet unit; the spatial pose of the magnetic source component within the field emitter is fixed; when the field emitter unit group stops working, the magnetic source component generates a detection magnetic field for performing interference detection on the field emitter.

In some embodiments, the magnetic source component includes a plurality of coils, the plurality of coils being uniformly distributed around the periphery of the field emitter unit group.

One or more embodiments of this specification provide an interference detection method for a field emitter, the interference detection method being applied to the field emitter according to any of the preceding embodiments; the interference detection method includes: controlling all rotating magnet units in the field emitter to stop rotating; controlling the magnetic source component to generate the detection magnetic field; acquiring measured detection magnetic field data obtained by the magnetic detection assembly detecting the detection magnetic field; determining a detection difference degree between the measured detection magnetic field data and reference detection magnetic field data; determining an interference detection result of the field emitter based on the detection difference degree.

In some embodiments, the magnetic source component includes a plurality of coils, the plurality of coils being uniformly distributed around the periphery of the field emitter unit group; the acquiring measured detection magnetic field data obtained by the magnetic sensor detecting a detection magnetic field includes: acquiring target measured detection magnetic field data obtained by the magnetic detection assembly detecting a target detection magnetic field; wherein the target detection magnetic field is a detection magnetic field generated by a target coil in the magnetic source component; the determining a detection difference degree between the measured detection magnetic field data and reference detection magnetic field data, and determining an interference detection result of the field emitter based on the detection difference degree, includes: determining a target detection difference degree between the target measured detection magnetic field data and target reference detection magnetic field data, and determining an interference detection result of the field emitter in a direction corresponding to the target coil based on the target detection difference degree.

One or more embodiments of this specification provide an interference detection system for a field emitter, applied to the field emitter according to any of the preceding embodiments; the interference detection system includes: a first control module, configured to control all rotating magnet units in the field emitter to stop rotating; a second control module, configured to control the magnetic source component to generate the detection magnetic field; a first acquisition module, configured to acquire measured detection magnetic field data obtained by the magnetic detection assembly detecting the detection magnetic field; a second acquisition module, configured to determine a detection difference degree between the measured detection magnetic field data and reference detection magnetic field data; a first determination module, configured to determine an interference detection result of the field emitter based on the detection difference degree.

One or more embodiments of this specification provide an interference detection device for a field emitter, comprising a processor configured to execute the interference detection method for a field emitter according to any of the preceding embodiments.

One or more embodiments of this specification provide a computer-readable storage medium, the storage medium storing computer instructions, and when a computer reads the computer instructions from the storage medium, the computer executes the interference detection method for a field emitter according to any of the preceding embodiments.

One or more embodiments of this specification provide an anomaly detection method for a field emitter, the anomaly detection method being applied to the field emitter according to any of the preceding embodiments; the anomaly detection method includes: acquiring measured working magnetic field data obtained by the magnetic detection assembly detecting a working magnetic field generated by the field emitter; determining a working difference degree between the measured working magnetic field data and reference working magnetic field data, and determining an operating state of the field emitter based on the working difference degree.

In some embodiments, the field emitter further includes: a magnetic source component; the determining an operating state of the field emitter based on the working difference degree includes: in response to determining that the working difference degree is less than or equal to a working difference degree threshold, determining that the field emitter is in a normal operating state; in response to determining that the working difference degree is greater than the working difference degree threshold, controlling all rotating magnet units in the field emitter to stop rotating; controlling the magnetic source component to generate a detection magnetic field; acquiring measured detection magnetic field data obtained by the magnetic detection assembly detecting the detection magnetic field; determining a detection difference degree between the measured detection magnetic field data and reference detection magnetic field data; determining an interference detection result of the field emitter based on the detection difference degree.

In some embodiments, the anomaly detection method further includes: after performing the interference detection on the field emitter, in response to determining that the detection difference degree is greater than a detection difference degree threshold, determining that the field emitter is in an abnormal operating state; in response to determining that the detection difference degree is less than or equal to the detection difference degree threshold, acquiring measured calibration magnetic field data obtained by the magnetic detection assembly detecting a target calibration magnetic field, wherein the target calibration magnetic field is a calibration magnetic field generated by a target rotating magnet unit, and the target rotating magnet unit is a rotating magnet unit to be calibrated in the field emitter unit group; acquiring magnet angle information of the target rotating magnet unit; determining target calibration parameters of the target rotating magnet unit based on the measured calibration magnetic field data and the magnet angle information.

In some embodiments, the method further includes: after performing the self-calibration on the field emitter, determining a calibration difference degree between the target calibration parameters and initial calibration parameters; in response to determining that the calibration difference degree is greater than a calibration difference degree threshold, determining that the field emitter is in an abnormal operating state; in response to determining that the calibration difference degree is less than or equal to the calibration difference degree threshold, determining that the field emitter is in a normal operating state; updating the target parameters of the field emitter to the target calibration parameters.

One or more embodiments of this specification provide an electro-magnetic transient simulator, the electro-magnetic transient simulator comprising a processor, a receiving device, and the field emitter according to any of the preceding embodiments, the receiving device being configured to detect a time-varying magnetic field generated by the field emitter, the processor being configured to control the operation of the field emitter and determine a real-time pose of the receiving device in the time-varying magnetic field based on magnetic field detection data from the receiving device.

One or more embodiments of this specification provide an anomaly detection system for a field emitter, applied to the field emitter according to any of the preceding embodiments; the anomaly detection system includes:

a second magnetic field data acquisition module, configured to acquire measured working magnetic field data obtained by the magnetic detection assembly detecting a working magnetic field generated by the field emitter;

An operating state determination module, configured to determine a working difference between the measured working magnetic field data and the reference working magnetic field data, and determine the operating state of the field emitter based on the working difference.

One or more embodiments of this specification provide an abnormality detection device for a field emitter, comprising a processor configured to execute the abnormality detection method for a field emitter according to any of the above embodiments.

One or more embodiments of this specification provide a computer-readable storage medium storing computer instructions, wherein when a computer reads the computer instructions from the storage medium, the computer executes the abnormality detection method for a field emitter according to any of the above embodiments.

One or more embodiments of this specification provide a field emitter, the field emitter comprising at least one rotating magnet unit, for each of the rotating magnet units, a reference axis is defined in the rotating magnet unit, the rotating magnet unit comprises a magnet capable of rotating about the reference axis, and a magnetic moment direction of the magnet is not parallel to the reference axis. When there are a plurality of the rotating magnet units, the reference axes of at least two of the rotating magnet units among the plurality of rotating magnet units are not parallel.

In some embodiments, at least one of the rotating magnet units comprises a self-checking component for detecting a magnetic field signal generated during the rotation of the magnet.

In some embodiments, the plurality of rotating magnet units comprises a first rotating magnet unit and a second rotating magnet unit; the first rotating magnet unit comprises a first motor and a first magnet, the second rotating magnet unit comprises a second motor and a second magnet, the first motor is used to drive the first magnet to rotate, and the second motor is used to drive the second magnet to rotate; or, the first magnet unit comprises a first motor and a first magnet, the second magnet unit comprises a first transmission assembly and a second magnet, the first motor is used to drive the first magnet to rotate, and the first motor is also used to drive the second magnet to rotate by driving the first transmission assembly.

In some embodiments, for each of the rotating magnet units, a reference axis is defined in the rotating magnet unit, the rotating magnet unit comprises a magnet capable of rotating about the reference axis, and a magnetic moment direction of the magnet is perpendicular to the reference axis; wherein: the reference axes of at least two of the rotating magnet units among the plurality of rotating magnet units are perpendicular to each other; or, the number of the plurality of rotating magnet units is four, the reference axes of the four rotating magnet units are in the same plane, and the reference axes of any two adjacent rotating magnet units among the four rotating magnet units are perpendicular to each other; or, the number of the plurality of rotating magnet units is three, and the reference axes of the three rotating magnet units are perpendicular to each other pairwise.

In some embodiments, the magnets are installed at ends of any two of the rotating magnet units among the plurality of rotating magnet units that are far away from each other.

In some embodiments, the field emitter further comprises a mounting body, the mounting body is provided with a plurality of mounting positions, and the plurality of mounting positions are used for installing at least one of the rotating magnet units.

This specification provides a field emitter, the field emitter requires at least only two rotating magnet units to be constructed, and the magnetic moment combination of the magnets of the two rotating magnet units can sweep through three orthogonal spatial directions, meeting the usage requirements of the field emitter. Therefore, this specification provides a simple and effective field emitter configuration, which requires at least only two rotating magnet units to build a field emitter that meets usage requirements, solving the problem that current field emitters lack simple and effective specific configurations, causing technicians to need to build based on their own experience, resulting in complex field emitter configurations that do not meet usage standards.

This specification provides a control method for a field emitter. When the field emitter is working, each rotating magnet unit works simultaneously, and each generates a single time-varying magnetic field. The single time-varying magnetic field generated by each rotating magnet unit will affect other rotating magnet units. Therefore, the driving load of each rotating magnet unit when working simultaneously is different from its driving load when working alone. In order to ensure that the magnet of each rotating magnet unit can stably rotate at a preset speed when the field emitter is working, the driving current of the rotating magnet unit needs to be modulated. In this specification, when the field emitter is working, the interaction torque on the magnet of each rotating magnet unit from the magnets of other rotating magnet units is calculated. For each rotating magnet unit, the interaction torque on its magnet is used as a feedforward input for its own drive assembly, and then the driving current can be modulated according to the time-varying characteristics of the interaction torque. For example, when the interaction torque is large, generating significant resistance to the rotation of the magnet, the current intensity of the driving current can be increased. Therefore, through the driving control method of the field emitter provided in this specification, each rotating magnet unit in the field emitter can adjust the driving current in a timely manner according to the interaction torque, thereby significantly improving the rotation control accuracy of the magnet and making the motor in the drive assembly operate more stably. This solves the problem of low rotation control accuracy of the magnet by each rotating magnet unit in existing field emitters.

This specification provides a control method for a field emitter. Controlling the magnets of rotating magnet units with different initial magnetic moment directions to produce the same rotational speed, and controlling the magnets of rotating magnet units with the same initial magnetic moment direction to produce different rotational speeds, can facilitate data decoupling, maintain positioning accuracy, and reduce the complexity of speed control.

This specification provides a rotating magnet unit and a self-check method for the rotating magnet unit. The rotating magnet unit includes a self-checking component. Before the rotating magnet unit operates, the self-checking component can detect the magnetic field signal generated by the magnet, and by comparing the current time-varying magnetic field signal of the magnet with a reference time-varying magnetic field signal, it can be determined whether the rotating magnet unit has an operational abnormality. Self-checking can be performed by the self-checking component before each use, promptly identifying its own problems through self-checking, avoiding the field emitter from generating a time-varying magnetic field that deviates from the standard during operation, and ultimately ensuring the positioning accuracy of the magnetic sensor.

This specification provides a field emitter and a self-calibration method for the field emitter. The field emitter includes a magnetic detection assembly. By providing a magnetic detection assembly in the field emitter, based on the magnetic field detection data of the magnetic detection assembly, the calibration parameters of each rotating magnet unit can be updated in the magnetic field model. The updated calibration parameters are more consistent with the actual state of the rotating magnet unit, thereby making the calculation results of the magnetic field model more accurate, which can improve the accuracy of electromagnetic navigation. The magnetic detection assembly can also detect the real-time working magnetic field generated by the field emitter during operation. By comparing the detected current time-varying magnetic field signal with a reference time-varying magnetic field signal, it can be determined whether the field emitter has an operational abnormality, so that abnormalities can be promptly discovered during the use of the field generator.

This specification provides a field emitter and an interference detection method for the field emitter. The field emitter includes a magnetic source component. Obtain measured detection magnetic field data obtained by a detection component detecting a detection magnetic field generated by the magnetic source component, determine a detection difference between the measured detection magnetic field data and reference detection magnetic field data, and determine an interference detection result of the field emitter based on the detection difference, so as to detect whether there is an interfering magnetic field around the field emitter, thereby promptly eliminating the interfering magnetic field and avoiding affecting electromagnetic navigation.

### BRIEF DESCRIPTION OF THE DRAWINGS

The present disclosure will be further illustrated by way of exemplary embodiments, which will be described in detail through the accompanying drawings. These embodiments are not limiting, and in these embodiments the same numbering indicates the same structure, wherein:
FIG. 1 is a schematic diagram of an application scenario of an electro-magnetic transient simulator according to some embodiments of this specification;
FIG. 2 is an exemplary top view of the structure of a field emitter according to some embodiments of this specification;
FIG. 3 is an exemplary side view of the structure of a field emitter according to some embodiments of this specification;
FIG. 4 is an exemplary top view of the structure of a field emitter according to other embodiments of this specification;
FIG. 5 is an exemplary structural side view of a field emitter according to other embodiments of the present description;
FIG. 6 is an exemplary structural top view of a magnetic detection assembly according to some embodiments of the present description;
FIG. 7 is an exemplary structural side view of a magnetic detection assembly according to some embodiments of the present description;
FIGS. 8 and 9 are exemplary structural diagrams of a rotating magnet unit according to some embodiments of the present description;
FIG. 10 is an exemplary flowchart of a control method for a field emitter according to some embodiments of the present description;
FIG.11 is an exemplary flowchart for determining time-varying characteristics according to some embodiments of the present description;
FIG. 12 is an exemplary flowchart of a control method for a field emitter according to some embodiments of the present description;
FIG. 13 is an exemplary flowchart of a self-check method for a rotating magnet unit according to some embodiments of the present description;
FIG. 14 is an exemplary flowchart for determining a self-check result according to some embodiments of the present description;
FIG. 15 is an exemplary flowchart of a self-check method for a field emitter according to other embodiments of the present description;
FIG. 16 is an exemplary flowchart for determining a target magnetic moment strength according to some embodiments of the present description;
FIG. 17 is an exemplary flowchart for determining a real-time operating status of a field emitter according to some embodiments of the present description;
FIG. 18 is an exemplary flowchart of a self-calibration method for a field emitter according to some embodiments of the present description;
FIG. 19 is an exemplary flowchart for determining an optimization target according to some embodiments of the present description;
FIG. 20 is an exemplary flowchart of an interference detection method for a field emitter according to some embodiments of the present description;
FIG. 21 is an exemplary flowchart of an anomaly detection method according to some embodiments of the present description;
FIG. 22 is an exemplary flowchart of an anomaly detection method according to other embodiments of the present description;
FIG. 23 is an exemplary flowchart of an anomaly detection method according to other embodiments of the present description;
FIG. 24 is an exemplary flowchart of an anomaly detection method according to other embodiments of the present description;
FIG. 25 is an exemplary flowchart of a method for using a field emitter according to some embodiments of the present description;
FIG. 26 is an exemplary flowchart of real-time positioning of a field emitter according to some embodiments of the present description;
FIG. 27 is an exemplary flowchart of a self-calibration method for a field emitter according to other embodiments of the present description;
FIG. 28 is an exemplary flowchart of an interference detection method for a field emitter according to other embodiments of the present description;
FIGS. 29 and 30 are exemplary schematic diagrams of another field emitter structure according to some embodiments of the present description;
FIG. 31 is a schematic diagram of a configuration of a field emitter according to some embodiments of the present description;
FIG. 32 is a schematic diagram of interaction torques of each rotating magnet unit according to some embodiments of the present description;
FIG. 33 is a schematic diagram of data acquisition during real-time positioning of a field emitter in the present description;
FIG. 34 is a structural schematic diagram of a rotating magnet unit according to some embodiments of the present description;
FIG. 35 is an exemplary module diagram of a self-check system for a rotating magnet unit according to some embodiments of the present description;
FIG. 36 is an exemplary module diagram of a self-check system for a field emitter according to some embodiments of the present description;
FIG. 37 is an exemplary module diagram of an electromagnetic navigation device according to some embodiments of the present description;
FIG. 38 is an exemplary module diagram of a control system for a field emitter according to some embodiments of the present description;
FIG. 39 is an exemplary module diagram of a control system for a field emitter according to some embodiments of the present description;
FIG. 40 is an exemplary module diagram of a self-calibration system for a field emitter according to some embodiments of the present description;
FIG. 41 is an exemplary module diagram of an interference detection system for a field emitter according to some embodiments of the present description;
FIG. 42 is an exemplary module diagram of an anomaly detection system for a field emitter according to some embodiments of the present description.

### DETAILED DESCRIPTION

To more clearly illustrate the technical solutions of the embodiments of the present description, the following briefly introduces the accompanying drawings required for describing the embodiments. Obviously, the drawings in the following description are only some examples or embodiments of the present description, and for those of ordinary skill in the art, the present description can be applied to other similar scenarios based on these drawings without creative effort. Unless otherwise apparent from the language context or otherwise stated, the same reference numerals in the figures represent the same structures or operations.

It should be understood that the terms "system," "apparatus," "unit," and/or "module" used herein are a method for distinguishing different levels of different components, elements, parts, sections, or assemblies. However, if other words can achieve the same purpose, the said words may be replaced by other expressions.

As shown in this description and the claims, unless the context clearly indicates an exception, words such as "a," "an," "one," and/or "the" are not specifically singular and may also include the plural. Generally speaking, the terms "include" and "comprise" only indicate the inclusion of explicitly identified steps and elements, which do not constitute an exclusive list, and the method or device may also include other steps or elements.

Flowcharts are used in this description to illustrate the operations performed by the system according to the embodiments of the present description. It should be understood that the preceding or following operations are not necessarily performed in exact order. Conversely, the steps may be processed in reverse order or simultaneously. Meanwhile, other operations may be added to these processes, or one or more steps may be removed from these processes.

Current field emitters lack a simple and effective specific configuration. A field emitter typically includes at least two rotating magnet units, each rotating magnet unit including a rotatable magnet. During use, the magnet may experience magnetic strength attenuation, or the rotating magnet unit may become loose or deformed, causing the magnetic moment direction of the magnet to change. When the above problems occur in the rotating magnet unit, the field emitter will generate a time-varying magnetic field that deviates from the standard during operation, ultimately reducing the positioning accuracy of the magnetic sensor.

On the other hand, when solving the pose of a magnetic sensor, the accuracy of the magnetic field model is a key prerequisite for obtaining high-precision solution results. Inevitable errors exist in any processing and installation, as well as in electronic control, such as the control of physical quantities like current intensity and frequency. Therefore, before performing high-precision navigation applications, the field emitter needs to be calibrated in detail and with high precision to obtain an accurate magnetic field model. However, even after detailed factory calibration, problems such as collision, thermal expansion and contraction, mechanical fatigue, and equipment aging inevitably occur during use, leading to a mismatch between the initial calibration parameters and the actual state of the field emitter, thereby reducing the accuracy of electromagnetic navigation. In view of the above problems, no effective solution has been proposed yet.

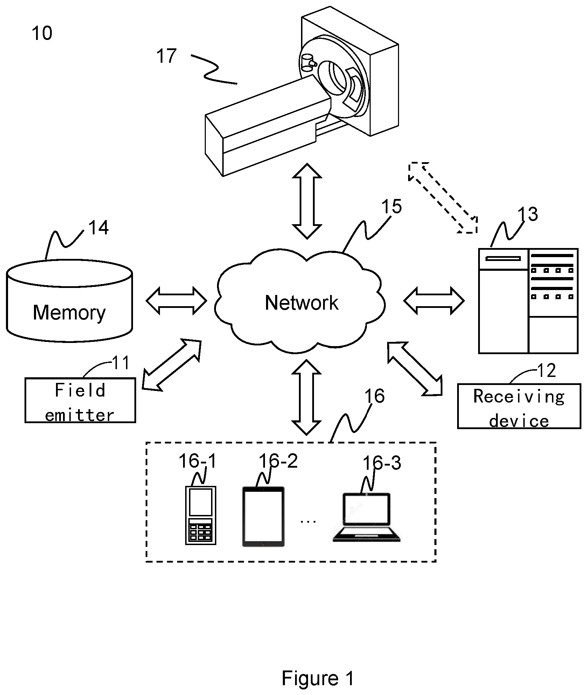

FIG. 1 is a schematic diagram of an application scenario of an electro-magnetic transient simulator according to some embodiments of the present description.

As shown in FIG. 1, the application scenario 10 of the electro-magnetic transient simulator may include a field emitter 11, a receiving device (receiver) 12, a processor 13, and a memory 14.

The field emitter 11 is one of the core components in the electro-magnetic transient simulator. The field generator 11 can be used to generate a precise and stable magnetic field. Through this magnetic field, a tracker (e.g., a small coil or sensor) inside or outside the target object senses the magnetic field. By analyzing the magnetic field signal sensed by the tracker, the position and orientation of the tracker in three-dimensional space can be determined. This positioning and tracking can be used in medical scenarios such as minimally invasive surgery and catheter insertion, and can also be applied in other scenarios. In some embodiments, the field generator 11 can be installed in an operating room, for example, located beside the operating bed.

The receiving device 12 can be used to receive the magnetic field signal emitted by the field generator 11. The receiving device 12 may include a magnetic sensor or an induction coil. In some embodiments, the receiving device 12 can be installed in a surgical device, for example, it can be installed in a surgical instrument (such as a probe or catheter).

The processor 13 can process data and/or information obtained from the field generator 11, the receiving device 12, the memory 14, or other components of the application scenario 10 of the electro-magnetic transient simulator. For example, the processor 13 can analyze and process the time-varying magnetic field generated by the field generator 11. In some embodiments, the processor 13 can be local or remote. For example, the processor 13 can be integrated into the receiving device or the emitter, or exist as an independent unit. When existing as an independent unit, the processor 13 can access information and/or data from the field generator 11, the receiving device 12, and/or the storage device 14 via a network. Exemplarily, the processor 13 can process the magnetic field signal received from the receiving device 12 in real-time to calculate the precise position of the receiving device 12 in three-dimensional space and convert it into an image coordinate system. In some embodiments, the processor 13 can be one or more (only one is shown in Figure 1). The processor 13 may include, but is not limited to, a microprocessor MCU or a programmable logic device FPGA, etc.

In a relatively typical application scenario, the field generator 11 emits a magnetic field signal and establishes a connection with the receiving device 12 through the processor 13. The receiving device can be installed on a surgical instrument or probe. When a doctor brings the surgical instrument close to or into the patient's body, the receiving device 12 can receive the electromagnetic waves emitted by the field generator and return the received magnetic field signal to the processor 13. The processor 13 processes the magnetic field signal received from the receiving device 12, for example, it can calculate the position of the receiving device 12 in real-time based on changes in the magnetic field signal and convert this position data into a position in the image coordinate system. Afterwards, the real-time position of the receiving device can be displayed via a display. In some embodiments, through integration with an imaging system, the spatial position of the receiving device 12 can be superimposed on the patient's real-time scan image. With the real-time image provided by the navigation system and the position information of the receiving device 12, the doctor can perform the surgical operation more precisely. For example, in brain surgery, a doctor can precisely guide a surgical instrument to a brain tumor site while avoiding damage to surrounding healthy brain tissue. In spinal surgery, electromagnetic navigation can help doctors perform precise screw implantation, ensuring the accuracy of screw placement and reducing surgical risks. In some embodiments, the real-time position of the receiving device can also be displayed without superimposing it on the patient's real-time scan image.

The memory 14 can be used to store data. The memory 14 can store calculation programs, data and/or information generated by other components of the application scenario 10 of the electro-magnetic transient simulator, etc. For example, software programs and modules of application software, such as the computer program corresponding to the drive control method of the field generator provided in the present invention. The processor 13 can execute various functional applications and data processing by running the computer program stored in the memory 14, thereby implementing the above-mentioned method. The memory 14 may include high-speed random access memory and may also include non-volatile memory, such as one or more magnetic storage devices, flash memory, or other non-volatile solid-state memory. In some instances, the memory 14 may further include memory remotely located relative to the processor 13, and these remote memories can be connected to the terminal via a network. Examples of the aforementioned network include, but are not limited to, the Internet, intranets, local area networks, mobile communication networks, and combinations thereof.

In some embodiments, the application scenario 10 of the electro-magnetic transient simulator may also include a terminal device 16. The terminal device 16 can enable the input of user commands and operations, or the output of the positioning of the tracker (receiving device 12). In some embodiments, a user can input a control request through the terminal device 16. In the embodiments of this specification, the terminal device 16 may include a mobile device 16-1, a tablet computer 16-2, a laptop computer 16-3, a display device, etc., or any combination thereof. In this specification, the user can be an operator of the medical device. For example, doctors, researchers, engineers, etc.

In some embodiments, the application scenario 10 of the electro-magnetic transient simulator may also include an imaging device 17. The imaging device 17 can be used to provide real-time anatomical images. The position of the receiving device 12 can be superimposed on the image and displayed via a display. The imaging device 17 can be a single-modality or multi-modality imaging device. For example, single-modality imaging devices can include CT devices, MRI devices, X-ray devices, PET devices, etc., and multi-modality imaging devices can include CT-MRI devices, CT-PET devices, etc. The imaging device 17 can also be omitted in the application scenario 10 of the electro-magnetic transient simulator.

In some embodiments, the application scenario 10 of the electro-magnetic transient simulator may also include a network 15. The network 15 may include any suitable network capable of facilitating the exchange of information and/or data. In some embodiments, at least one component of the application scenario 10 of the electro-magnetic transient simulator (e.g., field generator 11, receiving device 12, processor 13, terminal device 16, memory 14, imaging device 17, etc.) can exchange information and/or data with at least one other component of the system's application scenario 10 via the network 15.

It should be noted that the application scenario 10 of the electro-magnetic transient simulator is provided for illustrative purposes only and is not intended to limit the scope of this specification. For those of ordinary skill in the art, various modifications or changes can be made based on the description of this specification. For example, the application scenario 10 of the electro-magnetic transient simulator may also include a database. For another example, the application scenario 10 of the electro-magnetic transient simulator can implement similar or different functions on other devices. However, these changes and modifications will not depart from the scope of this specification.

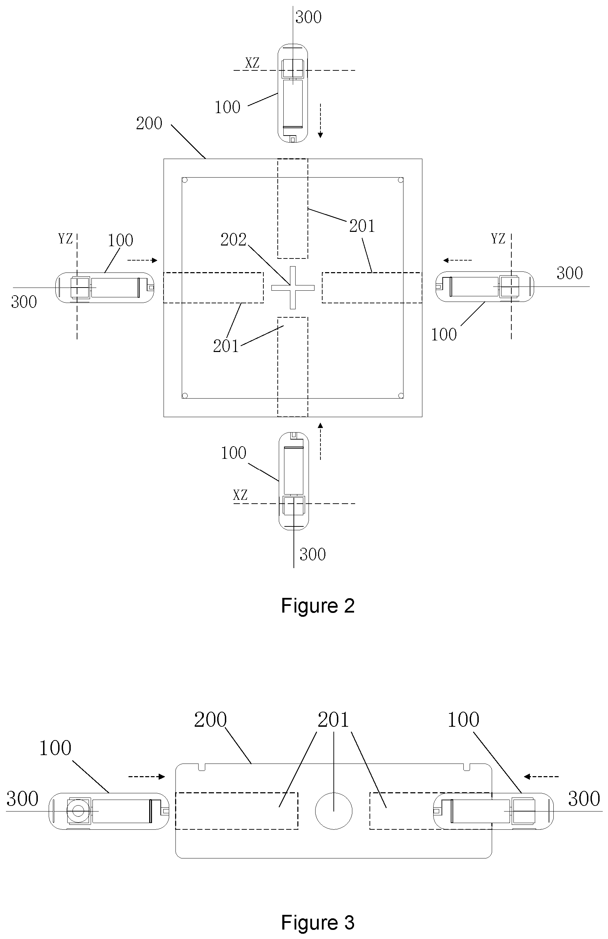

FIG. 2 is a top view of an exemplary structure of a field generator according to some embodiments of this specification, and FIG. 3 is a side view of an exemplary structure of a field generator according to some embodiments of this specification.

In some embodiments, the field generator includes at least one rotating magnet unit 100. For each rotating magnet unit 100, a reference axis 300 is defined in the rotating magnet unit. The rotating magnet unit includes a magnet capable of rotating about the reference axis 300, and the magnetic moment direction of the magnet is not parallel to the reference axis. Wherein, when there are multiple rotating magnet units, the reference axes of at least two of the multiple rotating magnet units are not parallel.

The rotating magnet unit is one of the basic components of the field generator. The rotating magnet unit has a magnet capable of rotating. The magnet can be a permanent magnet, and the material of the permanent magnet can be NdFeB; it can also be an electromagnet, such as an electromagnetic coil. The rotating magnet can provide a single time-varying magnetic field, and the reference axis of the rotating magnet unit is the rotation axis of its own magnet. The reference axis of the magnet can be perpendicular to the direction of the magnetic moment of the magnet. When the magnet rotates, its magnetic moment can sweep through two spatial directions, thereby defining a magnetic moment rotation plane. Furthermore, the reference axes of at least two rotating magnet units are perpendicular to each other, and the magnetic moment rotation planes of these two rotating magnet units are perpendicular, so that the combined magnetic moments of multiple magnets in the field emitter can sweep through three orthogonal spatial directions, meeting the usage requirements of the field emitter.

In some embodiments, the relationship between the rotation axis of the magnet and the direction of the magnetic moment of the magnet can be various. For example, the rotation axis of the magnet may not be parallel to the direction of the magnetic moment of the magnet; or, the rotation axis of the magnet may not be parallel to the direction of the magnetic moment of the magnet and passes through the center of mass of the magnet; or, the rotation axis of the magnet may be perpendicular to the direction of the magnetic moment of the magnet; or, the rotation axis of the magnet may be perpendicular to the direction of the magnetic moment of the magnet and passes through the center of mass of the magnet. The center of mass of the magnet can be located on the motor shaft. When the center of mass of the magnet is located on the motor reference axis, the mechanical structure stability of the entire unit is higher. When the center of mass of the magnet is not located on the motor shaft, the center of mass of the magnet deviates from the motor shaft. In this case, the rotation axis of the magnet is not parallel to the direction of the magnetic moment of the magnet.

The above embodiments illustrate various relationships between the rotation axis of the magnet and the direction of the magnetic moment of the magnet. In practice, preferably, the rotation axis (reference axis) of the magnet is perpendicular to the direction of the magnetic moment. The direction of the magnetic moment of the magnet is from the south pole (S) to the north pole (N) of the magnet. When the reference axis of the magnet is perpendicular to the direction of the magnetic moment, the direction of the magnetic moment of the magnet can be made to change within a fixed plane, facilitating data processing during subsequent electromagnetic navigation.

In some embodiments, the field emitter may include only a single rotating magnet unit. The magnet in this rotating magnet unit can undergo both revolution and rotation simultaneously, thereby generating a time-varying magnetic field. For example, the simultaneous revolution and rotation of the magnet can be achieved through a motor and a transmission assembly. The transmission assembly can be a gear set, such as a helical gear set, a bevel gear set, etc. The revolution axis and the rotation axis can intersect, for example, at a point deviating from the center of mass of the magnet. The revolution and rotation axes can also be non-coplanar, which is not limited in this embodiment. In some embodiments, the field emitter can be composed of at least two rotating magnet units, and the combined magnetic moments of the magnets of the two rotating magnet units can sweep through three orthogonal spatial directions, which is sufficient to meet the usage requirements of the field emitter.

The three orthogonal spatial directions that the magnetic moments of the rotating magnet units can sweep after combination should be as balanced as possible, meaning the number of magnetic moments sweeping through the three orthogonal spatial directions should be as close as possible or even equal. For example, if the three orthogonal spatial directions are divided into the X direction, Y direction, and Z direction, then the number of magnetic moments sweeping through the X direction, Y direction, and Z direction should be close or equal. Thus, the various magnetic moment directions can be combined to form multiple sets of "X-Y-Z orthogonal pairs," meaning that from the time series of magnetic moment directions, at least one magnetic moment combination pointing in the X direction, Y direction, and Z direction can always be formed. At each time point during the rotation process, the magnetic moments corresponding to the multiple rotating magnet units can cover the X, Y, and Z directions (that is, the magnetic moments do not need to fall exactly on the X, Y, and Z directions; the directions of the magnetic moments can also be other directions, as long as their components in the X, Y, and Z directions can cover the X, Y, and Z directions). As shown in Figure 2, an orthogonal coordinate system can be established by taking the direction of the reference axis of one of the magnet rotation units in the field emitter as one of the X direction, Y direction, and Z direction.

In some embodiments, each rotating magnet unit in the field emitter can adopt different rotational speeds, such as 10Hz, 20Hz, 30Hz, and 40Hz, respectively, which can also be adjusted according to actual usage scenarios and requirements. This ensures that the differences in the time-varying magnetic field over time and space meet the usage requirements.

In some embodiments, the end of any two of the multiple rotating magnet units that are far away from each other is the end where the magnet is installed.

The field emitter is composed of a combination of multiple rotating magnet units. The configuration of the field emitter has a certain influence on electromagnetic navigation. A rotatable magnet is installed inside the rotating magnet unit. For each rotating magnet unit, the distance between the internal magnets should be made as large as possible, so that the interaction force between the magnets is small, ultimately reducing the driving load of each rotating magnet unit and making the rotational drive of the magnets more stable.

For example, see Figure 2. In the two opposing rotating magnet units in Figure 2, the end opposite to the direction of the arrow is the end where the magnet is installed.

In some embodiments, the number of multiple rotating magnet units is four, and the reference axes of the four rotating magnet units lie in the same plane.

Since the direction of the magnetic moment of the magnet in each rotating magnet unit is perpendicular to the reference axis, the magnetic moments of the four rotating magnet units sweep through the YZ, XZ, YZ, and XZ planes respectively (refer to Figure 2). Because the rotational speeds of the individual rotating magnet units are different, the magnetic field signals generated separately by the four rotating magnet units are decouplable (this is an inherent characteristic; in actual processing, it is not necessarily required to actually perform decoupling calculations). Thus, within a certain time period, by extracting all the magnetic moment directions at each moment, multiple sets of "X-Y-Z orthogonal pairs" can be formed. Using these magnetic moment directions and the magnetic field data measured at the corresponding times for positioning, through simulation and experimental verification, a better positioning effect can be achieved.

In some embodiments, the reference axes of any two adjacent rotating magnet units among the four rotating magnet units are perpendicular to each other. Because in this field emitter configuration, the reference axes of each rotating magnet unit lie in the same plane, the overall field emitter has a flat structure. Compared to field emitters where the reference axes of each rotating magnet unit are distributed in three dimensions, the field emitter in this embodiment is smaller in volume, simpler in structure, and does not occupy excessive space. For example, the reference axes of the four rotating magnet units are generally arranged in a "+" shape, meaning the centers of mass of the four rotating magnet units are distributed circumferentially, and the reference axes of the four rotating magnet units intersect at the same point. When adopting the above configuration, the field emitter configuration has strong symmetry, resulting in better positioning uniformity during the electromagnetic navigation and positioning process.

The relative positions of the rotating magnet units in the field emitter have certain design principles: the rotation axes of at least two rotating magnet units are perpendicular to each other, so that the magnetic moments can sweep through the three orthogonal directions of X, Y, and Z, and the distribution is as uniform as possible.

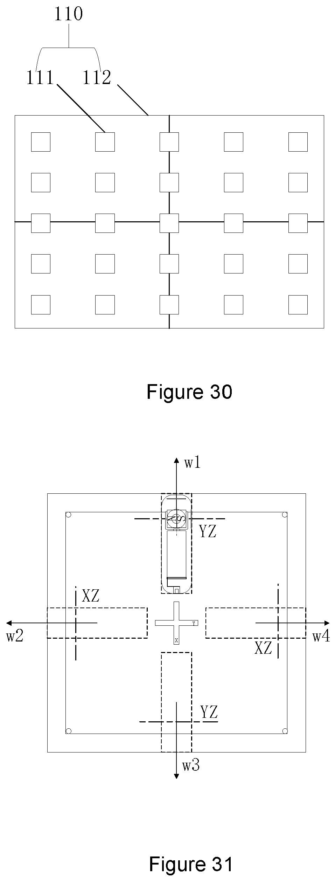

Figure 31 is a schematic diagram of the configuration of a field emitter according to some embodiments of this specification. Referring to Figure 31, different permanent magnets scan in different planes. Combined, they can form multiple sets of "X-Y-Z orthogonal pairs," meaning that from the time series of permanent magnet scans, multiple magnetic moment combinations pointing in the X direction, Y direction, and Z direction can always be formed. In the figure, w1, w2, w3, and w4 represent the directions of the rotation axes (reference axes of the rotating magnet units) of the four permanent magnets. The direction of the magnetic moment of the permanent magnet in each rotating magnet unit is perpendicular to its respective rotation axis. Therefore, the magnetic moments of the permanent magnets of the four rotating magnet units sweep through the YZ, XZ, YZ, and XZ planes respectively. Because the rotational speeds of the permanent magnets are different, in principle, the magnetic field signals they generate individually are decouplable to a certain extent (this is an inherent characteristic; in actual processing, it is not necessarily required to actually perform decoupling calculations). Therefore, within a certain time period, by extracting all the magnetic moment directions at each moment, multiple sets of "X-Y-Z orthogonal pairs" can be formed. Using these magnetic moment directions and the magnetic field data measured at the corresponding times for positioning, through simulation and experimental verification, a better positioning effect can be achieved.

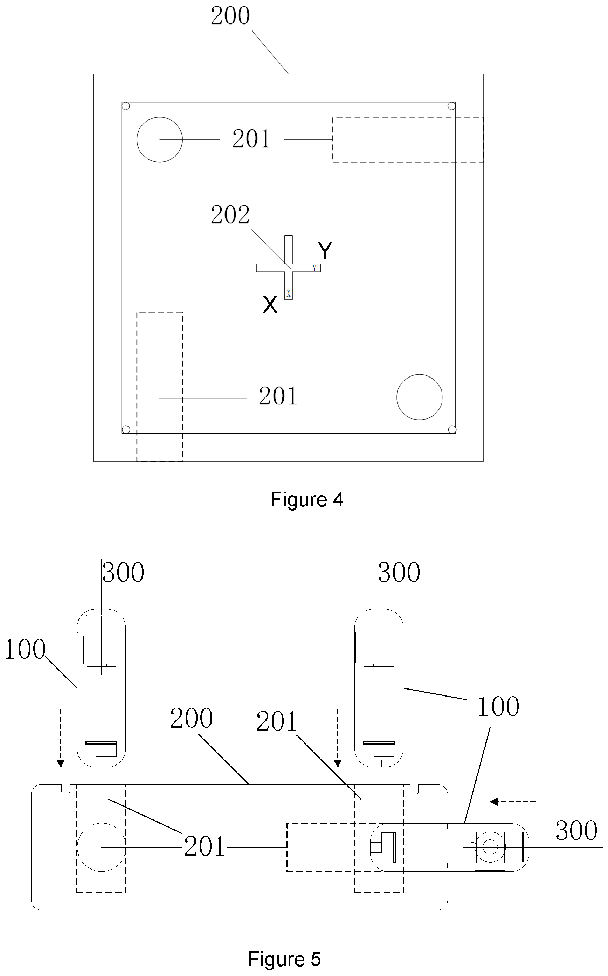

Figure 4 is a top view of an exemplary structure of a field emitter according to other embodiments of this specification, and Figure 5 is a side view of an exemplary structure of a field emitter according to other embodiments of this specification.

As shown in Figures 4 and 5, four rotating magnet units are arranged in the field emitter, and the reference axes of any two adjacent rotating magnet units are perpendicular, and the reference axes of the four rotating magnet units are not located in the same plane. The difference between the field emitters shown in Figure 2 and Figure 4 is that the reference axes of the rotating magnet units arranged in Figure 2 are all located in the same plane, while the reference axes of the four rotating magnet units of the field emitter shown in Figure 4 are not located in the same plane.

In some embodiments, the number of rotating magnet units is three, and the reference axes of the three rotating magnet units are perpendicular to each other. The field emitter uses three rotating magnet units, and the magnetic moment rotation planes of any two rotating magnet units are perpendicular to each other, which allows each of the three orthogonal spatial directions to be swept by the magnetic moments of exactly two magnets.

In some embodiments, after the field emitter is installed, at least one relative position calibration is required, i.e., determining the relative positions of each rotating magnet unit in the field emitter. For details on the magnetic detection assembly and self-calibration method used for self-calibration, see below.

After the relative positions of each rotating magnet unit are determined, the interaction torque calculation can be performed as a feedforward input for the motor drive control loop. When the relative distance of the rotating magnet units exceeds several times the magnet size (e.g., 4 times), the Dipole model can be used to approximate the interaction torque. Exemplarily, in a field emitter composed of 4 rotating magnet units, when calculating the interaction torque on the magnet of the first rotating magnet unit, first calculate the combined magnetic field time series **B**(*t*) generated by the other three rotating magnet units at the permanent magnet of the first rotating magnet unit, then calculate the magnetic moment sequence **m**(*t*) of the first rotating magnet unit based on its absolute position encoder data; finally, calculate the interaction torque sequence **T**(*t*) on the permanent magnet of the first rotating magnet unit. The calculation formula (1) is as follows: $T \left(t\right) = m \left(t\right) \times B \left(t\right)$

Multiplying the combined magnetic field and the magnetic moment at the same time instant yields the interaction torque at that same time instant. Performing the same calculation for data at each time instant yields the interaction torque sequence.

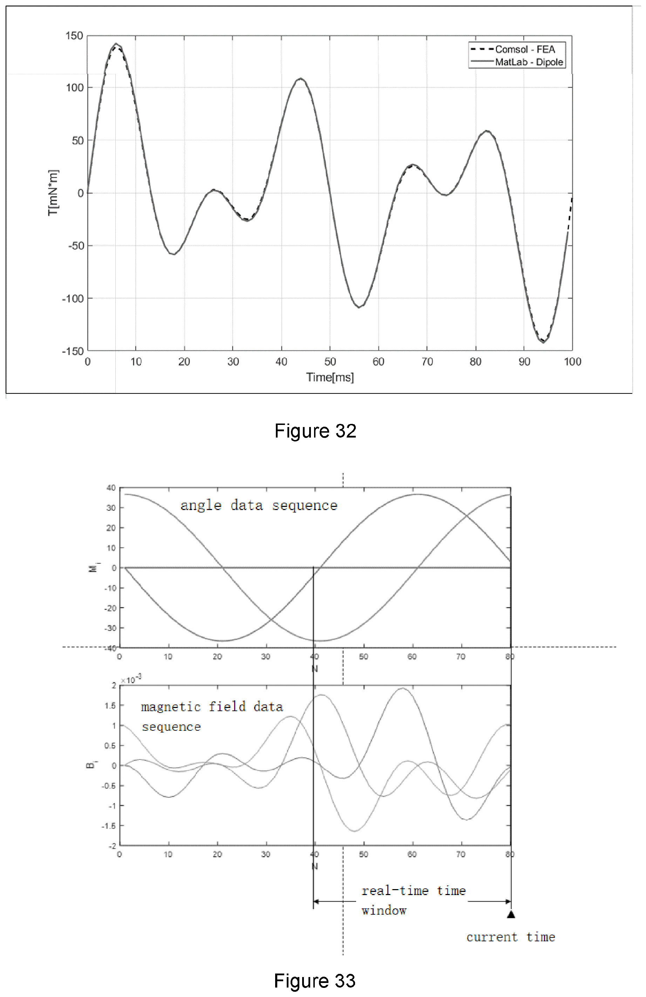

Figure 32 is a schematic diagram of the interaction torque of each rotating magnet unit according to some embodiments of this specification. When the magnetic strength of the permanent magnet is large, the interaction torque is relatively strong and can reach 10 ~ 10² *mN* · *m* , and the torque waveform experienced by the permanent magnet at different rotational speeds is different. Referring to Figure 32, the interaction torque is a time-varying strong load, which imposes a certain burden on the motor drive control. However, if the load is modeled more accurately and input into the control loop as a feedforward model, the drive module can adjust the drive current in a timely manner, thereby significantly improving the response speed and accuracy of the control, making the motor operate more stably. The modulation method of the drive current is a well-known technique in the field of motor control. Once the time-varying characteristics of the motor load are determined, these time-varying characteristics can be input into existing motor drive current modulation algorithms to obtain the modulated motor drive current.

When the field emitter size is small and the permanent magnet size is large, the Dipole model is not accurate enough. In this case, finite element analysis (FEA) can be used for simulation (e.g., software like Comsol).

The above embodiments introduce two specific field emitter configurations, both of which can be used to build a field emitter. It should be noted that the field emitter configuration can also take other forms, as long as the reference axes of at least two rotating magnet units are perpendicular to each other. When using more rotating magnet units, additional rotating magnet units can be added based on the two configurations mentioned above.

Figures 2 to 5 show two field emitter configurations. In practice, 2, 3, 5, or other numbers of rotating magnet units 100 can also be used (4 to 5 is optimal). In the field emitter, the rotational speed of each rotating magnet unit 100 is different, such as 10Hz, 20Hz, 30Hz, 40Hz, or alternatively 10Hz, 10Hz, 20Hz, 20Hz, etc. Among them, the initial magnetic moment directions of the rotating magnet units with the same rotational speed are different, for example, differing by 90°. Correspondingly, the number and rotational speed of each rotating magnet unit 100 can also be adjusted according to the actual usage scenario and requirements, so that the magnetic field has higher spatial and temporal variability.

The field emitter may further include a drive assembly. In some embodiments, the drive assembly may include a motor, a reduction mechanism, and an absolute position encoder. The output shaft of the motor is connected to the magnet via the reduction mechanism, and the absolute position encoder is used to collect the angular position information of the magnet.

In some embodiments, the drive assembly may further include a transmission device, and the motor drives the magnet to rotate via the transmission device. The transmission device can be a transmission shaft, a transmission belt, a transmission chain, gears, etc. This embodiment does not limit the specific form of the transmission device, as long as it can achieve the transmission function. One end of the transmission device can be connected to the motor, and the other end can be connected to the reduction mechanism.

The motor is the main driving component, which drives the magnet to rotate through the reduction mechanism. The absolute position encoder can collect the angular position information of the magnet in real-time and record the operating status of the motor, providing a basis for determining the time-varying magnetic field state during subsequent electromagnetic navigation, thereby determining the model value of the magnetic field state at the magnetic sensor.

In some embodiments, for a field emitter, among the multiple rotating magnet units it includes, one rotating magnet unit may include a motor and a transmission device, while other rotating magnet units include a transmission device but not a motor. For example, using one motor to simultaneously drive the magnets in multiple rotating magnet units to rotate, the magnet in a certain rotating magnet unit can be directly connected to the motor via a transmission shaft/reduction mechanism, while other rotating magnet units are connected to the motor via a transmission device, such as a transmission shaft or belt (and may also have a reduction mechanism), thereby achieving one motor driving the rotation of magnets in multiple rotating magnet units, and the rotational speeds of the magnets in the multiple rotating magnet units can be the same or different.

Exemplarily, each rotating magnet unit may include a transmission device, and the motor is installed outside the rotating magnet unit but inside the field emitter. This motor can be used to drive the rotation of the magnets of all rotating magnet units.

In some embodiments, for a field emitter, among the multiple rotating magnet units it includes, each rotating magnet unit may include a motor and a transmission device. For example, the magnet of each rotating magnet unit is driven to rotate by a single motor.

Exemplarily, the motor of each rotating magnet unit can be installed inside the rotating magnet unit, or outside the rotating magnet unit but inside the field emitter.

The above-mentioned drive assembly can be applied to all field emitters involved in this specification, for example, the field emitters in Figures 2 to 5, field emitters including a self-check assembly, field emitters including a magnetic detection assembly (see description below), field emitters including a magnetic source component (see description below), etc.

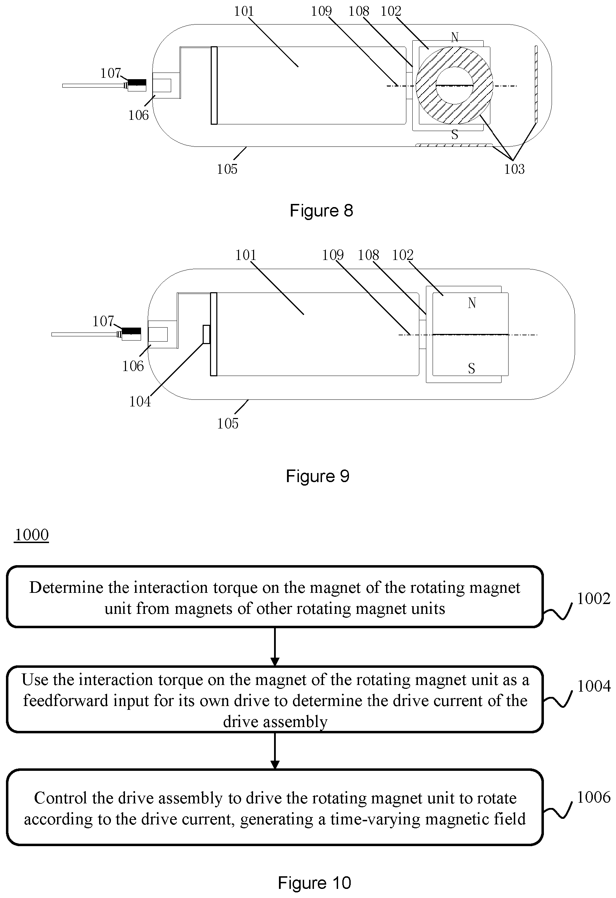

As shown in Figures 8 and 9, Figures 8 and 9 are exemplary structural diagrams of a rotating magnet unit according to some embodiments of this specification. This specification provides a rotating magnet unit. The rotating magnet unit may include a drive assembly 101, a magnet 102, and a self-check assembly. A field emitter may include multiple rotating magnet units, wherein at least one rotating magnet unit may be the rotating magnet unit in this embodiment. That is, among the multiple rotating magnet units in the field emitter, at least one rotating magnet unit may further include a self-check assembly. The relative positions of multiple rotating magnet units in the field emitter are not limited. For example, the relative positions of multiple rotating magnet units in the field emitter can be seen in the descriptions in Figures 2-5.

The drive assembly is connected to the magnet and is used to drive the magnet to rotate. In some embodiments, the drive assembly may include a motor and a transmission assembly. Among the multiple rotating magnet units, at least one rotating magnet unit includes a motor. For example, among the multiple rotating magnet units, one rotating magnet unit includes a motor, and the remaining rotating magnet units do not include a motor.

Exemplarily, the multiple rotating magnet units include a first rotating magnet unit and a second rotating magnet unit. The first rotating magnet unit includes a first motor and a first magnet, and the second rotating magnet unit includes a second motor and a second magnet. The first motor is used to drive the first magnet to rotate, and the second motor is used to drive the second magnet to rotate; alternatively, the first magnet unit includes a first motor and a first magnet, and the second magnet unit includes a first transmission assembly and a second magnet. The first motor is used to drive the first magnet to rotate, and the first motor is also used to drive the second magnet to rotate by driving the first transmission assembly. For more details on the drive assembly, please refer to the relevant description above.

The self-check assembly is used to detect the magnetic field signal generated during the rotation of the magnet.

The self-check assembly is a component in the rotating magnet unit used to detect the magnetic field generated by the rotation of the rotating magnet unit. In some embodiments, in addition to detecting the magnetic field signal generated by the magnet of the rotating magnet unit, the self-check assembly can also implement other functions, such as detecting interference magnetic fields in the field emitter environment. In some embodiments, the spatial pose of the self-check assembly remains fixed within the rotating magnet unit, meaning the spatial pose of the self-check assembly relative to the rotating magnet unit is fixed.

In the initial state of the rotating magnet unit, the self-check assembly can be used to detect the magnetic field signal generated when the magnet rotates at a preset speed, obtaining a reference time-varying magnetic field signal. Here, the initial state of the rotating magnet unit can be the factory state. The rotating magnet unit undergoes strict calibration of strength and magnetic moment direction at the factory. During subsequent use, the self-check assembly can be used again to detect the magnetic field signal generated when the magnet rotates at the preset speed, obtaining the current time-varying magnetic field signal of the magnet. By comparing the current time-varying magnetic field signal of the magnet with the reference time-varying magnetic field signal, it can be determined whether the rotating magnet unit has an operational anomaly.

Exemplarily, the current magnetic field strength can be determined based on the current time-varying magnetic field signal, as well as the proportional relationship of each component in the current time-varying magnetic field signal (e.g., the proportional relationship of the x-component, y-component, and z-component). If the current magnetic field strength has decreased by a significant proportion compared to the reference magnetic field strength (the magnetic field strength corresponding to the reference time-varying magnetic field signal), for example, a decrease ratio of 5%, it fully indicates that the magnet strength in the rotating magnet unit has degraded by 5%. Then, the magnetic moment strength value of the rotating magnet unit needs to be corrected in the subsequent positioning algorithm model. If the proportional relationship of each component in the current time-varying magnetic field signal has changed significantly compared to the proportional relationship of each component in the reference time-varying magnetic field signal, it may be that the magnetic moment direction of the magnet has changed, the mechanical structure has loosened, or there is interference in the surroundings (such as ferromagnetic materials, other time-varying magnetic fields, etc.). Then, interference detection needs to be performed on the environment around the magnet, or the rotating magnet unit needs to be removed for shutdown maintenance. For details on the interference detection method, please refer to the relevant description below.

In some embodiments, the field emitter can perform a self-check through the self-check assembly in the rotating magnet unit before use. During the self-check, first determine the rotating magnet unit to be self-checked in the field emitter, then lock the magnets of the other rotating magnet units. For the rotating magnet unit to be self-checked, use the drive assembly to drive the magnet to rotate at a preset speed, while using the self-check assembly to detect the magnetic field signal generated by the magnet, obtaining the current time-varying magnetic field signal of the magnet during this self-check process. After obtaining the current time-varying magnetic field signal, compare it with the reference time-varying magnetic field signal, determine the signal difference between the two, and determine the self-check result of the rotating magnet unit based on this signal difference. Here, the reference time-varying magnetic field signal is the time-varying magnetic field signal that the self-check assembly should detect when the rotating magnet unit is operating normally and the magnet is rotating at the preset speed. For example, it can be a time-varying magnetic field signal obtained by simulating a normally rotating magnet unit; it can also be a time-varying magnetic field signal detected by the self-check assembly when the rotating magnet unit leaves the factory. The principle is that under normal conditions, the current time-varying magnetic field signal and the reference time-varying magnetic field signal should be the same. If the signal difference exceeds a threshold, it indicates that the corresponding rotating magnet unit has an anomaly. If the self-check result is normal, or the anomaly issue has been resolved (such as modifying the magnet strength or eliminating the interference source), the field emitter can be used normally.

In some embodiments, when the field emitter includes a single rotating magnet unit, during the self-check, this rotating magnet unit is taken as the target rotating magnet unit. Since there are no other rotating magnet units in the field emitter besides the target rotating magnet unit, there is no need to lock other rotating magnet units. Afterwards, the self-check can be performed according to the self-check method described in the above embodiments.

For a detailed description of the self-check process, please refer to the descriptions in Figures 10-15.

In some embodiments, the self-check assembly includes three ring coils 103 whose normal vectors are pairwise orthogonal; the three ring coils 103 are respectively located on three sides of the magnet. The normal vector of one of the ring coils coincides with the rotation axis 109 of the magnet, and the normal vectors of the other two ring coils are perpendicular to the rotation axis 109 of the magnet.

In this embodiment, three ring coils with pairwise orthogonal normal vectors are used to detect the magnetic field signal generated by the magnet. Specifically, the three ring coils are respectively located on three sides of the magnet, and one of the ring coils can be arranged on the side of the magnet away from the drive assembly. When the magnet rotates, the three ring coils can generate corresponding induced voltages. The induced voltage of each ring coil indirectly reflects the strength of the magnet at the position of the corresponding ring coil, so the magnetic field signal generated by the magnet can be determined based on the induced voltages of the ring coils.

It should be noted that in some other embodiments, the number of ring coils may not be limited to three, and the relative pose relationship where the normal vectors are pairwise orthogonal may not be required. Here, the self-check assembly needs to detect the signal components of the magnetic field signal in three spatial dimensions. Therefore, using three ring coils with pairwise orthogonal normal vectors is a preferred embodiment. The three ring coils can respectively detect the signal components of the magnetic field signal in the three spatial dimensions. In other embodiments, it is only necessary to ensure that the normal vectors of the multiple ring coils are not in the same plane. In this case, by decoupling the detection signals of the multiple ring coils, the signal components of the magnetic field signal in the three spatial dimensions can be obtained.

In some embodiments, the self-check assembly may also include a magnetic sensor 104. The magnetic sensor 104 is located on the side of the drive assembly away from the magnet. For example, the self-check assembly can collect the magnetic field signal through the magnetic sensor, and then perform the self-check.

In this embodiment, the self-check assembly uses a magnetic sensor to detect the magnetic field signal generated by the magnet. The magnetic sensor can be a MEMS (Micro Electro Mechanical System) magnetic sensor, etc. MEMS magnetic sensors can be Hall sensors, anisotropic magnetoresistance (AMR) sensors, tunnel magnetoresistance (TMR) sensors, etc. Preferably, the magnetic sensor can be arranged on the side of the drive assembly away from the magnet, so that the distance between the magnetic sensor and the magnet is larger, effectively preventing the magnetic sensor from saturating. Preferably, the magnetic sensor is a three-axis digital sensor, enabling more accurate detection of the magnetic field signal generated by the magnet.

Through the above self-check assembly, each rotating magnet unit in the field emitter can be self-checked sequentially, for example, one rotating magnet unit at a time. Before each use of the field emitter, the user can perform a self-check on each rotating magnet unit through its internal self-check assembly, promptly identifying any problematic rotating magnet units, preventing the field emitter from generating a time-varying magnetic field that deviates from the standard during operation, and ultimately ensuring the positioning accuracy of the magnetic sensor. This solves the problem that existing field emitters lack a self-check function, and when a rotating magnet unit malfunctions, it cannot be promptly detected through self-check, causing the field emitter to generate a time-varying magnetic field deviating from the standard during operation, ultimately reducing the positioning accuracy of the magnetic sensor.

In this embodiment, the self-check assembly can detect the magnetic field signal generated by the magnet. By comparing the current time-varying magnetic field signal of the magnet with a reference time-varying magnetic field signal, it can be determined whether the rotating magnet unit is operating abnormally. Therefore, the rotating magnet unit provided in this specification can perform a self-check through the self-check assembly before each use, promptly identifying its own problems, preventing the field emitter from generating a time-varying magnetic field deviating from the standard during operation, and ultimately ensuring the positioning accuracy of the magnetic sensor. This solves the problem that when an existing rotating magnet unit malfunctions, it causes the field emitter to generate a time-varying magnetic field deviating from the standard during operation, ultimately reducing the positioning accuracy of the magnetic sensor.

Furthermore, since the rotating magnet unit provided in this specification has a self-check function, the field emitter also has a self-check function in this embodiment. Before each use of the field emitter, the user can perform a self-check on each rotating magnet unit through its internal self-check assembly, promptly identifying any problematic rotating magnet units, preventing the field emitter from generating a time-varying magnetic field that deviates from the standard during operation, and ultimately ensuring the positioning accuracy of the magnetic sensor. This solves the problem that existing field emitters lack a self-check function, and when a rotating magnet unit malfunctions, it cannot be promptly detected through self-check, causing the field emitter to generate a time-varying magnetic field deviating from the standard during operation, ultimately reducing the positioning accuracy of the magnetic sensor.

In some embodiments, the self-check assembly can also be used for real-time interference detection. During the positioning process of magnetic navigation, the self-check assembly of each rotating magnet unit can capture the magnetic field waveform in real time. This function is different from the self-check of the rotating magnet unit. In the self-check of the rotating magnet unit, only one rotating magnet unit operates at a time, while the other rotating magnet units are locked. When the interference detection function is executed, the field emitter is in a normal positioning working state, and all rotating magnet units are rotating. Therefore, the magnetic field waveform captured by the magnetic detection at this time is different from that obtained during the self-check of the rotating magnet unit. During the interference detection process, if the environment is stable and there is no interference, the waveforms captured by each self-check assembly are also known. When the self-check signal captured by the self-check assembly shows an out-of-limit change, it is highly likely that there is magnetic field interference in the surroundings (such as ferromagnetic material interference), and problem investigation is required.

In some embodiments, the rotating magnet unit further includes a mounting housing 105. The drive assembly, the magnet, and the self-check assembly are all installed inside the mounting housing 105; the mounting housing 105 is provided with a functional interface 106, and the functional interface 106 connects the drive assembly and the self-check assembly.

The mounting housing serves as the installation carrier for the components of the rotating magnet unit, fixing and protecting the internal components. To avoid affecting the time-varying magnetic field, the mounting housing is preferably made of non-metallic material. A fixing member 108 for the magnet 102 is arranged inside the mounting housing 105. Meanwhile, to achieve data transmission and power transmission, the mounting housing can also be provided with a functional interface for connecting to an external interface 107. Through this functional interface, encoder data from the drive assembly can be output, detection data from the self-check assembly can be output, or an external power source can be connected to the drive assembly.

In some embodiments, the overall shape of the mounting housing can be a rectangular structure. The body coordinate system of the field emitter can be constructed with reference to the mounting housing. The rotating magnet unit is fixed relative to the mounting housing, and the spatial pose of the rotating magnet unit in the field emitter body coordinate system is fixed.

Magnetic detection assemblies can also be arranged on the upper and lower surfaces of the mounting housing. For details on the magnetic detection assembly, please refer to the description below.

In some embodiments, the field emitter further includes a mounting body 200, which is provided with a plurality of mounting positions 201 for installing a plurality of rotating magnet units. For example, each of the plurality of mounting positions is used to install one rotating magnet unit.

The mounting body serves as the installation carrier for each rotating magnet unit. In some embodiments, the dimensions of each mounting body can be the same. If the field emitter uses mounting bodies of the same size, the configuration center of the field emitter configurations shown in Figures 2 and 3 needs to be set at the center position of the mounting body, while in the configurations of the field emitter shown in Figures 4 and 5, the intersection point of the reference axes of the three rotating magnet units can be set at a corner position of the mounting body, thereby increasing the spacing between the magnets in each rotating magnet unit, ultimately reducing the interaction force between the magnets.

In some embodiments, the mounting body is provided with at least two mounting positions, which can be groove-like structures arranged on the surface of the mounting body. The arrangement positions of the groove-like structures match the field emitter configuration. The rotating magnet unit is installed in the corresponding groove-like structure.

Meanwhile, a power source is also arranged inside the mounting body, and functional interfaces matching the rotating magnet unit can be arranged on the mounting positions. After the rotating magnet unit is installed, the functional interface on the mounting position is connected to the functional interface on the rotating magnet unit for power supply and data transmission.

In some embodiments, the mounting body 200 of the field emitter is also provided with an indicator groove 202, which is used to mark the XY plane of the body coordinate system of the field emitter, while the Z-axis is perpendicular to the XY plane and points outward from the surface. Four mounting positions are used to install the rotating magnet units, with corresponding fixing fixtures, power supply, and data interfaces (not shown) inside, which can be connected to the functional interfaces of the rotating magnet units. After the rotating magnet units are installed, their relative positional relationships are fixed.

In some embodiments, the orientation of the mounting positions is different from the previous configuration. Comparatively, the distance between the magnets of the rotating magnet units is relatively larger, so the interaction force between them is smaller, the motor load of each rotating magnet unit during rotation is lower, and the drive is easier to stabilize. The previous configuration is an axisymmetric structure, so the uniformity of the positioning effect is relatively better.

In some embodiments, the rotating magnet unit further includes a locking assembly. The locking assembly is used to lock the magnet of the rotating magnet unit so that the magnet does not rotate. For example, the locking assembly can be used to lock the angular position of the magnet. Each rotating magnet unit can include a locking assembly.

In some embodiments, since the field emitter is composed of multiple rotating magnet units, when performing a self-check on a target rotating magnet unit, the influence of other rotating magnet units on the target rotating magnet unit needs to be avoided, meaning the magnets in the other rotating magnet units must not rotate. Therefore, the rotating magnet unit can further include a locking assembly, through which the angular position of the magnet can be locked. Exemplarily, the locking assembly may adopt a clamping structure to clamp the magnet, preventing it from rotating under significant friction. The locking assembly may also adopt a snap-fit structure. In this case, the magnet surface can be provided with a slot that cooperates with the snap-fit structure. When the snap-fit structure extends into the slot, it can restrict the rotation of the magnet.

The target rotating magnet unit refers to the magnet unit undergoing self-check among the plurality of rotating magnet units.

The aforementioned mounting housing, mounting body, and locking assembly can be applied to all field emitters involved in this specification, for example, the field emitters in Figures 2 to 5, field emitters including a self-check assembly, field emitters including a magnetic detection assembly (described below), field emitters including a magnetic source assembly (described below), etc.

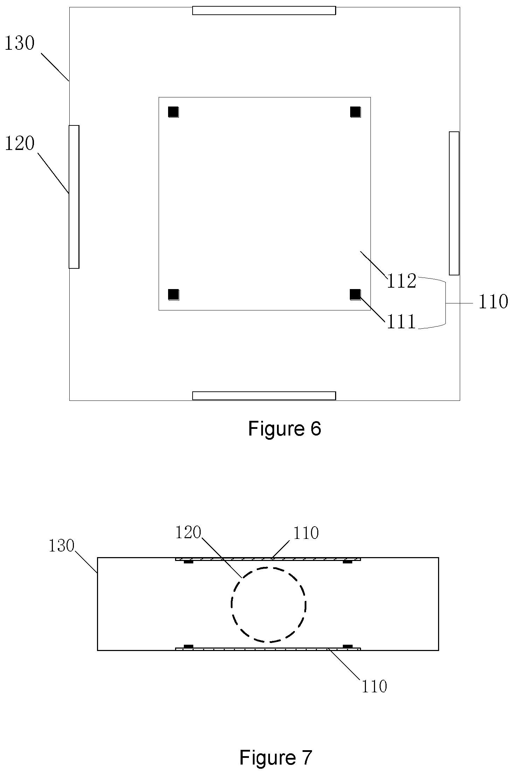

FIG. 6 is a top view of an exemplary structure of a magnetic detection assembly according to some embodiments of this specification, and FIG. 7 is a side view of an exemplary structure of a magnetic detection assembly according to some embodiments of this specification.

In some embodiments, the magnetic detection assembly can be used to detect magnetic field signals generated by the magnet of each of the plurality of rotating magnet units. In some embodiments, the spatial pose of the magnetic detection assembly remains fixed within the field emitter, meaning the spatial pose of the magnetic detection assembly relative to the field emitter is fixed and unchanged.

In some embodiments, the field emitter may include a field emitter unit group and a magnetic detection assembly 110; the field emitter unit group includes a plurality of rotating magnet units; the magnetic detection assembly 110 is used to detect the calibration magnetic field generated by the rotating magnet units. In this embodiment, the field emitter may not include a self-check assembly, or, among the plurality of rotating magnet units in the field emitter, at least one rotating magnet unit may further include a self-check assembly. The relative positions of the plurality of rotating magnet units in the field emitter are not limited. For example, the relative positions of the plurality of rotating magnet units in the field emitter can be seen in the description of Figures 2-5.

In some embodiments, the magnetic detection assembly 110 may include a plurality of magnetic sensors 111 and a circuit board 112, with the plurality of magnetic sensors 111 uniformly installed on the circuit board 112. The plurality of magnetic sensors can be spaced apart from each other and fixedly installed on the circuit board as uniformly as possible. The transmission control circuit for the magnetic sensors can be integrated on the circuit board to facilitate data output from the magnetic sensors. When the magnetic detection assembly uses multiple magnetic sensors, compared to using a single magnetic sensor, the parameter calibration of the rotating magnet unit is ultimately more accurate because more data is available for reference during the optimization process.

Referring to Figures 6 and 7, in some embodiments, the field emitter unit group and the magnetic detection assembly can be installed (e.g., fixedly installed) inside the mounting housing. Specifically, the field emitter unit group can be fixedly installed in the middle of the mounting housing, while the magnetic detection assembly can be installed on the inner side wall of the mounting housing. For example, when the magnetic detection assembly includes a circuit board and magnetic sensors installed on the circuit board, the circuit board can be directly fixedly installed on the inner side wall of the mounting housing. If there are two sets of magnetic detection assemblies, the two sets can be fixedly installed on the top wall and bottom wall of the mounting housing, respectively.

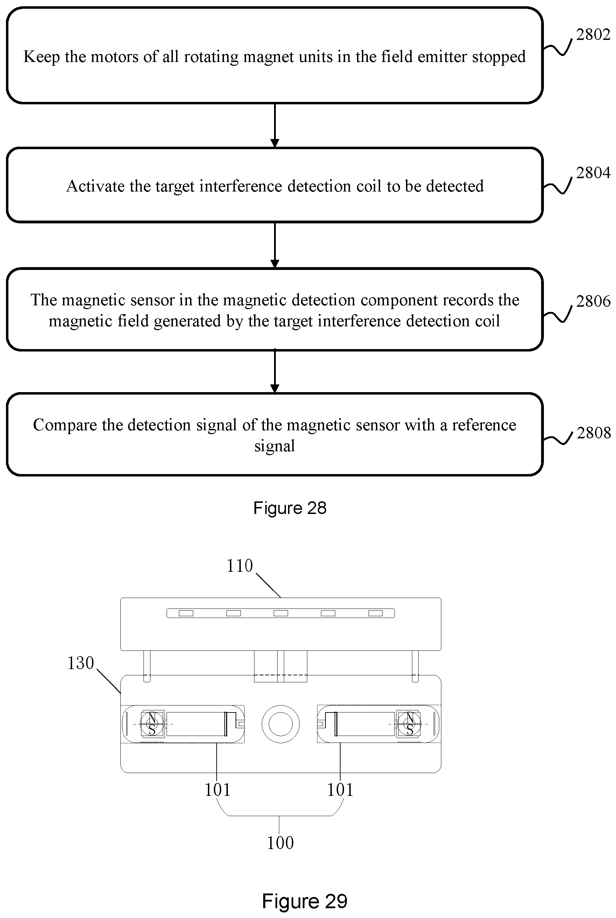

Correspondingly, referring to Figures 29 and 30, Figures 29 and 30 are exemplary schematic diagrams of another field emitter structure according to some embodiments of this specification. In some embodiments, the field emitter unit group is installed inside the mounting housing, while the magnetic detection assembly is detachably or non-detachably installed outside the mounting housing. The magnetic detection assembly can be installed above the mounting housing via corresponding connecting fixtures. In this case, to ensure that the installation position of the magnetic detection assembly is the same as the designed position, corresponding positioning grooves can be provided on the mounting housing, and the magnetic detection assembly is provided with positioning protrusions that cooperate with the positioning grooves. Through the matching of the positioning grooves and positioning protrusions, after the magnetic detection assembly is installed, its relative pose to the mounting housing meets the design requirements and is fixed.

In the above embodiments, whether the magnetic detection assembly is installed inside or outside the mounting housing, the spatial pose of the magnetic detection assembly relative to the mounting housing is fixed. Therefore, the spatial pose of the magnetic detection assembly in the field emitter body coordinate system is relatively fixed.

In some embodiments, the magnetic detection assembly contains multiple magnetic sensors (e.g., 8), distributed on the inner side of the field emitter's mounting housing. The multiple magnetic sensors are spaced apart by a certain distance to be distributed as uniformly as possible inside the mounting housing. The multiple magnetic sensors are fixedly connected to the mounting housing of the field emitter (after the magnetic sensors are installed, they can be fixed with glue), jointly defining a coordinate system {s}, which is the field emitter body coordinate system. Therefore, the position of each magnetic sensor within the coordinate system {s} is determined. The magnetic sensors can be any type of magnetic sensor, such as single-axis or three-axis induction coils, three-axis MEMS sensors, etc. Inside the side surface of the field emitter's mounting housing, there are also several coils for interference detection (e.g., 4). These coils constitute the magnetic source assembly, with one coil arranged on each side surface of the mounting housing. The coils are used to generate designed magnetic field signals, such as sinusoidal signals, square wave signals, etc. The magnetic field signals can be detected by the magnetic sensors.

In some embodiments, the field emitter includes a magnetic source assembly 120, whose spatial pose within the field emitter is fixed.

When the magnetic source assembly operates, it can generate a detection magnetic field. When the magnetic source assembly operates, the magnetic detection assembly is also used to detect the detection magnetic field generated by the magnetic source assembly. For example, the magnetic detection assembly includes a magnetic sensor, which can be used to detect the detection magnetic field generated by the magnetic source assembly. In some embodiments, when the field emitter includes a magnetic source assembly but does not include a magnetic detection assembly, the detection magnetic field generated by the magnetic source assembly can also be received by other detection assemblies. For example, when the field emitter includes a self-check assembly, the detection magnetic field generated by the magnetic source assembly can also be received by the self-check assembly. For another example, the detection magnetic field generated by the magnetic source assembly can also be received by other detection assemblies outside the field emitter.

The detection magnetic field can be used to detect whether there is magnetic interference in the working environment of the field emitter, for example, interference detection.

In some embodiments, the magnetic source assembly includes a plurality of coils uniformly distributed around the periphery of the plurality of rotating magnet units.

In this embodiment, the magnetic source assembly uses multiple coils. The coils have the characteristic of controllable magnetic fields, generating a detection magnetic field when energized and not generating a detection magnetic field when de-energized. Therefore, when interference detection is needed, the coils are turned on to generate a detection magnetic field; at other times, the coils are turned off so they do not generate a detection magnetic field, preventing the detection magnetic field from affecting other functions of the field emitter. Here, turning on the coils means energizing the coils, and turning off the coils means de-energizing the coils. At the same time, there are multiple coils, uniformly distributed around the periphery of the rotating magnet unit group. For example, they can be fixedly installed on various sides of the mounting housing 130 of the field emitter. Considering that the influence of a smaller magnetic field source on the field emitter's magnetic field is local, for example, affecting the field emitter's magnetic field only in a certain direction. Therefore, multiple coils are arranged around the field emitter, each used to detect whether there is an interference source in the corresponding direction. During detection, they generate detection signals one by one and perform the same phase comparison detection, thereby more comprehensively detecting the conditions around the field emitter.

In this embodiment, the magnetic source assembly is used to provide a detection magnetic field, and the spatial pose of the magnetic source assembly in the field emitter body coordinate system is relatively fixed. Therefore, when the detection magnetic field provided by the magnetic source component remains unchanged, the magnetic field signal obtained by the magnetic sensor detecting the detection magnetic field should be constant. If the magnetic field data detected by the magnetic sensor for the detection magnetic field changes compared to the reference detection magnetic field data, it indicates the presence of a magnetic field interference source around the field emitter. Here, the reference detection magnetic field data refers to the magnetic field data that should be obtained by the magnetic sensor detecting the detection magnetic field in an environment without interference sources. Interference sources can affect the self-calibration and electromagnetic navigation accuracy of the field emitter, so interference sources around the field emitter need to be eliminated promptly.

The spatial pose of the magnetic detection assembly within the field emitter is relatively fixed; for example, the centroid position and attitude of the magnetic detection assembly within the field emitter are fixed. The spatial pose of the magnetic detection assembly within the field emitter can be pre-calibrated. Similarly, the spatial pose of the rotating magnet unit group within the field emitter is also relatively fixed and can be pre-calibrated.

Typically, the field emitter undergoes rigorous calibration before leaving the factory to determine corresponding calibration parameters, such as the position coordinates of the magnet center of each rotating magnet unit in the field emitter body coordinate system, magnetic moment strength, and other magnet-related parameters. However, due to inevitable issues such as collision, thermal expansion and contraction, mechanical fatigue, and equipment aging during the use of the field emitter, the magnet-related parameters may change compared to the calibration parameters. If the electromagnetic navigation positioning algorithm continues to use the initial calibration parameters, the positioning accuracy will decrease.

To address the above issues, the magnetic detection assembly provided in the field emitter of this specification is arranged between the rotating magnet unit and includes at least one magnetic sensor. When the magnetic detection assembly is operating, the magnetic sensor is used to detect the calibration magnetic field generated by the rotating magnet unit. The magnetic sensor can be any type of magnetic field sensor, such as a single-axis or three-axis induction coil, or a three-axis MEMS (Micro Electro Mechanical System) magnetic sensor.

When calibrating the parameters of any rotating magnet unit in the field emitter, the target rotating magnet unit to be calibrated needs to be operated, while other non-target rotating magnet units are turned off; the non-target rotating magnet units will not generate a time-varying magnetic field. Operating the rotating magnet unit means putting the rotating magnet unit into a working state, such as driving the permanent magnet to rotate via its internal motor; at this time, the rotating magnet unit generates a time-varying magnetic field. Turning off the rotating magnet unit means putting the rotating magnet unit into a non-working state, such as locking its internal permanent magnet to prevent rotation; at this time, the rotating magnet unit does not generate a time-varying magnetic field. The target rotating magnet unit operates alone to generate a time-varying calibration magnetic field, and the magnetic sensor in the magnetic detection assembly detects this calibration magnetic field, obtaining the measured calibration magnetic field data at the magnetic sensor. Since the pose relationship between the magnetic sensor and the target rotating magnet unit is known and fixed, the model calibration magnetic field data at the magnetic sensor can be calculated using the magnetic field model of the target rotating magnet unit.

If all magnet-related parameters in the magnetic field model accurately match the current actual state of the target rotating magnet unit, the measured calibration magnetic field data and the model calibration magnetic field data should be consistent. If there is an error between the measured calibration magnetic field data and the model calibration magnetic field data, it indicates that the magnet-related parameters in the magnetic field model do not match the current actual state of the target rotating magnet unit. This means that the target rotating magnet unit has undergone a state change during operation, and the magnet-related parameters of the target rotating magnet unit need to be recalibrated. Specifically, the calibration method takes minimizing the error between the measured calibration magnetic field data and the model calibration magnetic field data as the optimization goal, continuously optimizing and adjusting the magnet-related parameters of the target rotating magnet unit, ultimately obtaining calibration parameters that match the current actual state of the target rotating magnet unit.

In some embodiments, for multiple rotating magnet units in the field emitter, the above method can be used sequentially to calibrate the parameters of the rotating magnet units. Each parameter calibration operates only one rotating magnet unit, i.e., only one rotating magnet unit is determined as the target rotating magnet unit at a time. Since the relevant data of each rotating magnet unit is collected separately, data processing can be simplified. Correspondingly, in other embodiments, for multiple rotating magnet units in the field emitter, the above method can be used to calibrate the parameters of multiple or all rotating magnet units simultaneously. Each parameter calibration operates multiple or all rotating magnet units, i.e., multiple or all rotating magnet units are determined as target rotating magnet units.

Since the magnetic detection assembly is provided, the calibration magnetic field generated by the rotating magnet unit can be detected by the magnetic sensor in the magnetic detection assembly. Furthermore, the measured calibration magnetic field data output by the magnetic sensor can be used to calibrate the magnet-related parameters of the corresponding rotating magnet unit, obtaining calibration parameters that match the actual state of the rotating magnet unit. Updating the calibration parameters of each rotating magnet unit in the magnetic field model makes the calculation results of the magnetic field model more accurate, thereby improving electromagnetic navigation accuracy.

During the calibration process of the rotating magnet unit, multiple magnetic sensors simultaneously detect the calibration magnetic field generated by the target rotating magnet unit (the rotating magnet unit being calibrated) and output their respective measured calibration magnetic field data. Through the magnetic field model, the model calibration magnetic field data of the working magnetic field at each magnetic sensor can be calculated. At this point, the error between the measured calibration magnetic field data and the model calibration magnetic field data corresponding to the same spatial position can be calculated separately. Then, taking minimizing the cumulative error or the mean error of the magnetic field data at multiple spatial positions as the optimization goal, the magnet-related parameters of the target rotating magnet unit are continuously optimized and adjusted, ultimately obtaining calibration parameters that match the current actual state of the target rotating magnet unit.

Some embodiments of this specification provide a rotating magnet unit, a field emitter including the rotating magnet unit, and its configuration and usage method. The field emitter is a time-varying magnetic field emitter based on a rotating magnet unit, which can be used in multiple scenarios such as surgical navigation. The field emitter includes at least one rotating magnet unit (TxU), which has the advantages of simplicity and stability. Rotating magnet units can be combined into more complex field emitters through preset combination principles. The field emitter has good scalability and is easy to calibrate, maintain, and replace.

FIG. 10 is an exemplary flowchart of a control method for a field emitter according to some embodiments of this specification. In some embodiments, the process 1000 shown in FIG. 10 may be executed by a processing device (e.g., processor 13). In some embodiments, process 1000 may include the following operations.

In some embodiments, the field emitter used to implement process 1000 includes multiple rotating magnet units, at least one of the rotating magnet units includes a magnet and a drive assembly, the drive assembly is connected to the magnet and is used to drive the magnet to rotate. For each rotating magnet unit in the field emitter, the operations shown in steps 1002-1006 below can be performed to generate a time-varying magnetic field. Process 1000 can be applied to all field emitters provided in this specification, for example, the field emitters of FIG. 2 to FIG. 5, the field emitter including a self-check assembly, the field emitter including a magnetic detection assembly, the field emitter including a magnetic source component, etc.

Step 1002, determine the interaction torque on the magnet of the rotating magnet unit from the magnets of other rotating magnet units.

Interaction torque refers to the torque generated between two or more magnets due to the interaction of magnetic fields.

In some embodiments, calculating the interaction torque on the magnet of each rotating magnet unit in a field emitter from magnets of other rotating magnet units may include: determining a target rotating magnet unit to be calculated in the field emitter; calculating the time-varying characteristic of the combined magnetic field at the magnet of the target rotating magnet unit, and calculating the time-varying characteristic of the target magnetic moment of the magnet of the target rotating magnet unit; wherein the combined magnetic field is jointly generated by non-target rotating magnet units in the field emitter; determining the time-varying characteristic of the interaction torque on the magnet of the target rotating magnet unit based on the time-varying characteristic of the target magnetic field and the time-varying characteristic of the target magnetic moment.

Wherein, the combined magnetic field refers to a magnetic field formed by the superposition of individual magnetic fields generated by multiple rotating magnet units. Exemplarily, when the relative distance between rotating magnet units exceeds several times the magnet size (e.g., 4 times), a Dipole model can be used to approximate the calculation of the interaction torque.

It should be noted that for a field emitter comprising only a single rotating magnet unit, the other rotating magnet units here may be rotating magnet units from other field emitters (e.g., field emitters located in the same space as this field emitter).

The process of determining the interaction torque on the magnet of the target rotating magnet unit includes: first, calculating the time-varying characteristic of the magnetic field of the combined magnetic field generated by other rotating magnet units at the magnet of the target rotating magnet unit; then, calculating the time-varying characteristic of the magnetic moment of the magnet of the target rotating magnet unit based on the absolute position encoder data of the target rotating magnet unit; finally, calculating the time-varying characteristic of the interaction torque on the magnet of the target rotating magnet unit.

Wherein, the time-varying characteristics of various physical quantities can be characterized in a sequential manner, primarily by multiplying the magnetic field vector of the combined magnetic field at the magnet at each moment with the magnetic moment vector of that magnet to obtain a vector product, which is the interaction torque on that magnet at the corresponding moment. The time-varying characteristic of the interaction torque on the magnet includes the interaction torque on the magnet at each moment.

When the field emitter size is small and the magnet size is large, the Dipole model may not be sufficiently accurate, and simulation using Finite Element Analysis (FEA) may be necessary. Therefore, in some embodiments, the time-varying characteristic of the interaction torque on the magnet of each rotating magnet unit can be determined through simulation. Specifically, this involves modeling the field emitter structure in simulation software and setting the operating parameters of each component, such as the rotation speed of each rotating magnet unit, magnet strength, and magnetic moment direction. The interaction torque between the magnets of each rotating magnet unit is calculated using the simulation software.

In some embodiments, before determining the interaction torque between the magnets of each rotating magnet unit, the relative positions between each rotating magnet unit can also be determined. Specifically, the coordinate information of each rotating magnet unit in the field emitter coordinate system can be determined.

Step 1004, using the interaction torque on the magnet of the rotating magnet unit as a feedforward input for its own drive, determining the drive current of the drive assembly.

When the field emitter is operating, each rotating magnet unit works simultaneously, and each generates a single time-varying magnetic field. The single time-varying magnetic field generated by each rotating magnet unit affects the other rotating magnet units. Therefore, the drive load of each rotating magnet unit when operating simultaneously differs from its drive load when operating alone. To ensure that the magnet of each rotating magnet unit can rotate stably at a preset speed when the field emitter is operating, the drive current of the rotating magnet unit needs to be modulated. In this specification, when the field emitter is operating, the interaction torque on the magnet of each rotating magnet unit from the magnets of other rotating magnet units is calculated. For each rotating magnet unit, the interaction torque on its magnet is used as a feedforward input for its own drive assembly, thereby modulating the drive current based on the time-varying characteristic of the interaction torque. For example, when the interaction torque is large, causing significant resistance to the rotation of the magnet, the current intensity of the drive current can be increased.

Step 1006, controlling the drive assembly to drive the rotating magnet unit to rotate based on the drive current, generating a time-varying magnetic field.

When the drive assembly applies the drive current to rotate the rotating magnet unit, the relative position of the magnet in space changes continuously, causing the magnetic field it generates to change accordingly.

In this embodiment, each rotating magnet unit in the field emitter can adjust the drive current in a timely manner based on the interaction torque, thereby significantly improving the rotational control accuracy of the magnet and making the motor in the drive assembly operate more stably. This solves the problem of low rotational control accuracy of the magnet by each rotating magnet unit in existing field emitters.

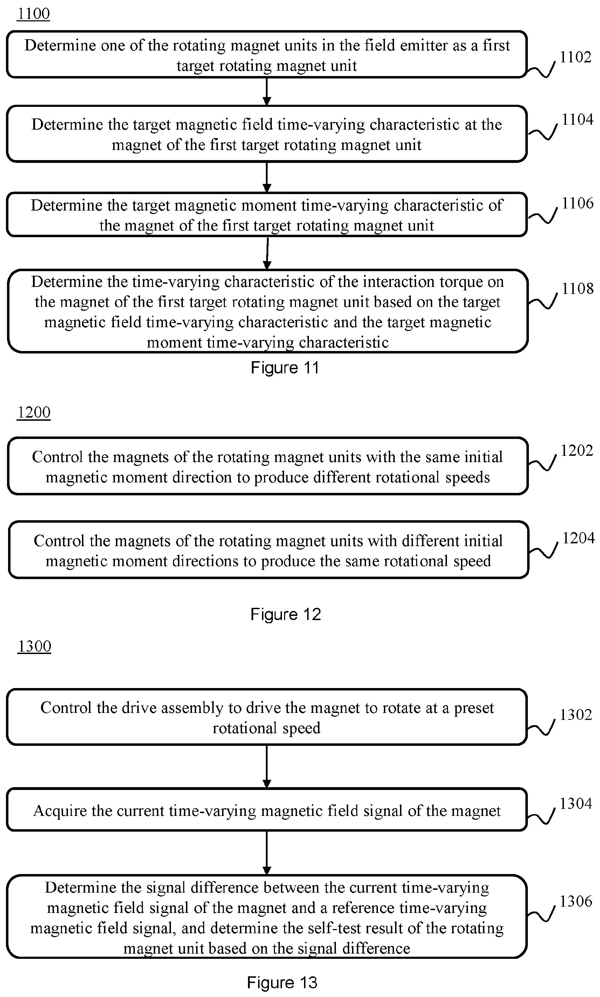

FIG. 11 is an exemplary flowchart for determining time-varying characteristics according to some embodiments of this specification. In some embodiments, the process 1100 shown in FIG. 11 can be executed by a processing device (e.g., processor 13) or an electro-magnetic transient simulator. As shown in FIG. 11, process 1100 may include the following operations.

In some embodiments, the field emitter used to implement process 1100 includes multiple rotating magnet units, at least one of the rotating magnet units includes a magnet and a drive assembly, the drive assembly is connected to the magnet and is used to drive the magnet to rotate. For each of the rotating magnet units in the field emitter, process 1100 can be executed to generate a time-varying magnetic field. Process 1100 can be applied to all field emitters provided in this specification, for example, the field emitters of FIG. 2 to FIG. 5, a field emitter including a self-check assembly, a field emitter including a magnetic detection assembly, a field emitter including a magnetic source assembly, etc.

Step 1102, determining one of the rotating magnet units in the field emitter as a first target rotating magnet unit.

In some embodiments, the processor can designate one rotating magnet unit in the field emitter as the first target rotating magnet unit, or it can randomly determine one from the multiple rotating magnet units of the field emitter as the first target rotating magnet unit.

Step 1104, determining the time-varying characteristic of the target magnetic field of the combined magnetic field at the magnet of the first target rotating magnet unit.

In some embodiments, the combined magnetic field is jointly generated by non-first target rotating magnet units in the field emitter. The time-varying characteristic of the target magnetic field of the combined magnetic field at the magnet of the first target rotating magnet unit can be detected by a magnetic sensor.

Step 1106, determining the time-varying characteristic of the target magnetic moment of the magnet of the first target rotating magnet unit.

The time-varying characteristic of the target magnetic moment refers to the time-varying characteristic of the magnet of the first target rotating magnet unit.

In some embodiments, the time-varying characteristic of the target magnetic moment can also be detected by a magnetic sensor.

Step 1108, determining the time-varying characteristic of the interaction torque on the magnet of the first target rotating magnet unit based on the time-varying characteristic of the target magnetic field and the time-varying characteristic of the target magnetic moment.

As described above, the time-varying characteristics of each target magnetic field and the time-varying characteristics of the target magnetic moment can be characterized in a sequential manner. Subsequently, the magnetic field vector of the combined magnetic field at the magnet at each moment is multiplied by the magnetic moment vector of the first target rotating magnet unit to obtain a vector product, which is the interaction torque on the magnet of the first target rotating unit at the corresponding moment. Subsequently, the time-varying characteristic of the interaction torque on the magnet of the first target rotating magnet unit can be determined through simulation.

The interaction torque on the magnet of each rotating magnet unit in the field emitter can be obtained through process 1000 and process 1100.

FIG. 12 is an exemplary flowchart of a control method for a field emitter according to some embodiments of this specification. In some embodiments, the process 1200 shown in FIG. 12 may be executed by a processing device (e.g., processor 13) or an electro-magnetic transient simulator. As shown in FIG. 12, the process 1200 may include the following operations.

The control method for the field emitter disclosed in this embodiment can be applied to a field emitter, which may include a plurality of rotating magnet units, wherein at least one of the rotating magnet units includes a drive assembly and a magnet. The process 1200 can be applied to all field emitters provided in this specification, for example, the field emitters of FIGS. 2 to 5, a field emitter including a self-check assembly, a field emitter including a magnetic detection assembly, a field emitter including a magnetic source assembly, etc.

Step 1202, controlling the magnets of the rotating magnet units having the same initial magnetic moment direction to generate different rotational speeds.

In some embodiments, when there are rotating magnet units with the same initial magnetic moment direction, the magnets of the rotating magnet units with the same initial magnetic moment direction can be controlled to generate different rotational speeds.

The magnetic moment direction of a rotating magnet is the magnetic moment direction of its own magnet, and the initial magnetic moment direction of a rotating magnet is the magnetic moment direction it has before the execution of the drive control method. When there are multiple rotating magnet units in the field emitter, the initial magnetic moment directions of the multiple rotating magnet units can be the same or different. For rotating magnet units with different initial magnetic moment directions, the rotational speeds of their magnets can be the same or different during operation.

Step 1204, controlling the magnets of the rotating magnet units having different initial magnetic moment directions to generate the same rotational speed.

In some embodiments, in some field emitters, there may be rotating magnet units with the same initial magnetic moment direction. During operation, it is necessary to control the magnets of these rotating magnet units to generate different rotational speeds, so as to facilitate the decoupling of the operating state data of the field emitter during data processing. It should be noted that in a rotating magnet unit, the magnet is usually driven to rotate by a motor. Therefore, the rotational speed control of the magnet can be achieved by controlling the operating current or operating voltage of the corresponding motor.

Different magnets use different rotational speeds to facilitate data decoupling. Therefore, in some embodiments, the rotational speeds of the magnets of multiple rotating magnet units are different from each other. In addition, the magnetic moment direction can also be used for data decoupling, i.e., there is a phase difference between the magnetic moment directions of different rotating magnet units. Therefore, the magnets of rotating magnet units with different initial magnetic moment directions can use the same rotational speed, and the phase difference in the magnetic moment direction is used for data decoupling. In this way, the drive control of the field emitter can be simplified. For example, for a field emitter including four rotating magnet units, assume that the initial magnetic moment directions of the first three rotating magnet units are perpendicular to each other, and the initial magnetic moment direction of the last rotating magnet unit is the same as the initial magnetic moment direction of any other rotating magnet unit. Then, the magnets of the first three rotating magnet units can use the same rotational speed (e.g., 20 Hz), and the magnet of the last rotating magnet unit uses a different rotational speed (e.g., 10 Hz).

It should be noted that when the motor cannot achieve a higher rotational speed, such as a maximum rotational speed of only 20 Hz. If the rotational speeds of the magnets of different rotating magnet units are all different, the speed difference between different magnets will be relatively small, which is not conducive to data decoupling between rotating magnet units with the same initial magnetic moment direction, and the complexity of speed control is relatively high. Controlling the magnets of rotating magnet units with different initial magnetic moment directions to generate the same rotational speed, and controlling the magnets of rotating magnet units with the same initial magnetic moment direction to generate different rotational speeds, can facilitate data decoupling, maintain positioning accuracy, and reduce the complexity of speed control.

It should be noted that before the field emitter operates, the initial magnetic moment direction of each rotating magnet unit can be adjusted so that the initial magnetic moment directions of some rotating magnet units are different. Thus, during the operation of the field emitter, the magnets of the rotating magnet units with different initial magnetic moment directions are controlled to generate the same rotational speed, thereby simplifying the drive control of the field emitter.

In some embodiments, different initial magnetic moment directions mean that the initial magnetic moment directions are perpendicular. Therefore, in this embodiment, the magnets of the rotating magnet units with perpendicular initial magnetic moment directions can be controlled to generate the same rotational speed.

It should be noted that there is no necessary relationship between step 1202 and step 1204; step 1202 and step 1204 can be executed independently.

FIG. 13 is an exemplary flowchart of a self-check method for a rotating magnet unit according to some embodiments of this specification. In some embodiments, the process 1300 shown in FIG. 13 may be executed by a processing device (e.g., processor 13) or an electro-magnetic transient simulator. As shown in FIG. 13, the process 1300 may include the following operations.

The self-check method for a rotating magnet unit disclosed in this embodiment can be applied to the rotating magnet unit including the self-check assembly described above.

Step 1302, controlling the drive assembly to drive the magnet to rotate at a preset rotational speed.

In some embodiments, a drive control signal can be sent to the drive assembly of the field emitter, so that the drive assembly drives the magnet to rotate at a preset rotational speed, generating a rated magnetic field.

Step 1304, acquiring a current time-varying magnetic field signal of the magnet.

The current time-varying magnetic field signal can be obtained by the self-check assembly detecting the magnetic field signal generated by the magnet during rotation.

Step 1306, determining a signal difference between the current time-varying magnetic field signal of the magnet and a reference time-varying magnetic field signal, and determining a self-check result of the rotating magnet unit based on the signal difference.

The reference time-varying magnetic field signal is the time-varying magnetic field signal that the self-check assembly should detect when the rotating magnet unit is operating normally and the magnet is rotating at the preset rotational speed. The reference time-varying magnetic field signal can be obtained by detecting, via the magnetic detection assembly, the magnetic field signal generated when the magnet rotates at the preset rotational speed in the initial state of the rotating magnet unit. For example, it can be a time-varying magnetic field signal obtained by simulating a normally rotating magnet unit; it can also be a time-varying magnetic field signal detected by the self-check assembly when the rotating magnet leaves the factory.

In some embodiments, the reference time-varying magnetic field signal can be obtained by detecting, via the self-check assembly, the magnetic field signal generated when the magnet rotates at the preset rotational speed in the initial state of the rotating magnet unit (it is necessary to ensure that only the magnetic field generated by the rotation of the magnet of this rotating magnet unit is present, without other interfering magnetic fields).

In some embodiments, the signal difference between the current time-varying magnetic field signal of the magnet and the reference time-varying magnetic field signal can be obtained in the following manner.

For example, the preset rotational speed can be between 10 Hz and 100 Hz. The self-check process mainly involves driving the magnet to rotate via the drive assembly, and during the rotation of the magnet, detecting the magnetic field signal generated by the magnet via the self-check assembly to obtain the current time-varying magnetic field signal of the magnet during this self-check process. The current time-varying magnetic field signal reflects the time-varying characteristics of the magnetic field generated by the current magnet at the self-check assembly. After obtaining the current time-varying magnetic field signal, it is compared with the reference time-varying magnetic field signal to determine the signal difference between the two. Then, the self-check result of the rotating magnet unit can be determined based on this signal difference.

In some embodiments, the operating state of the field emitter can be determined based on the self-check result of the rotating magnet unit determined by the signal difference. For example, a normal operating state, an abnormal operation, etc.

In this embodiment, a specific method for determining the reference time-varying magnetic field signal is provided. The initial state of the rotating magnet unit refers to the state after rigorous calibration of strength and magnetic moment direction. It can typically be the factory state, as the rotating magnet unit undergoes strict calibration of strength and magnetic moment direction before leaving the factory. At this point, a self-check is performed using the magnetic detection assembly, and the resulting reference time-varying magnetic field signal has good reference significance. The reference time-varying magnetic field signal corresponds to the calibration characteristics of the rotating magnet unit (the calibrated strength and magnetic moment direction of the magnet). If the subsequent time-varying magnetic field signal changes compared to the reference time-varying magnetic field signal, it indicates that the real-time characteristics of the rotating magnet unit have changed relative to the calibration characteristics. In some cases, changes in the characteristics of the rotating magnet unit can be determined based on changes in the time-varying magnetic field signal.

In this embodiment, the self-check assembly can detect the magnetic field signal generated by the magnet. By comparing the current time-varying magnetic field signal of the magnet with the reference time-varying magnetic field signal, it can be determined whether the rotating magnet unit is operating abnormally. This method can be used to perform a self-check on the rotating magnet unit before each use, or it can be used to perform self-checks regularly or irregularly. Through self-checking, problems with the rotating magnet unit can be discovered in a timely manner, preventing the field emitter from generating a time-varying magnetic field that deviates from the standard during operation, ultimately ensuring the positioning accuracy of the receiving device. This solves the problem that when an existing rotating magnet unit malfunctions, it can cause the field emitter to generate a time-varying magnetic field that deviates from the standard during operation, ultimately reducing the positioning accuracy of the receiving device.

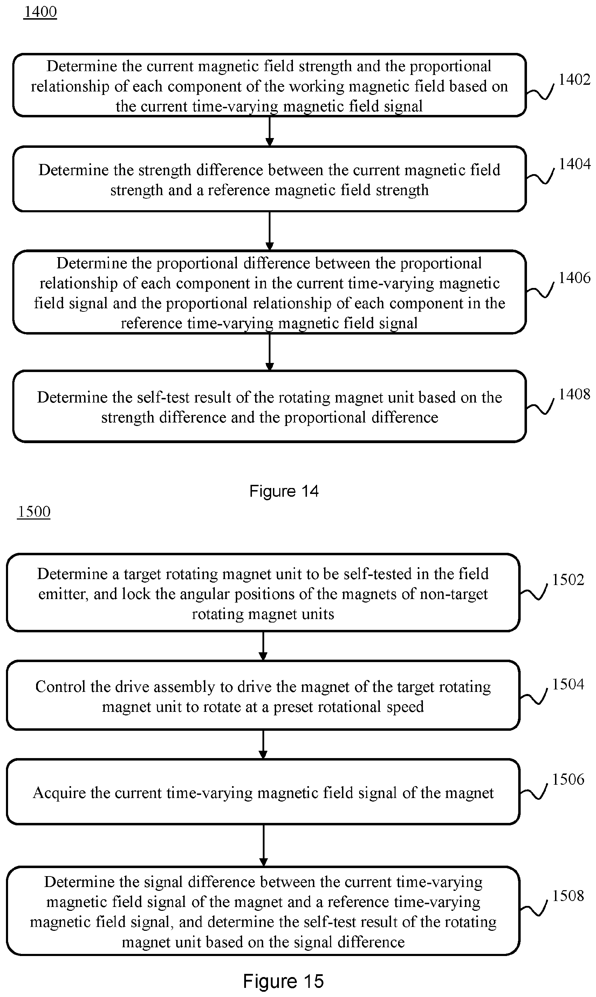

FIG. 14 is an exemplary flowchart for determining a self-check result according to some embodiments of this specification. In some embodiments, the process 1400 shown in FIG. 14 may be executed by a processing device (e.g., processor 13) or an electro-magnetic transient simulator. As shown in FIG. 14, the process 1400 may include the following operations.

In some embodiments, determining the signal difference between the current time-varying magnetic field signal of the magnet and the reference time-varying magnetic field signal may include the following operations.

Step 1402, determining a current magnetic field strength and a proportional relationship of each component of the working magnetic field based on the current time-varying magnetic field signal.

Step 1404, determining a strength difference between the current magnetic field strength and a reference magnetic field strength.

Wherein, the reference magnetic field strength can be determined based on the reference time-varying magnetic field signal.

Step 1406, determining a proportional difference between the proportional relationship of each component in the current time-varying magnetic field signal and the proportional relationship of each component in the reference time-varying magnetic field signal.

The difference between the current time-varying magnetic field signal and the reference time-varying magnetic field signal includes the strength difference and the proportional difference.

Step 1408, determining the self-check result of the rotating magnet unit based on the strength difference and the proportional difference.

During the implementation of the above steps, the current time-varying magnetic field signal output by the self-check assembly can first be obtained. The sampling rate of the self-check assembly can be 100Hz~1000Hz. For example, the current time-varying magnetic field signal is a magnetic field vector waveform (Bx(t), By(t), Bz(t)), which has corresponding components Bx(t), By(t), Bz(t) in three orthogonal directions. Then, based on the current time-varying magnetic field signal, the current magnetic field strength and the proportional relationship of each component are calculated. If the measured current magnetic field strength shows a significant proportional decrease relative to the reference magnetic field strength (the magnetic field strength corresponding to the reference time-varying magnetic field signal), for example, the decrease ratio (the ratio of the difference between the current magnetic field strength and the reference magnetic field strength to the reference magnetic field strength) is 5%, it fully indicates that the magnet strength has degraded by 5%. Then, the magnetic moment strength value |M| of this rotating magnet unit needs to be corrected in the subsequent navigation and positioning algorithm model. The proportional relationship of each component is Bx(t)∶ By(t)∶Bz(t). Wherein, each component includes multiple frequency components, and the proportional relationship of each component includes the proportional relationship between at least one identical frequency component of each component. Exemplarily, an information sequence over a period of time can be selected, and the intensity values of the main frequency components or the frequency components with stronger intensity of each component can be obtained through Fast Fourier Transform analysis: Bx1, Bx2..., By1, By2,..., and Bz1, Bz2,... Then, the proportional relationships between different frequency components of each component are obtained: Bx1 ∶ By1 ∶ Bz1, Bx2 ∶ By2 ∶ Bz2, ... During comparison, the ratio relationship of each frequency component of each component in the current time-varying magnetic field signal is compared with the ratio relationship of the corresponding frequency component of each component in the reference time-varying magnetic field signal. If the proportional relationship of each component of the self-check magnetic field changes beyond the limit, it may be that the magnetic moment direction of the magnet has changed, the mechanical structure has loosened, or there is interference in the surroundings (such as ferromagnetic materials, other time-varying magnetic fields, etc.). In this case, further inspection and repair of the rotating magnet unit is required. Wherein, when the ratio relationship of at least one frequency component of each component in the current time-varying magnetic field signal changes beyond the limit, it can be determined that the rotating magnet unit is abnormal. In some embodiments, for each sampling time point, a first comparison result between the current magnetic field strength and the reference magnetic field strength is determined, as well as a second comparison result between the ratio relationship of each frequency component of each component in the current time-varying magnetic field signal and the ratio relationship of the corresponding frequency component of each component in the reference time-varying magnetic field signal. Then, a first mean of the first comparison results and a second mean of the second comparison results for all sampling points are determined. If at least one of the first mean and the second mean exceeds the limit, it can be determined that the rotating magnet unit is abnormal. For example, when the mean of the ratio relationship of at least one frequency component of each component across all sampling time points changes beyond the limit, it can be determined that the rotating magnet unit is abnormal.

It should be noted that the above self-check process is an example of comparing the strength values of the current time-varying magnetic field signal and the reference time-varying magnetic field signal. In some embodiments, the strength value and the magnetic field direction of the current time-varying magnetic field signal can also be compared together.

FIG. 15 is an exemplary flowchart of a self-check method for a field emitter according to other embodiments of this specification. In some embodiments, the process 1500 shown in FIG. 15 may be executed by a processing device (e.g., processor 13) or an electro-magnetic transient simulator. As shown in FIG. 15, the process 1500 may include the following operations.

The field emitter self-check method disclosed in this embodiment can be applied to a field emitter where at least one rotating magnet unit includes a drive component, a magnet, and a self-check assembly.

Step 1502, determining a target rotating magnet unit to be self-checked in the field emitter, and locking (e.g., using a locking component) the angular position of the magnet of a non-target rotating magnet unit.

In some embodiments, before controlling at least one of the rotating magnet units to rotate at the preset rotational speed, one of the rotating magnet units in the field emitter is determined as a non-target rotating magnet unit; and, the angular position of the magnet of the non-target rotating magnet unit is locked.

Step 1504, controlling the drive component to drive the magnet of the target rotating magnet unit to rotate at a preset rotational speed.

Step 1506, acquiring a current time-varying magnetic field signal of the magnet.

Wherein, the current time-varying magnetic field signal is obtained by the self-check assembly detecting the magnetic field signal generated by the magnet during rotation.

Step 1508, determining a signal difference between the current time-varying magnetic field signal of the magnet and a reference time-varying magnetic field signal corresponding to the preset rotational speed, and determining the self-check result of the rotating magnet unit based on the signal difference.

Specifically, when the field emitter uses the rotating magnet unit provided in this specification, the self-check method of the rotating magnet unit provided in this specification can also be used to perform self-checks on each rotating magnet unit in the field emitter. That is, any embodiment of the self-check method for the rotating magnet unit described above can be used to perform self-checks on each rotating magnet unit in the field emitter. When performing a self-check on a target rotating magnet unit, interference from other magnetic fields needs to be eliminated. Therefore, it is necessary to turn off the non-target rotating magnet units in the field emitter, i.e., lock the angular positions of the magnets of the non-target rotating magnet units. Then, use the self-check method for the rotating magnet unit provided in this specification to perform a self-check on the target rotating magnet unit.

Through the above method, perform self-checks on each rotating magnet unit in the field emitter sequentially. Before each use of the field emitter, the user can perform a self-check on each rotating magnet unit through its internal self-check assembly. Through the self-check, problematic rotating magnet units can be discovered in a timely manner, preventing the field emitter from generating a time-varying magnetic field that deviates from the standard during operation, ultimately ensuring the positioning accuracy of the self-check assembly. This solves the problem that existing field emitters lack a self-check function. When a rotating magnet unit is abnormal, it cannot be discovered in time through self-check, causing the field emitter to generate a time-varying magnetic field that deviates from the standard during operation, ultimately reducing the positioning accuracy of the self-check assembly.

It should be noted that when the field emitter includes only one rotating magnet unit, the self-check result of each rotating magnet unit is the self-check result of that rotating magnet unit.

For more details on steps 1502 to 1504, please refer to the relevant description in Figure 13.

Some embodiments of this specification also disclose an electro-magnetic transient simulator. The electro-magnetic transient simulator includes a processor, a receiving device, and a field emitter including a drive assembly, a magnet, and a self-check assembly. The receiving device is used to detect the time-varying magnetic field generated by the field emitter. The processor is used to control the operation of the field emitter and determine the real-time pose of the receiving device in the time-varying magnetic field based on the magnetic field detection data from the receiving device.

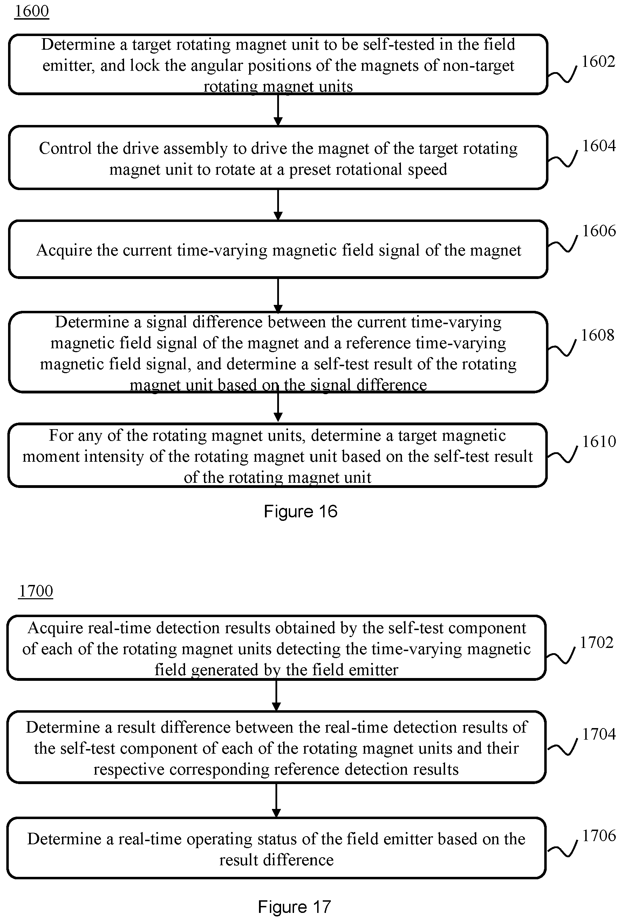

Figure 16 is an exemplary flowchart for determining a target magnetic moment strength according to some embodiments of this specification. In some embodiments, the process 1600 shown in Figure 16 can be executed by a processing device (e.g., processor 13) or an electromagnetic transient simulator. As shown in Figure 16, process 1600 may include the following operations.

In some embodiments, the electromagnetic navigation method may include performing a self-check on the field emitter to obtain self-check results for each rotating magnet unit in the field emitter. For example, the target magnetic moment strength of the rotating magnet unit can be determined. Exemplarily, it may include the following operations.

Step 1602, determine the target rotating magnet unit to be self-checked in the field emitter, and lock the angular positions of the magnets of the non-target rotating magnet units.

Step 1604, control the drive assembly to drive the magnet of the target rotating magnet unit to rotate at a preset speed.

Step 1606, obtain the current time-varying magnetic field signal of the magnet.

Wherein, the current time-varying magnetic field signal is obtained by the self-check assembly detecting the magnetic field signal generated by the magnet during rotation.

Step 1608, determine the signal difference between the current time-varying magnetic field signal of the magnet and a reference time-varying magnetic field signal, and determine the self-check result of the rotating magnet unit based on the signal difference.

Steps 1602 to 1608 are similar to the self-check process described above and will not be repeated here.

Step 1610, for any rotating magnet unit, determine the target magnetic moment strength of the rotating magnet unit based on the self-check result of the rotating magnet unit.

In some embodiments, the field emitter can be self-checked using the self-check method for the field emitter provided in this specification to obtain self-check results for each rotating magnet unit in the field emitter. For any rotating magnet unit, the target magnetic moment strength of the rotating magnet unit can be determined based on the operating status of the rotating magnet unit.

Specifically, for any abnormal operating state of a rotating magnet, the related problem can be solved by correcting the magnetic moment strength of that rotating magnet unit.

For example, for any rotating magnet unit, if the measured current magnetic field strength has decreased by a significant proportion relative to the reference magnetic field strength, then the magnetic moment strength of the corresponding rotating magnet unit is corrected in the navigation and positioning algorithm. For example, if the decrease ratio is 5%, it fully indicates that the magnet strength has degraded by 5%. Then, the magnetic moment strength value |M| of the rotating magnet unit needs to be corrected in the navigation and positioning algorithm model. For instance, reduce the magnetic moment strength by 5%, and the corrected magnetic moment strength becomes the target magnetic moment strength. Correspondingly, if the measured current magnetic field strength is the same as or similar to the reference magnetic field strength, i.e., the decrease ratio is less than a threshold, then there is no need to correct the magnetic moment strength of the rotating magnet unit, and the original magnetic moment strength becomes the target magnetic moment strength.

The field emitter in the electro-magnetic transient simulator provided in this specification has a self-check function. Before the system positions the receiving device, the user can perform a self-check on each rotating magnet unit in the field emitter through its internal self-check assembly. Through the self-check, problematic rotating magnet units can be discovered in a timely manner, and the magnetic moment strength of the corresponding rotating magnet unit can be corrected to keep the actual value of its time-varying magnetic moment characteristics consistent with the model value, preventing the field emitter from generating a time-varying magnetic field that deviates from the standard during operation, ultimately ensuring the positioning accuracy of the self-check assembly.

In some embodiments, determining the real-time pose of the receiving device in the time-varying magnetic field (electromagnetic navigation method) includes: obtaining magnetic field data of the time-varying magnetic field generated by the field emitter; wherein the magnetic field data of the time-varying magnetic field is obtained by the receiving device detecting the time-varying magnetic field; determining the real-time pose of the receiving device in the time-varying magnetic field based on the magnetic field data of the time-varying magnetic field and the time-varying characteristics of the time-varying magnetic field.

The receiving device refers to a magnetic sensor or a device containing a magnetic sensor. When the electro-magnetic transient simulator is operating, the field emitter provides a time-varying magnetic field, and the receiving device moves within the time-varying magnetic field. By using the magnetic field data detected by the magnetic sensor and the time-varying characteristics of the time-varying magnetic field, the pose of the receiving device can be located in real-time.

In some embodiments, the time-varying characteristics of the time-varying magnetic field include the time-varying magnetic moment characteristics of each rotating magnet unit in the field emitter in the field emitter coordinate system. The time-varying magnetic moment characteristics include the time-varying magnetic moment direction characteristics and the magnetic moment strength.

The electromagnetic navigation process is essentially a real-time positioning process for the receiving device. During real-time positioning, each rotating magnet unit rotates at a different frequency, and each receiving device to be positioned collects magnetic field data in real-time. Wherein, the receiving device is a magnetic sensor or a device containing a magnetic sensor.

Typically, the positioning algorithm requires two types of data input: the magnetic field data of each receiving device and the angle data sequence of the absolute position encoder of each rotating magnet unit within a time window. Wherein, the angle data sequence is used to determine the time-varying characteristics of the time-varying magnetic field; the time-varying characteristics of the time-varying magnetic field include the time-varying magnetic moment characteristics of each rotating magnet unit in the field emitter in the field emitter coordinate system.

The time-varying magnetic moment characteristics mainly include the time-varying magnetic moment direction characteristics and the magnetic moment strength. The magnetic moment strength is assumed to remain constant within that time window, while the angle data sequence of the absolute position encoder can determine the time-varying magnetic moment direction characteristics.

After the two types of data are input, clock alignment of the two data sets is performed, meaning the algorithm requires the magnetic field data at each moment and the magnetic moment direction of each rotating magnet unit at that moment. These two types of data can be input into a nonlinear optimization solver or a Kalman filter for calculation. The objective function for the optimization solution is shown in formula (2): ${arg min}_{{P}_{x} , {R}_{x}} {\sum }_{t} {\left‖{\sum }_{i} f\left({M}_{i}\left(t\right),{P}_{i}\left(t\right),{P}_{x}\right)-{R}_{x}{B}_{x}\left(t\right)\right‖}^{2}$

Among them, and are the magnetic moment vector and position coordinates of the i-th rotating magnet unit 100 in the field emitter body coordinate system at time t, respectively; is the position coordinate of the magnetic transducer in the field emitter body coordinate system; is the rotation matrix of the magnetic transducer relative to the field emitter body coordinate system; is the expression of the combined magnetic field vector collected by the magnetic transducer in its own coordinate system. Therefore, the above optimization goal is to determine the optimal transducer position and attitude to minimize the difference between the magnetic field model and the measured combined magnetic field.

It should be noted that the above solution algorithm does not require separation of the magnetic field; it can directly use the measured value of the combined magnetic field and the combined magnetic field model.

In some embodiments, determining the real-time pose of the receiving device in the time-varying magnetic field based on the magnetic field data of the time-varying magnetic field and the time-varying characteristics of the time-varying magnetic field includes: calculating the pose of the receiving device in the time-varying magnetic field in real time based on the magnetic field data of the time-varying magnetic field within a real-time time window and the time-varying characteristics of the time-varying magnetic field; wherein the window width of the real-time time window is determined based on the real-time requirements for calculating the pose of the receiving device.

Specifically, the navigation and positioning algorithm requires data within a real-time time window to locate the receiving device, but there is no quantitative relationship constraint between the width of this time window and the rotation period of the rotating magnet unit. The window width can be adjusted based on the actual signal-to-noise ratio and real-time requirements. When real-time requirements are not high, the width of the sliding real-time time window can be longer, resulting in a higher signal-to-noise ratio for the solution; when real-time requirements are relatively high, the width of the sliding real-time time window needs to be shortened, which may cause some "jitter" in the positioning result, because the data signal-to-noise ratio decreases, and stronger noise leads to a larger variance in the positioning result.

Additionally, the rotation speed of the rotating magnet unit can also be adjusted. Higher frequency time-varying magnetic fields are more likely to induce eddy currents in metals, thereby affecting the magnetic field of the surrounding environment and reducing positioning accuracy. Therefore, the rotation speed can be appropriately reduced to improve the system's antiinterference capability. However, in scenarios with high real-time requirements, the rotation speed should not be too low, otherwise the positioning speed will be low.

FIG. 17 is an exemplary flowchart for determining the real-time operating status of a field emitter according to some embodiments of this specification. In some embodiments, the process 1700 shown in FIG. 17 can be executed by a processing device (e.g., processor 13) or an electromagnetic transient simulator. As shown in FIG. 17, the process 1700 may include the following operations.

Step 1702, obtaining real-time detection results from the self-check assemblys of each of the rotating magnet units detecting the time-varying magnetic field generated by the field emitter.

In some embodiments, when performing electromagnetic navigation of the receiving device, each rotating magnet unit operates according to its respective rotation frequency. During this process, the self-check assemblys of each rotating magnet unit can detect and acquire magnetic field signals in real time, obtaining the measured detection magnetic field data for each self-check assembly.

Step 1704, determining the degree of result difference between the real-time detection results of the self-check assemblys of each of the rotating magnet units and their corresponding reference detection results.

Since the operating characteristics of each rotating magnet unit are known, such as the relative positions between the rotating magnet units, as well as the time-varying characteristics of the magnetic moment intensity and magnetic moment direction of each rotating magnet unit, the reference detection magnetic field data for each magnetic detection assembly can be pre-calculated and determined. If the measured detection magnetic field data of each component differs significantly from its respective reference detection magnetic field data, exceeding a preset threshold, it indicates that magnetic field interference (e.g., ferromagnetic material interference) is likely present in the surrounding area, requiring troubleshooting.

In some embodiments, before leaving the factory, several sets of operating parameters for the field emitter can be set. For example, each set of parameters includes the operating rotation frequency of each magnet. For each set of parameters, a reference detection result is determined.

Step 1706, determining the real-time operating status of the field emitter based on the degree of result difference.

The real-time operating status can be the current operating status of the field emitter. The field emitter has multiple rotating magnet units, each with its own degree of result difference. In some embodiments, if the degree of result difference of any one rotating magnet unit is greater than a preset threshold, it can be determined that the field emitter is in an abnormal operating state. Correspondingly, the degree of result difference of each rotating magnet unit can be compared using the same preset threshold, or a different preset threshold for the degree of result difference can be set for each rotating magnet unit.

It should be noted that the detection process in the above steps is different from the self-check process of the field emitter (e.g., the self-check method in FIG. 15). The self-check of the field emitter is performed before real-time positioning of the receiving device, and at the same time, a single rotating magnet unit is self-checked while other rotating magnet units are turned off. The detection in the above steps is performed during the electromagnetic navigation of the receiving device, where each rotating magnet unit is operating, and their respective self-check assemblys acquire magnetic field signals simultaneously.

In FIG. 15, the difference between the current time-varying magnetic field signal and the reference time-varying magnetic field signal corresponding to the preset rotation speed is represented by the signal difference degree, while in FIG. 17, the result difference degree is used. The method for obtaining the two difference degrees is the same, the only difference being the terminology used. Therefore, for further explanation of FIG. 17, reference can be made to the relevant description of FIG. 15. For example, regarding the real-time detection result, reference can be made to the description of the current time-varying magnetic field signal in FIG. 15; regarding the reference detection result, reference can be made to the description of the reference time-varying magnetic field signal in FIG. 15.

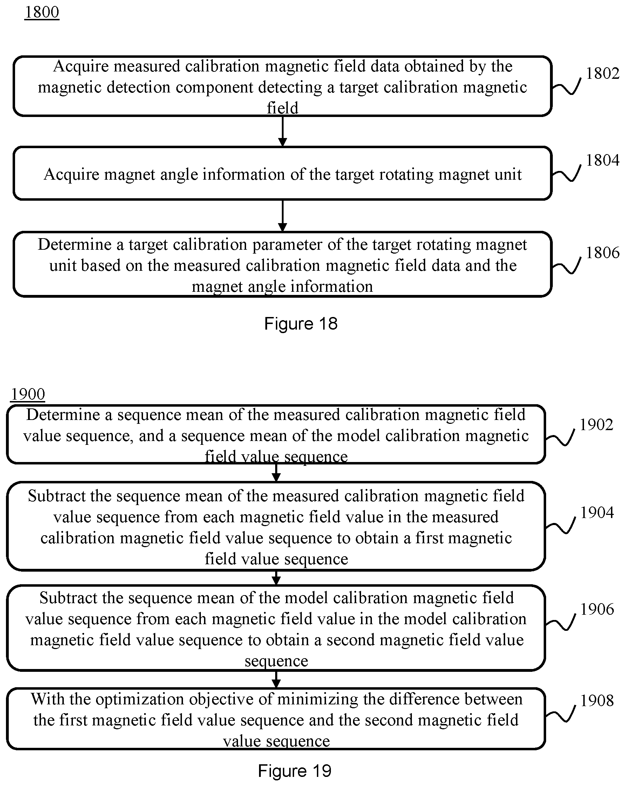

FIG. 18 is an exemplary flowchart of a self-calibration method for a field emitter according to some embodiments of this specification. In some embodiments, the process 1800 shown in FIG. 18 can be executed by a processing device (e.g., processor 13) or an electromagnetic transient simulator. As shown in FIG. 18, the process 1800 may include the following operations.

The self-calibration method for a field emitter disclosed in this embodiment can be applied to a field emitter including a field emitter unit group and a magnetic detection assembly. In some embodiments, the self-calibration method for the field emitter may include the following operations.

Step 1802: Obtain measured calibration magnetic field data obtained by the magnetic detection assembly detecting a target calibration magnetic field.

Wherein, the target calibration magnetic field is a calibration magnetic field generated by a target angulation magnet unit, and the target angulation magnet unit is the angulation magnet unit to be calibrated in the field emitter unit group.

Step 1804: Obtain magnet angle information of the target angulation magnet unit.

Step 1806: Determine target calibration parameters of the target angulation magnet unit based on the measured calibration magnetic field data and the magnet angle information.

In some embodiments, to improve calibration accuracy and simplify subsequent data processing, each angulation magnet unit may be calibrated individually, i.e., calibrating a single angulation magnet unit sequentially, or multiple or all angulation magnet units may be calibrated simultaneously.

For example, first, a target angulation magnet unit to be calibrated may be determined from the angulation magnet unit group, and the target angulation magnet unit is operated to generate a time-varying target calibration magnetic field. To prevent other angulation magnet units from affecting the calibration result of the target angulation magnet unit, the non-target angulation magnet units need to be turned off; after being turned off, the angulation magnet units will not generate a time-varying magnetic field. Then, the magnetic transducer in the magnetic detection assembly is used to detect the target calibration magnetic field to obtain corresponding measured calibration magnetic field data, and the magnet angle information output by the absolute position encoder in the target angulation magnet unit is obtained; wherein, the magnet angle information refers to the angle information of the reference axis of the electric motor driving the magnet, and the magnetic moment direction of the magnet can be determined through this angle information.Finally, based on the measured calibration magnetic field data and the magnet angle information, the target parameters of the target rotating magnet unit can be calibrated to obtain the target calibration parameters. The target calibration parameters are calibration parameters that conform to the actual state of the rotating magnet unit.

It should be noted that when the rotating magnet unit group includes only one rotating magnet unit, that rotating magnet unit is the target rotating magnet unit to be calibrated. Furthermore, during the self-calibration process, since there are no non-target rotating magnet units, there is no need to deactivate any non-target rotating magnet units. The other steps for calibrating this target rotating magnet unit can be the same as those described above.

In some embodiments, determining the target calibration parameters of the target rotating magnet unit based on the current time-varying magnetic field signal and the magnet angle information includes: determining the model calibration magnetic field data of the target calibration magnetic field at the magnetic detection assembly based on the magnet angle information, the model parameters of the target rotating magnet unit, and the spatial pose of the magnetic detection assembly; optimizing the model parameters of the target rotating magnet unit with the optimization objective of minimizing the difference between the measured calibration magnetic field data and the model calibration magnetic field data, to obtain the target calibration parameters of the target rotating magnet unit.

Here, the model parameters of the target rotating magnet unit refer to the current parameters of the target rotating magnet unit in the magnetic field model. These current parameters may be obtained from calibration when the field generator left the factory, or from the last self-calibration of the field generator. As the field generator is used, the usage state of each rotating magnet unit changes, causing the model parameters of the target rotating magnet unit to potentially not conform to its actual state. Therefore, it is necessary to perform optimization calibration on them to obtain target calibration parameters that conform to the actual state of the target rotating magnet unit.

Here, based on the magnet angle information, the model parameters of the target rotating magnet unit, and the spatial pose of the magnetic detection assembly, the model calibration magnetic field data of the target calibration magnetic field at the magnetic detection assembly can be calculated using a magnetic field model. Here, the magnetic field model can be a Dipole magnetic field model. If the model calibration magnetic field data is the same as the measured calibration magnetic field data, it indicates that the model parameters of the target rotating magnet unit conform to its actual state; if the model calibration magnetic field data is different from the measured calibration magnetic field data, it indicates that the model parameters of the target rotating magnet unit do not conform to its actual state, and calibration correction of the model parameters of the target rotating magnet unit is required. At this point, minimizing the difference between the measured calibration magnetic field data and the model calibration magnetic field data can be set as the optimization objective. The model parameters of the target rotating magnet unit are iteratively optimized, and the final model parameters obtained are taken as the target parameters.

It should be noted that in embodiments where the magnetic detection assembly includes multiple magnetic sensors, the measured calibration magnetic field data and the model calibration magnetic field data corresponding to different magnetic sensors are different; furthermore, since the calibration magnetic field generated by the rotating magnet unit is a time-varying magnetic field, the measured calibration magnetic field data and the model calibration magnetic field data corresponding to different time instants are also different. Therefore, when taking minimizing the difference between the measured calibration magnetic field data and the model calibration magnetic field data as the optimization objective, the differences between the measured calibration magnetic field data and the model calibration magnetic field data corresponding to different magnetic sensors and different time instants can be calculated first, and minimizing the mean of the above differences can be taken as the optimization objective.

In some embodiments, the target parameters of the target rotating magnet unit include the target spatial position of the target rotating magnet unit.

Illustratively, first, the magnetic moment of the target rotating magnet unit is determined based on the magnet angle information; then, based on the magnetic moment of the target rotating magnet unit, the model spatial position of the target rotating magnet unit, and the spatial position of the magnetic sensor, the model calibration magnetic field data of the target calibration magnetic field at the magnetic sensor is determined; finally, with the optimization objective of minimizing the difference between the measured calibration magnetic field data and the model calibration magnetic field data, the model spatial position of the target rotating magnet unit is optimized to obtain the target spatial position of the target rotating magnet unit. Here, the magnetic moment of the target rotating magnet unit refers to the magnetic moment of the magnet within the target rotating magnet unit, including the magnetic moment direction and magnetic moment strength. The model spatial position of the target rotating magnet unit refers to the spatial position of the magnet center of the target rotating magnet unit. Specifically, the optimization formula (3) is as follows: ${arg min}_{{P}_{TxU}} {\sum }_{t} {\sum }_{i} {\left‖f\left(M\left(t\right),{P}_{TxU},{P}_{c i}\right)-{B}_{c}\left(i, t\right)\right‖}^{2}$

Here, *f*(*x*) is the magnetic field model, **M**(*t*) is the magnetic moment vector of the target rotating magnet unit at time t, **P**_{TxU} is the model position coordinate of the magnet center of the target rotating magnet unit in the emitter body coordinate system; **P_{*c*i}** is the position coordinate of the i-th magnetic sensor in the magnetic detection assembly in the field generator body coordinate system; then *f*(**M**(*t*)*,* **P**_{TxU}, **P**_{*c*i}) is the model calibration magnetic field data (model magnetic field vector) of the target rotating magnet unit at the i-th magnetic sensor; **B***_{c}*(*i,t*) is the measured calibration magnetic field data (measured magnetic field vector) measured by the i-th magnetic sensor in the magnetic detection assembly at time t.

In some embodiments, the target parameters of the target rotating magnet unit include at least one of the following parameters: the target reference axis geometric parameter of the target rotating magnet unit, the target magnet zero-position homogeneous transformation matrix, and the target magnetic moment strength.

Specifically, the reference axis geometric parameter refers to a geometric parameter related to the reference axis. Illustratively, it includes the reference axis screw, which characterizes the offset of the magnet's center of mass relative to the reference axis. The magnet zero-position homogeneous transformation matrix refers to the pose transformation between the actual zero-position direction of the magnet's magnetic moment and the target zero-position direction. For example, under standard design, the magnet's center of mass needs to be on its own reference axis, and the actual zero-position direction of the magnet's magnetic moment is the target zero-position direction. However, in an actual field generator, there are errors in the above design requirements. These errors are characterized by the reference axis screw and the magnet zero-position homogeneous transformation matrix. In the magnetic field model, it is necessary to calibrate the target reference axis geometric parameters and the magnet zero-position homogeneous transformation matrix, thereby improving the accuracy of electromagnetic navigation.

First, based on the model reference axis geometric parameters, the model magnet zero-position homogeneous transformation matrix, and the model magnetic moment strength of the target rotating magnet unit, as well as the magnet angle information and the spatial position of the magnetic sensor, the model calibration magnetic field data of the target calibration magnetic field at the magnetic sensor is determined; then, with the optimization objective of minimizing the difference between the measured calibration magnetic field data and the model calibration magnetic field data, the model reference axis geometric parameters, the model magnet zero-position homogeneous transformation matrix, and the model magnetic moment strength of the target rotating magnet unit are optimized to obtain the target reference axis geometric parameters, the target magnet zero-position homogeneous transformation matrix, and the target magnetic moment strength of the target rotating magnet unit.

It should be noted that all of the above multiple target parameters can be calibrated, or some of the target parameters can be calibrated sequentially. Multiple target parameters can be calibrated simultaneously, or each target parameter can be calibrated sequentially.

Furthermore, taking minimizing the difference between the measured calibration magnetic field data and the model calibration magnetic field data as the optimization objective includes: taking minimizing the difference between the magnitude of the measured calibration magnetic field data and the magnitude of the model calibration magnetic field data as the optimization objective.

Specifically, considering that when installing magnetic sensors in the magnetic detection assembly, their positional accuracy is relatively easy to guarantee, but their orientation accuracy is relatively difficult to guarantee. Therefore, when calculating the difference between the measured calibration magnetic field data and the model calibration magnetic field data, if the vector difference of the two sets of magnetic field data is calculated, uncertain errors may be introduced. Since the magnitude of the magnetic field data is independent of the orientation of the magnetic sensor and is only related to the position of the magnetic sensor, calculating the scalar difference between the two sets of magnetic field data, i.e., the difference in the magnitudes of the two sets of magnetic field data, can improve calibration accuracy.

Specifically, when the target parameters of the target rotating magnet unit include the target reference axis screw, the target magnet zero-position homogeneous transformation matrix, and the target magnetic moment strength of the target rotating magnet unit, its optimization formula (4) is as follows: ${arg min}_{s , {T}_{0} , \left|m\right|} {\sum }_{i} {\sum }_{n} {\left[\left‖f\left({e}^{\left[S\right] θ \left(n\right)}{T}_{0},\left|m\right|,{P}_{ai}\right)\right‖-\left‖B i b\left(n\right)\right‖\right]}^{2}$

Where, **T₀** is the homogeneous transformation matrix of the model magnet zero position of the target rotating magnet unit, *θ*(*n*) is the angle of the magnet reference axis of the target rotating magnet unit at *t = n*Δ*Tₛ,* S is the model reference axis screw of the target rotating magnet unit, *e*^{[S]*θ*(*n*)} is the exponential expression of the homogeneous transformation matrix, then **T**ₙ = *e*^{[*S*]*θ*(*n*)}**T**₀ represents the *t* = *n*Δ*Tₛ* real-time homogeneous transformation matrix of the model magnet of the target rotating magnet unit at time, Δ*Tₛ* is the sampling interval of the sensor, such as 1ms~10ms; |**m**| is the magnetic strength of the target rotating magnet unit, **P**ₐᵢ is the position coordinate of the i-th magnetic sensor in the magnetic detection assembly in the field emitter body coordinate system; ∥*f*(^{*e*[s]*θ*(*n*}**⁾T**₀,|**m**|, **P**ₐᵢ)∥ represents the modulus of the model calibration magnetic field data of the magnet of the target rotating magnet unit at **P**ₐᵢ at *t = n*Δ*Tₛ*; ∥*^{b}***B**ᵢ(**n**)∥ is the modulus of the measured calibration magnetic field data measured by the i-th magnetic sensor in the magnetic detection assembly at *t = n*Δ*Tₛ .*

Meanwhile, according to the above optimization formula, when calibrating only one or two parameters, it is only necessary to treat the non-target parameters as fixed values in the optimization formula.

After determining the target parameter, the degree of difference between the target parameter and the reference parameter can also be determined, and it is judged whether the degree of difference exceeds a threshold; if so, the target rotating magnet unit corresponding to the target parameter needs maintenance; if not, update the parameter corresponding to the target rotating magnet unit.

The reference parameter can be determined when the field emitter leaves the factory. For example, by calibrating the field emitter at the factory, parameter information in the initial state can be obtained, and this parameter information can be recorded and used as a reference parameter.

Figure 19 is an exemplary flowchart for determining an optimization objective according to some embodiments of this specification. In some embodiments, the process 1900 shown in Figure 19 can be executed by a processing device (e.g., processor 13) or an electromagnetic transient simulator. As shown in Figure 19, process 1900 may include the following operations.

In some embodiments, the measured calibration magnetic field data includes a measured calibration magnetic field value sequence, and the model calibration magnetic field data includes a model calibration magnetic field value sequence; the optimization objective of minimizing the difference between the measured calibration magnetic field data and the model calibration magnetic field data may include the following operations.

Step 1902, determine the sequence mean of the measured calibration magnetic field value sequence and the sequence mean of the model calibration magnetic field value sequence.

Step 1904, subtract the sequence mean of the measured calibration magnetic field value sequence from each magnetic field value in the measured calibration magnetic field value sequence to obtain a first magnetic field value sequence.

Step 1906, subtract the sequence mean of the model calibration magnetic field value sequence from each magnetic field value in the model calibration magnetic field value sequence to obtain a second magnetic field value sequence.

Step 1908, take minimizing the difference between the first magnetic field value sequence and the second magnetic field value sequence as the optimization objective.

Considering the existence of the environmental magnetic field, the magnetic field data detected by the magnetic detection assembly is essentially the combined magnetic field of the calibration magnetic field and the environmental magnetic field. This embodiment removes the influence of the static environmental magnetic field during the calibration process, improving calibration accuracy.

The calibration magnetic field provided by the rotating magnet unit can be divided into a time-varying part and a time-invariant part. Among them, the periodic mean of the time-varying part is zero, and the mean of the time-invariant part is itself. Therefore, the measured data mean of the measured calibration magnetic field data represents the measured data mean of the time-invariant part of the calibration magnetic field and the measured data mean of the environmental magnetic field. Specifically, since data is continuously acquired, the measured calibration magnetic field data includes a measured calibration magnetic field value sequence, and the model calibration magnetic field data includes a model calibration magnetic field value sequence. The sequence mean of the measured calibration magnetic field value sequence is the measured data mean of the time-invariant part of the calibration magnetic field and the measured data mean of the environmental magnetic field, and the sequence mean of the model calibration magnetic field value sequence is the model data mean of the time-invariant part of the calibration magnetic field. After subtracting the sequence mean from each magnetic field value in the sequence, each magnetic field value becomes the magnetic field value of the time-varying part of the calibration magnetic field. Furthermore, the first magnetic field value sequence is the measured data of the time-varying part of the calibration magnetic field, and the second magnetic field value sequence is the model data of the time-varying part of the calibration magnetic field. Furthermore, when calculating the difference between the first magnetic field value sequence and the second magnetic field value sequence, the influence of the environmental magnetic field is eliminated. Specifically, since the magnetic field values in the first magnetic field value sequence and the second magnetic field value sequence correspond one-to-one (determined based on acquisition time), calculating the difference between the two sequences can be the difference between multiple groups of corresponding magnetic field values, and the cumulative difference is taken as the difference between the two sequences.

This embodiment provides a self-calibration method for a field emitter. The following is an explanation of the principle of the self-calibration method for the field emitter.

Due to machining errors, installation errors, wear and tear during use, collisions, etc., there are errors between the relevant parameters of the magnet in the field emitter and the model parameters in the algorithm model, which can lead to a decrease in positioning accuracy.

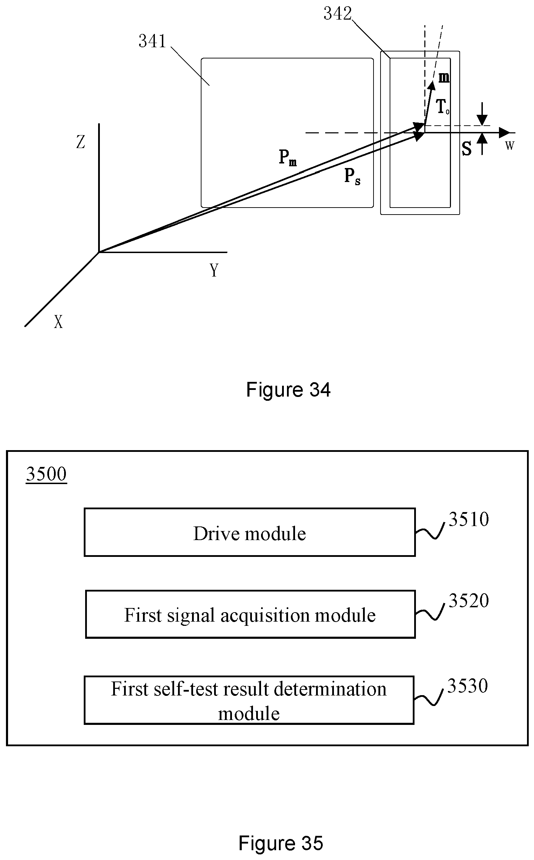

Figure 34 is a schematic structural diagram of a rotating magnet unit according to some embodiments of this specification. Referring to Figure 34, the rotating magnet unit includes a motor 341 and a permanent magnet 342. It is assumed that the design requires that in the rotating magnet unit, the magnetic center of mass of the permanent magnet is located on the motor shaft w, and when the motor is at the zero position (measured by an absolute position encoder), the magnetic moment direction of the magnet is along the positive direction of the Z-axis (based on the coordinate system {s}). However, the actual situation may be that the magnetic center of mass deviates from the motor shaft w, and when the motor is at the zero position, the magnetic moment direction of the magnet deviates from the positive direction of the Z-axis. The reference axis screw S and the homogeneous transformation matrix **T₀** of the magnet at the zero position (considering the magnetic moment as an active vector, starting at the magnetic center of mass, with the direction being the magnetic moment direction) are expressed in the coordinate system {s} as formula (5): $\begin{matrix}S = {\left[{ω}^{⏜},-{ω}^{⏜}\times {P}_{s}\right]}^{T} \\ {T}_{0} = \left[\begin{matrix}{R}_{m} & {P}_{m} \\ 0 & 1\end{matrix}\right]\end{matrix}$

Where, **ω̂** represents the unit vector of the rotation axis direction or angular velocity, **Rₘ** is the rotation matrix of the magnet, and Pm and Ps represent the magnetic center of mass and a point on the reference axis (the point where the magnetic center of mass should be located), respectively. From the above error description, it can be seen that at this time, **Pₛ** ≠ **Pₘ, Rₘ(:,3) ≠ ẑ**. In addition to the magnetic moment direction, the magnetic moment strength of the magnet |**m**| may also change. Therefore, the self-calibration method of the field emitter provided in this specification is to calibrate the offset **S** between the magnetic center of mass and the motor shaft w, the homogeneous transformation matrix **T₀** of the magnet at the zero position, and the magnetic moment strength |**m**| of the magnet. Among them, **S** and **T₀** are both six-dimensional parameters.

It should be noted that in this specification, the left superscript of all physical quantities indicates the coordinate system in which the vector is expressed. If not explicitly shown in the text, coordinates, rotation matrices, vectors, etc., are expressed in the coordinate system {s}.

Assume that N magnetic sensors (magnetic detection assemblys) are distributed at fixed positions in the field emitter. Here, a 3-axis MEMS sensor is taken as an example. The position **Pₐᵢ** of the magnetic sensor in the coordinate system {s} is known (e.g., error ≤ 0.01mm, significantly higher than the navigation positioning requirement). Keep other rotating magnet units inactive, and only make one rotating magnet unit rotate at a relatively low speed each time to generate a calibration magnetic field, such as 1Hz~10Hz. The system acquires the angle information *θ*(*t*) of the absolute position encoder and the measured calibration magnetic field data **^{b}Bᵢ(t)** of each magnetic sensor in real time, *i* =1*,*2*,...,N.* **^{b}Bᵢ(t)** is the projection of the calibration magnetic field onto the magnetic sensor's own coordinate system {b}. The above physical quantities to be calibrated can be solved using an optimization method through an objective function.

It should be noted that when the sensor is mounted on a patch, the position accuracy is easy to guarantee, but its attitude is difficult to guarantee. Therefore, in this embodiment, the difference in the modulus of the magnetic field data is calculated.

It is difficult to ensure that the axis direction inside the sensor is completely parallel to the actual design direction, resulting in a pose transformation between the magnetic sensor's own coordinate system {b} and the field emitter body coordinate system {s}, i.e., **^{b}Bᵢ(n)** ≠^{s} **Bᵢ(n).** If vectors are used for comparison, some uncertain errors will be introduced. However, since the magnitude of the magnetic field data is independent of the attitude of the magnetic sensor and only related to its position, that is ∥^{b}**B**ᵢ(**n**)∥ = ∥*^{s}***B**ᵢ(**n**)∥. The position of the magnetic sensor can be accurately measured using a coordinate measuring machine, so using the magnitude of the magnetic field data can reduce the introduction of errors.

To further improve calibration accuracy, the above objective function can be further optimized. For MEMS sensors, the measured value ^{b}**B**ᵢ(**n**) is the combined magnetic field, which is the combination of the calibration magnetic field generated by the rotating magnet unit and the environmental magnetic field. Specifically, as shown in formula (6): $\begin{matrix}B i b \left(n\right) \\ = R i s b \left({B}_{i - pm}\left(n\right)+{B}_{i - g}\right) + {B}_{i - d} \\ = R i s b \left({B}_{i - pmac}\left(n\right)+{B}_{i - pmdc}+{B}_{i - g}\right) + {B}_{i - d} \\ = R i s b {B}_{i - pmac} \left(n\right) + R i s b \left({B}_{i - pmdc}+{B}_{i - g}\right) + {B}_{i - d}\end{matrix}$

Where, $R i s b$ is the inverse of the rotation matrix of the i-th magnetic sensor, representing the transformation from coordinate system {s} to coordinate system {b}; **Bᵢ₋ₚₘ (n)** is the magnetic field contributed by the rotating magnet unit, which can be further divided into a time-varying part **B_{i-pmac} (n)** and a time-invariant part **B_{i-pmdc}** ; **B_{i-g}** is the environmental magnetic field, such as the geomagnetic field and static magnetic fields generated by other materials and equipment in the environment; **B_{i-d}** is the zero-drift parameter of the sensor, which does not change with attitude. Due to the complex and unknown environment, other stationary units also contribute static magnetic fields, so **B_{i-g}** it is a quantity that cannot be ignored and cannot be modeled. In this embodiment, the mean subtraction method is used to remove the environmental magnetic field. Specifically, as shown in formula (7): $\begin{matrix}B i - deavg b \left(n\right) \\ = B i b \left(n\right) - \frac{1}{{N}_{T}} {\sum }_{n} B i b \left(n\right) \\ = R i s b {B}_{i - pmac} \left(n\right)\end{matrix}$

Where, *N_{T}* is the number of sampling points within a fixed time. Assuming the fixed time is 1s and a rotation speed of 10Hz corresponds to a sampling rate of 800Hz, then 1s corresponds to 10 rotations, *N_{T}* = 800. The meaning of the above formula is to first collect a series of magnetic field data, calculate their mean, and then subtract this mean ${\sum }_{n} B i b \left(n\right) / {N}_{T}$ from the magnetic field data sequence one by one. Since the magnetic field contribution of the rotating magnet unit is divided into a time-varying part and a time-invariant part, the periodic mean of the time-varying part is zero, the mean of the time-invariant part is itself, and the mean of the environmental magnetic field is also itself. Therefore, after mean subtraction, only the time-varying part $R i s b {B}_{i - pmac} \left(n\right)$ of the magnetic field contribution from the rotating magnet unit remains, reflecting the characteristics of the magnet's rotation. At the same time, optimize *fe*^{[s]*θ*(*n*)}T₀,|m|, Pₐᵢ) so that after calculating the theoretical values, the mean of all theoretical values is calculated, and finally, the sequence mean is also subtracted from each theoretical value. The optimized objective function is as shown in the following formula (8): ${arg min}_{S , {T}_{0} , \left|m\right|} {\sum }_{i} {\sum }_{n} {\left[\left‖{f}_{deavg}\left({e}^{\left[S\right] θ \left(n\right)}{T}_{0},\left|m\right|,{P}_{ai}\right)\right‖-\left‖B i - deavg b\left(n\right)\right‖\right]}^{2}$

Some embodiments of this specification provide a field emitter, which may include a field emitter unit group and a magnetic source assembly. The field emitter unit group includes a plurality of rotating magnet units; the spatial pose of the magnetic source assembly within the field emitter is fixed; when the field emitter unit group stops working, the magnetic source assembly generates a detection magnetic field for performing interference detection on the field emitter.

The magnetic source assembly may include a plurality of coils, and the plurality of coils are evenly distributed around the periphery of the field emitter unit group.

Based on this field emitter, some embodiments of this specification also provide an interference detection method for a field emitter. Additionally, the interference detection method for the field emitter can also be applied to the field emitter including the magnetic source assembly disclosed above.

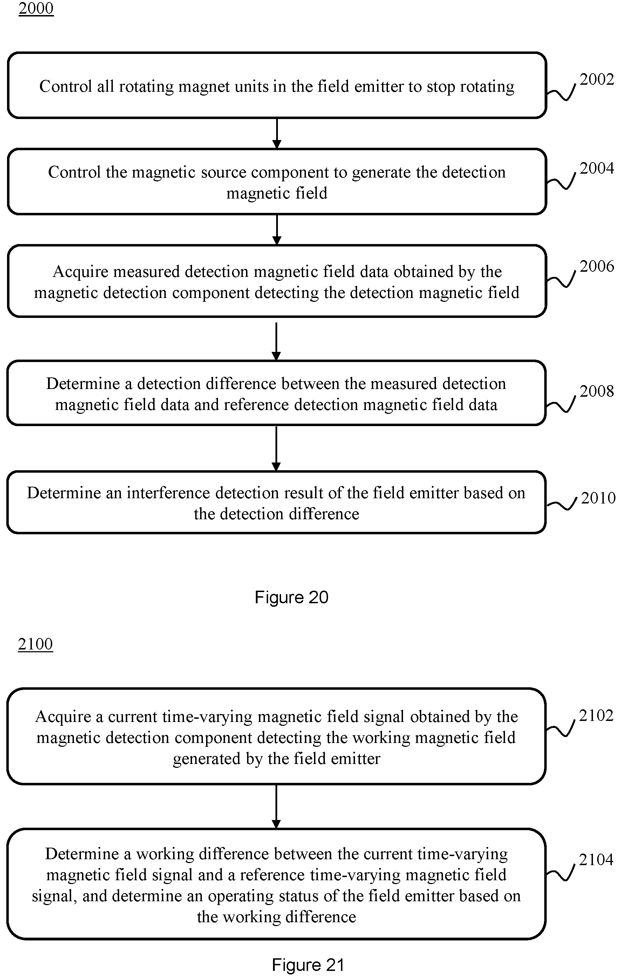

FIG. 20 is an exemplary flowchart of an interference detection method for a field emitter according to some embodiments of this specification. In some embodiments, the process 2000 shown in FIG. 20 may be executed by a processing device (e.g., processor 13) or an electro-magnetic transient simulator. As shown in FIG. 20, the process 2000 may include the following operations.

Step 2002, control all rotating magnet units in the field emitter to stop rotating.

Step 2004, control the magnetic source assembly to generate the detection magnetic field.

Step 2006, obtain the measured detection magnetic field data obtained by the magnetic detection assembly detecting the detection magnetic field.

In some embodiments, the magnetic source assembly includes a plurality of coils, the coils are used to generate a detection magnetic field, and the plurality of coils are evenly distributed around the periphery of the rotating magnet unit group. The obtaining the measured detection magnetic field data obtained by the magnetic detection assembly detecting the detection magnetic field includes: obtaining the measured detection magnetic field data obtained by the magnetic detection assembly detecting a target detection magnetic field. Wherein, the target detection magnetic field is a detection magnetic field generated by a target coil among the plurality of coils in the magnetic source assembly.

In this embodiment, the magnetic source assembly uses a plurality of coils, which can generate a detection magnetic field when energized. The detection magnetic field generated by the coil can be a sinusoidal signal. The frequency of the detection magnetic field can be set slightly higher than the operating frequency of the field emitter during electromagnetic navigation, such as 50Hz~100Hz. The coil has the characteristic of controllable magnetic field, generating a detection magnetic field when energized and not generating a detection magnetic field when de-energized.

Step 2008, determine the degree of detection difference between the measured detection magnetic field data and the reference detection magnetic field data.

Step 2010, determine the interference detection result of the field emitter based on the degree of detection difference.

In some embodiments, when interference detection is required, the coil is turned on to generate a detection magnetic field, and at other times, the coil is turned off so that it does not generate a detection magnetic field, preventing the detection magnetic field from affecting other functions of the field emitter. For example, when the magnetic source assembly generates the detection magnetic field, the plurality of rotating magnet units are turned off.

In some embodiments, the main function of the rotating magnet unit is to generate a time-varying magnetic field. To better detect the detection magnetic field, the rotating magnet units in the field emitter can be turned off during the detection process. Turning off the rotating magnet unit means stopping it from generating the time-varying magnetic field. For example, for a rotating magnet unit that uses a motor to drive a permanent magnet to rotate, turning off the rotating magnet unit means turning off the motor and locking the permanent magnet so that it cannot rotate; for a rotating magnet unit that uses electromagnetic coils, turning off the rotating magnet unit means putting the electromagnetic coil in a de-energized state.

After all rotating magnet units are turned off, the magnetic sensor in the magnetic detection assembly detects the detection magnetic field generated by the magnetic source assembly, and the measured detection magnetic field data of the magnetic sensor is read. Then, the measured detection magnetic field data is compared with the reference detection magnetic field data to determine the degree of detection difference between the two. Finally, the interference detection result of the field emitter is determined based on the degree of detection difference.

At the same time, there are multiple coils, which are evenly distributed around the periphery of the rotating magnet unit group, for example, they can be fixedly installed on various sides of the installation housing of the field emitter. Considering that the influence of a smaller magnetic field source on the magnetic field of the field emitter is local, for example, it only affects the magnetic field of the field emitter in a certain direction. Therefore, multiple coils are arranged around the field emitter, respectively used to detect whether there is an interference source in the corresponding direction. During detection, detection signals are generated one by one and the same phase comparison detection is performed, thereby comprehensively detecting the conditions around the field emitter.

Therefore, during the interference detection process, each coil is sequentially used as the target coil, and it is determined whether there is an interference source in the direction corresponding to the target coil. After the interference detection for the current target coil is completed, the next coil is determined as the new target coil, and the interference detection steps are repeated.

In some embodiments, determining the detection difference between the measured detection magnetic field data and the reference detection magnetic field data, and determining the interference detection result of the field emitter based on the detection difference, includes: determining a target detection difference between the target measured detection magnetic field data and the target reference detection magnetic field data, and determining the interference detection result of the field emitter in the direction corresponding to the target coil based on the target detection difference.

Here, the detection difference is primarily used to determine whether other magnetic field sources exist in the current environment. Therefore, the reference detection magnetic field data refers to the magnetic field data that the magnetic detection assembly should obtain when detecting the detection magnetic field in an environment without interference sources. Specifically, the reference detection magnetic field data can be obtained by detecting the detection magnetic field using the magnetic detection assembly during the factory calibration of the field emitter, because the calibration environment at the factory is controllable and can be ensured to be free of interference sources. Meanwhile, the reference detection magnetic field data can also be obtained through simulation calculation when the relative pose of the magnetic source component and the magnetic detection assembly is fixed and known.

Further, the measured detection magnetic field data is compared with the reference detection magnetic field data. The comparison method can be comparing the phase between the two, such as comparing peak positions, calculating correlation coefficients, etc. This comparison method can be applied to detect interference signals with the same frequency but different phases. Common examples include metal eddy currents which can generate interference signals with the same frequency but different phases. Therefore, the signal difference can be a phase difference. Correspondingly, the signal difference can also be a peak magnitude difference. Meanwhile, both the measured detection magnetic field data and the reference detection magnetic field data include several magnetic field data points sequenced by time, and there is a one-to-one correspondence between them. When comparing the two, it is sufficient to sequentially compare the magnetic field data with the same index.

In some embodiments, determining the interference detection result of the field emitter based on the detection difference includes: when the detection difference is greater than a preset threshold, determining that a magnetic field interference source exists around the field emitter. In this embodiment, it is not necessary for the measured detection magnetic field data to be exactly identical to the reference detection magnetic field data. Typically, a magnetic field interference source is considered to exist around the field emitter only when the detection difference is greater than the preset threshold. Further, the detection magnetic field data is usually a vector, which includes magnetic field component data in three spatial dimensions. Therefore, when comparing the measured detection magnetic field data with the reference detection magnetic field data, the magnetic field component data in each spatial dimension can be compared separately. For example, when the detection difference of the magnetic field component data in a certain spatial dimension is greater than the preset threshold, it can be determined that a magnetic field interference source exists around the field emitter. Correspondingly, it can also be determined that a magnetic field interference source exists around the field emitter only when the detection differences of the magnetic field component data in two or three spatial dimensions are all greater than the preset threshold.

The above description of the interference detection method takes the magnetic detection assembly detecting the detection magnetic field generated by the magnetic source component as an example. It can be understood that the interference detection method can also be applied to other detection components (e.g., a self-check assembly or other detection components besides the field emitter) detecting the detection magnetic field generated by the magnetic source component.

It should be noted that the self-calibration method, interference detection method, and anomaly detection method for the field emitter provided in some embodiments of this specification are related in their usage. Specifically, before each electromagnetic navigation using the field emitter, the interference detection method can be used first to detect the working environment of the field emitter and determine whether a magnetic field interference source exists around the field emitter. If there is one, it needs to be eliminated first. If there is none, or after the interference source is eliminated, the self-calibration method is used to self-calibrate the field emitter. When the model parameters of the field emitter do not match the actual state, the model parameters of the field emitter are updated in real time. After the self-calibration is completed, electromagnetic navigation is performed using the field emitter. During the electromagnetic navigation process, the anomaly detection method is used to detect the operating status of the field emitter in real time. Furthermore, after self-calibration and before electromagnetic navigation, the self-check assembly can also be used to perform a self-check method on the rotating magnet unit.

In view of this, some embodiments of this specification also provide an anomaly detection method for a field emitter.

FIG. 21 is an exemplary flowchart of an anomaly detection method according to some embodiments of this specification. In some embodiments, the process 2100 shown in FIG. 21 can be executed by a processing device (e.g., processor 13) or an electro-magnetic transient simulator. As shown in FIG. 21, the process 2100 may include the following operations.

The anomaly detection method for a field emitter disclosed in this embodiment can be applied to the field emitter described above, which includes a field emitter unit group and a magnetic detection assembly. In some embodiments, the anomaly detection method may include the following operations.

Step 2102, obtaining a current time-varying magnetic field signal obtained by the magnetic detection assembly detecting the working magnetic field generated by the field emitter.

Step 2104, determining a working difference between the current time-varying magnetic field signal and a reference time-varying magnetic field signal, and determining the operating status of the field emitter based on the working difference.

The anomaly detection method for a field emitter provided in this embodiment can perform real-time anomaly detection on the field emitter during electromagnetic navigation. During electromagnetic navigation, the field emitter generates a working magnetic field. At this time, each rotating magnet unit in the field emitter operates at its respective operating frequency. The combined magnetic field generated by all rotating magnet units together is the working magnetic field of the field emitter.

In some embodiments, when the field emitter includes only one rotating magnet unit, the combined magnetic field here can be the magnetic field generated by the revolution and rotation of that rotating magnet unit.

When the field emitter generates the working magnetic field, the magnetic sensor in the magnetic detection assembly detects the working magnetic field, thereby obtaining the measured working magnetic field data output by the magnetic sensor. Then, the measured working magnetic field data is compared with the reference working magnetic field data to determine the working difference between the two. Finally, the operating status of the field emitter is determined based on the working difference.

Here, the working difference is primarily used to determine whether the operating status of the field emitter is abnormal. An abnormal status means that the field emitter generates a working magnetic field that does not conform to the calibration value. The main reasons are the presence of other magnetic field interference or the real-time status of the field emitter not matching the calibrated status. Therefore, the reference working magnetic field data refers to the magnetic field data that the magnetic sensor should obtain when detecting the working magnetic field in the calibrated state in an environment without interference sources. Specifically, the reference working magnetic field data can be obtained by detecting the working magnetic field using the magnetic sensor during the factory calibration of the field emitter. At this time, the field emitter is not only in the calibrated state, but the calibration environment at the factory is controllable, ensuring that the calibration environment is free of interference sources. Meanwhile, the reference working magnetic field data can also be obtained through simulation calculation when the relative pose of the field emitter unit group and the magnetic sensor is fixed and known.

In some embodiments, determining the operating status of the field emitter based on the working difference includes: when the working difference is greater than a second preset threshold, determining that the operating status of the field emitter is abnormal.

Specifically, the working difference can be the cumulative value or the mean value of the errors between corresponding data points in the measured working magnetic field data and the reference working magnetic field data. For example, the calculation formula (9) for the mean error L is as follows: $L = \frac{1}{{N}_{T}} {\sum }_{n} {\left‖B i ′ b\left(n\right)-B i - 0 b\left(n\right)\right‖}^{2}$

Among them, $B i ′ b \left(n\right)$ is the measured working magnetic field data, **^{b}Bᵢ₋₀** (n) is the reference working magnetic field data, the symbol " " denotes the modulus value, and NT is the number of data points used for difference calculation. When calculating the mean error between corresponding data points, the corresponding measured working magnetic field data and reference working magnetic field data can be substituted into the above formula at sequential intervals.

As described above, this is the anomaly detection method for the field emitter provided by the present invention, which can perform real-time detection of the operating status of the field emitter during electromagnetic navigation.

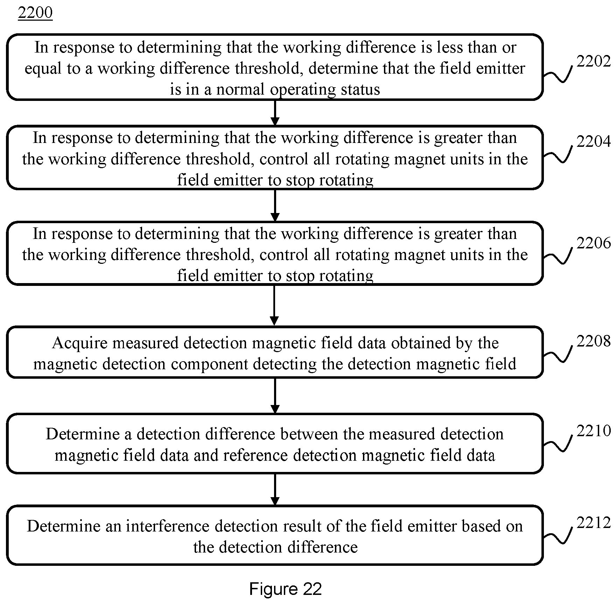

FIG. 22 is an exemplary flowchart of an anomaly detection method according to other embodiments of this specification. In some embodiments, the process 2200 shown in FIG. 22 can be executed by a processing device (e.g., processor 13) or an electro-magnetic transient simulator. As shown in FIG. 22, the process 2200 may include the following operations.

Further, in some embodiments, when the field emitter further includes a magnetic source assembly, the determining the operating status of the field emitter based on the working discrepancy may further include the following operations.

Step 2202, in response to determining that the working discrepancy is less than or equal to a working discrepancy threshold, determining that the field emitter is in a normal operating state;
Step 2204, in response to determining that the working discrepancy is greater than the working discrepancy threshold, controlling all angulation magnet units in the field emitter to stop rotating.

Step 2206, in response to determining that the working discrepancy is greater than the working discrepancy threshold, controlling all angulation magnet units in the field emitter to stop rotating.

Step 2208, obtaining measured detection magnetic field data obtained by the magnetic detection assembly detecting the detection magnetic field.

Step 2210, determining a detection discrepancy between the measured detection magnetic field data and reference detection magnetic field data.

Step 2212, determining an interference detection result of the field emitter based on the detection discrepancy.

Steps 2202 and 2204 are subsequent workflows required for anomaly detection of the field emitter under different operating states. For more details on steps 2206-2212, please refer to the relevant description of FIG. 20, which will not be repeated here.

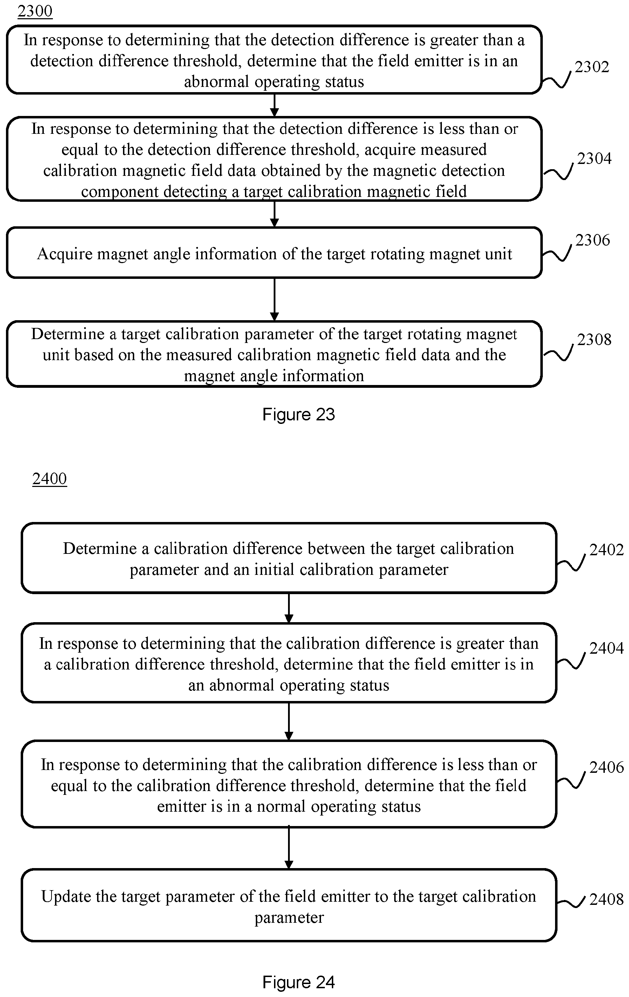

FIG. 23 is an exemplary flowchart of an anomaly detection method according to other embodiments of this specification. In some embodiments, the process 2300 shown in FIG. 23 can be executed by a processing device (e.g., processor 13) or an electro-magnetic transient simulator. As shown in FIG. 23, the process 2300 may include the following operations.

In some embodiments, after performing interference detection on the field emitter, the anomaly detection method for the field emitter may further include the following operations.

Step 2302, in response to determining that the detection discrepancy is greater than a detection discrepancy threshold, determining that the field emitter is in an abnormal operating state.

Step 2304, in response to determining that the detection discrepancy is less than or equal to the detection discrepancy threshold, obtaining measured calibration magnetic field data obtained by the magnetic detection assembly detecting a target calibration magnetic field.

Wherein, the target calibration magnetic field is a calibration magnetic field generated by a target angulation magnet unit, and the target angulation magnet unit is an angulation magnet unit to be calibrated in the field emitter unit group.

Step 2306, obtaining magnet angle information of the target angulation magnet unit.

Step 2308, determining a target calibration parameter of the target angulation magnet unit based on the measured calibration magnetic field data and the magnet angle information.

Steps 2302 and 2304 are subsequent workflows required for anomaly detection of the field emitter when the detection discrepancy is greater than the detection discrepancy threshold and less than the detection discrepancy threshold, respectively. For more details on steps 2306-2308, please refer to the relevant descriptions of FIG. 18 and FIG. 19, which will not be repeated here.

FIG. 24 is an exemplary flowchart of an anomaly detection method according to other embodiments of this specification. In some embodiments, the process 2400 shown in FIG. 24 can be executed by a processing device (e.g., processor 13) or an electro-magnetic transient simulator. As shown in FIG. 24, the process 2400 may include the following operations.

In some embodiments, after performing self-calibration on the field emitter, the anomaly detection method for the field emitter may further include the following operations.

Step 2402, determining a calibration discrepancy between the target calibration parameter and an initial calibration parameter.

Step 2404, in response to determining that the calibration discrepancy is greater than a calibration discrepancy threshold, determining that the field emitter is in an abnormal operating state.

Step 2406, in response to determining that the calibration discrepancy is less than or equal to the calibration discrepancy threshold, determining that the field emitter is in a normal operating state.

Step 2408, updating the target parameter of the field emitter to the target calibration parameter.

The calibration discrepancy refers to the parameter difference between the target calibration parameter and the initial calibration parameter. Steps 2404 and 2406 are subsequent workflows required for anomaly detection of the field emitter when the calibration discrepancy is greater than the calibration discrepancy threshold and less than the calibration discrepancy threshold, respectively. For more details on steps 2406-2408, please refer to the relevant descriptions of FIG. 18 and FIG. 19, which will not be repeated here.

It should be noted that FIG. 22 to FIG. 24 together can constitute a complete embodiment of the anomaly detection method for the field emitter disclosed in the embodiments of this specification.

On the other hand, some embodiments of this specification also disclose a usage workflow for the field emitter. For details of the field emitter usage workflow, please refer to the detailed description in other parts of this specification based on the content of FIG. 21 to FIG. 24.

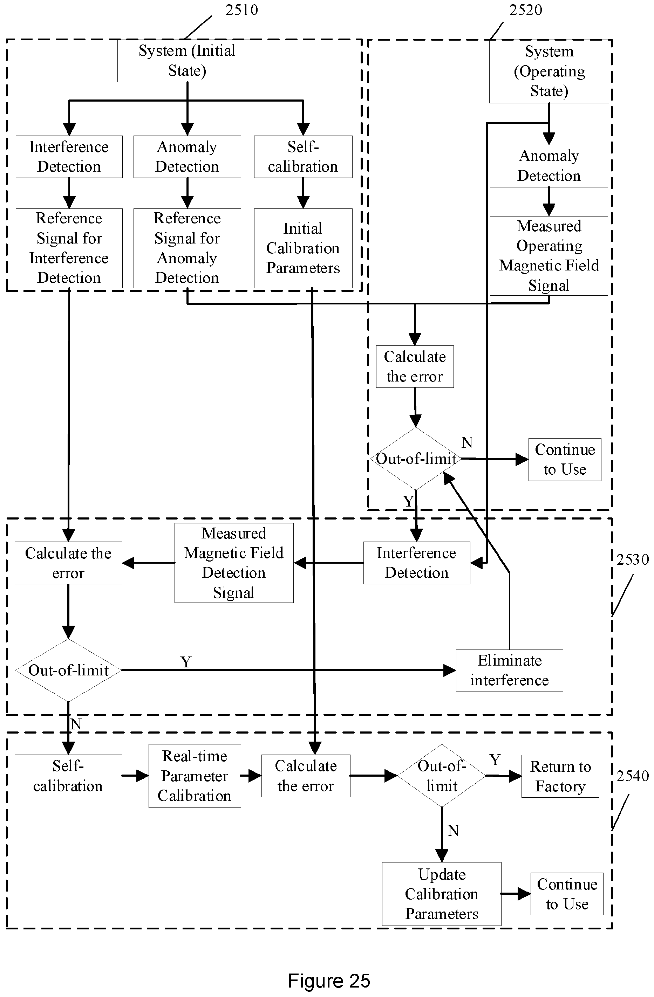

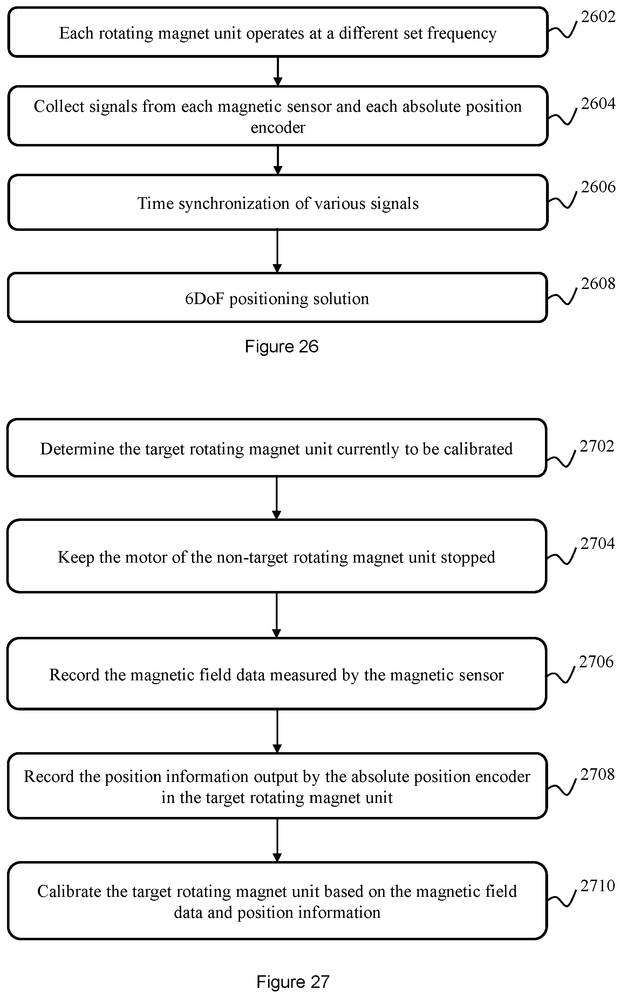

FIG. 25 is an exemplary flowchart of a method for using a field emitter according to some embodiments of this specification. Referring to FIG. 26, the method for using the field emitter includes: a pre-shipment detection stage 2510, a real-time anomaly detection stage 2520, an on-site interference detection stage 2530, and an on-site self-calibration stage 2540.

In the pre-shipment detection stage 2510 of the field emitter, interference detection, anomaly detection, and self-calibration are required, and the interference detection reference signal, anomaly detection reference signal, and initial calibration parameters are recorded respectively. Among them, the corresponding methods in this specific embodiment can be used for interference detection, anomaly detection, and self-calibration. The field emitter is put into use after shipment.

The real-time anomaly detection stage 2520 is in the electromagnetic navigation state of the field emitter. In this stage, the field emitter operates, and real-time anomaly detection is performed using the anomaly detection method in this specific embodiment, and the measured signal of the working magnetic field of the field emitter is recorded. The measured signal of the working magnetic field is compared with the anomaly detection reference signal, and the error is calculated. If the error does not exceed the limit, continue to use; if the error exceeds the limit, proceed to the on-site interference detection stage 2530.

In the on-site interference detection stage 2530, on-site interference detection is performed using the interference detection method in this specific embodiment, and the measured signal of the detection magnetic field generated by the interference detection coil is recorded. The measured signal of the detection magnetic field is compared with the interference detection reference signal, and the error is calculated. If the error does not exceed the limit, proceed to the on-site self-calibration stage; if the error exceeds the limit, interference elimination is required, and after interference elimination, the error between the measured signal of the working magnetic field and the anomaly detection reference signal is recalculated to determine whether it exceeds the limit.

In the on-site self-calibration stage 2540, the self-calibration method in this specific embodiment is used for on-site self-calibration, and the real-time calibration parameters of each rotating magnet unit are recorded. The real-time calibration parameters are compared with the initial calibration parameters, and the error is calculated. If the error does not exceed the limit, the calibration parameters are updated and continued use is allowed; if the error exceeds the limit, the field emitter is returned to the factory for repair.

It should be noted that some of the above processes do not need to be executed every time the field emitter is used. For example, the relative position calibration between each rotating magnet unit only needs to be performed periodically according to a set cycle. The calculation of the interaction torque between the magnets of each rotating magnet unit does not need to be repeated when the system parameters (such as rotational speed) remain unchanged. Preferably, the field emitter system pre-records interaction torque data under multiple rotational speed configurations, which can be looked up after parameter adjustment. The self-check of the rotating magnet unit can be performed when the field emitter leaves the factory, or once before each startup. Performing the self-check before startup is the power-on check.

In the above description, in response to determining that the working difference is greater than the working difference threshold, it indicates that the working state of the field emitter is abnormal. Subsequently, interference detection and self-calibration are performed in sequence to eliminate the cause of the abnormality. For example, interference detection is performed first. If no interfering magnetic field is found, self-calibration is then performed to determine whether there is a deviation in the pose of the rotating magnet unit in the field emitter. In some embodiments, in response to determining that the working difference is greater than the working difference threshold, self-calibration can also be performed first. If the calibration parameter error is found not to exceed the limit, interference detection is then performed. In some embodiments, in response to determining that the working difference is greater than the working difference threshold, a self-check assembly can also be used for self-checking to determine whether the magnet strength has decayed or the direction of the magnetic moment of the magnet has changed. The inspection order of interference detection, self-calibration, and self-check is not limited.

Based on the same inventive concept, some embodiments of this specification also provide an electro-magnetic transient simulator. The electro-magnetic transient simulator includes a processor, a receiving device, and the emitter including the magnetic detection assembly described above. The receiving device is used to detect the time-varying magnetic field generated by the field emitter. The processor is used to control the operation of the field emitter and determine the real-time pose of the receiving device in the time-varying magnetic field based on the magnetic field detection data of the receiving device.

The electromagnetic navigation method provided in this specification can be applied in the electro-magnetic transient simulator provided in this specification. Since the field emitter in the electro-magnetic transient simulator provided in this specification has a self-check function, the user can perform a self-check on each rotating magnet unit in the field emitter through its internal self-check assembly before the system positions the receiving device. Through the self-check, problematic rotating magnet units can be discovered in time, and the magnetic moment strength of the corresponding rotating magnet unit can be corrected to keep the actual value of its time-varying magnetic moment characteristic consistent with the model value, preventing the field emitter from generating a time-varying magnetic field that deviates from the standard during operation, ultimately ensuring the positioning accuracy of the magnetic sensor.

Figure 26 is an exemplary flowchart of real-time positioning of the field emitter according to some embodiments of this specification. Referring to Figure 26, real-time positioning includes the following steps:
Step 2602, each rotating magnet unit operates at a different set frequency;
Step 2604, collect signals from each magnetic sensor and each absolute position encoder;
Step 2606, time synchronization of various signals;
Step 2608, 6DoF positioning solution.

Specifically, during real-time positioning, each rotating magnet unit rotates at a different frequency, and each receiving device to be positioned (including a three-axis magnetic field sensor) collects magnetic field data in real time. The positioning algorithm requires two types of data input: the magnetic field data of each receiving device and the angle data sequence of the absolute position encoder of each rotating magnet unit within a time window; then, clock alignment of the two types of data is performed, meaning the algorithm requires the magnetic field data at each moment and the orientation of the magnetic moment in each rotating magnet unit at that moment; finally, these two types of data can be input into a nonlinear optimization solver or a Kalman filter for calculation. The objective function for the optimization solution is shown in formula (4) above.

It should be noted that the above solution algorithm does not require separation of the time-varying magnetic field; the combined magnetic field measurement value and the combined magnetic field model can be used directly.

Figure 33 is a schematic diagram of data acquisition during real-time positioning of the field emitter in this specification. Referring to Figure 33, the positioning algorithm requires data within a real-time time window, but there is no quantitative relationship limitation between the width of this real-time time window and the rotation period of the rotating magnet unit. The width of this real-time time window can be adjusted based on actual signal-to-noise ratio and real-time requirements. When real-time requirements are not high, the sliding real-time time window width can be longer, resulting in a higher signal-to-noise ratio for the solution; when real-time requirements are relatively high, the sliding real-time time window width needs to be shortened, which may cause some 'jitter' in the positioning result, because the data signal-to-noise ratio decreases, and stronger noise leads to increased variance in the positioning result.

Additionally, the rotational speed of the rotating magnet unit can also be adjusted. Higher frequency time-varying magnetic fields are more likely to induce eddy currents in metals, thereby affecting the magnetic field of the surrounding environment and reducing positioning accuracy. Therefore, the rotational speed can be appropriately reduced to improve the antiinterference capability of the field emitter system. However, in scenarios with high real-time requirements, the rotational speed should not be too low, otherwise the positioning speed will be insufficient.

Figure 27 is an exemplary flowchart of a self-calibration method for a field emitter according to other embodiments of this specification. Referring to Figure 27, the self-calibration method for the field emitter includes the following steps:
Step 2702, determine the target rotating magnet unit currently to be calibrated;
Step 2704, keep the motors of the non-target rotating magnet units stopped;
Step 2706, record the magnetic field data measured by the magnetic sensor;
Step 2708, record the position information output by the absolute position encoder in the target rotating magnet unit;
Step 2710, calibrate the target rotating magnet unit based on the magnetic field data and position information.

Specifically, the above steps S530 and S540 can be performed simultaneously. For all rotating magnet units in the field emitter, the above steps are performed one by one for calibration. For a detailed description of the self-calibration method, refer to the descriptions of Figure 18 and Figure 19.

In this specific embodiment, an interference detection method for the field emitter is also provided. The following is an explanation of the principle of the interference detection method for the field emitter.

When leaving the factory, the field emitter also undergoes a calibration, and its basic process is similar to the self-calibration process described above. However, the environment during factory calibration is more controllable. For example, the calibration environment can be controlled to have no other magnetic field sources, no large metal objects, and no ferromagnetic materials nearby. On-site calibration involves many uncertainties, such as various interference sources that may exist in the surrounding environment, causing distortion of the measured magnetic field. Directly calibrating the field emitter may introduce many errors. To eliminate this interference, an interference detection coil is also introduced into the field emitter, along with the magnetic sensor in the magnetic detection assembly. Both are fixed to the installation housing of the field emitter (e.g., encapsulated with adhesive). Therefore, the relative position of the interference detection coil and the magnetic sensor is stable.

FIG. 28 is an exemplary flowchart of an interference detection method for a field emitter according to other embodiments of the present specification. Referring to FIG. 28, the interference detection method for the field emitter includes the following steps:
Step 2802, keep the motors of all rotating magnet units in the field emitter stopped;
Step 2804, activate the target interference detection coil to be detected;
Step 2806, the magnetic sensor in the magnetic detection assembly records the magnetic field generated by the target interference detection coil;
Step 2808, compare the detection signal of the magnetic sensor with a reference signal.

Specifically, for all interference detection coils in the field emitter, the above steps are performed one by one for detection.

The detection signal generated by the coil can be a sinusoidal signal, and to ensure a clean navigation working frequency band, the detection signal frequency can be set slightly higher than the operating frequency of the electromagnetic navigation, such as 50Hz~100Hz. During detection, all rotating magnet units are stopped, eliminating all known time-varying magnetic field sources. The magnetic sensor collects this signal $B ij - e ′ b \left(n\right)$ and compares it with a reference signal ^{b}Bᵢⱼ₋ₑ (n) recorded in a clean environment at the factory, where ^{b}Bᵢⱼ₋ₑ (n) represents the magnetic field signal generated by the j-th interference detection coil recorded by the i-th magnetic sensor. The comparison method can be phase-based, such as comparing peak positions, calculating correlation coefficients, etc. (It is known that interference such as metal eddy currents are interference signals with the same frequency but different phases). The disturbance of some small interference sources to the magnetic field is usually local. Therefore, multiple interference detection coils are arranged around the field emitter. During detection, each coil generates a detection signal one by one and performs the same phase comparison detection, thereby more comprehensively detecting the conditions around the field emitter and ensuring that the self-calibration process is in an environment free of magnetic field interference. When the interference detection finds perceptible interference in the surroundings (e.g., the phase difference exceeds a certain limit), the field emitter will not start the self-calibration procedure and will notify the user of the presence of interference. The user can then eliminate the interference source on their own. If no interference is perceived, the self-calibration procedure can be started.

It should be noted that ^{b}Bᵢⱼ₋ₑ (n) and $B ij - e ′ b \left(n\right)$ are obviously not measured simultaneously, but both use n to represent the sequence number. n is labeled from 0 to *N_{T}*, where represents the signal start time. Therefore, although ^{b}Bᵢⱼ₋ₑ (n) and $B ij - e ′ b \left(n\right)$ are not measured simultaneously, they can still be compared one-to-one.

In this specific embodiment, an abnormality detection method for the field emitter is also provided. The following is a description of the principle of the abnormality detection method for the field emitter.

When the field emitter is used on-site, the magnetic sensor in the magnetic detection assembly continuously records the magnetic field value $B i ′ b \left(n\right)$ during navigation and periodically (e.g., 1s) compares it with the recorded reference magnetic field value $B i ′ b \left(n\right)$, as shown in formula (3), which will not be repeated here. Whether it is a change in intensity or phase, it will be reflected in the error quantity L (Err0). When the value of L is within a certain limit, the system is considered normal and can continue to be used; if it exceeds a certain limit, the system will stop the navigation operation and enter the "interference detection" phase.

It should be noted that the magnetic field $B i ′ b \left(n\right)$ measured by the magnetic sensor during real-time abnormality detection is different from the the magnetic field ^{b}Bᵢ(n) measured during the self-calibration process described above. During measurement $B i ′ b \left(n\right)$, the field emitter is in a "navigation state", where all rotating magnet units are operating normally. During measurement ^{b}Bᵢ(n), only one rotating magnet unit operates at a time, while the other rotating magnet units do not operate. For a detailed description of the interference detection method, refer to the description of FIG. 20.

In some embodiments, the self-calibration method, interference detection method, and real-time abnormality detection method performed using the magnetic detection assembly can be replaced by using a self-check assembly. For example, the field emitter includes a self-check assembly but does not include a magnetic detection assembly. The self-check assembly detects the calibration magnetic field of the rotating magnet unit, the detection magnetic field generated by the magnetic source component, and the real-time working magnetic field generated by the field emitter during the electromagnetic navigation process. The structure and configuration of the self-check assembly are as described above. In the method for determining the real-time operating status of the field emitter using the self-check assembly (FIG. 17) and the real-time abnormality detection method using the magnetic detection assembly (FIG. 21) described above, the devices for detecting the real-time working magnetic field of the field emitter are different (the self-check assembly and the magnetic detection assembly, respectively), but the detection methods are similar.

FIG. 35 is an exemplary module diagram of a self-check system for a rotating magnet unit according to some embodiments of the present specification. The system is applied to the rotating magnet unit shown in some embodiments of the present specification. The self-check system of the rotating magnet unit can be implemented in the processor 13 in the form of hardware or software. As shown in FIG. 35, the self-check system 3500 includes the following modules:
A driving module 3510, configured to control the driving component to drive the magnet to rotate at a preset speed;
A first signal acquisition module 3520, configured to acquire a current time-varying magnetic field signal of the magnet; wherein the current time-varying magnetic field signal is obtained by the self-check assembly detecting the magnetic field signal generated by the magnet when rotating;
A first self-check result determination module 3530, configured to determine a signal difference degree between the current time-varying magnetic field signal of the magnet and a reference time-varying magnetic field signal, and determine the self-check result of the rotating magnet unit based on the signal difference degree.

The self-check system 3500 can use the driving module 3510, the first signal acquisition module 3520, and the first self-check result determination module 3530 to execute the self-check method for the rotating magnet unit in the present specification. For details on the self-check method for the rotating magnet unit, please refer to the previous description.

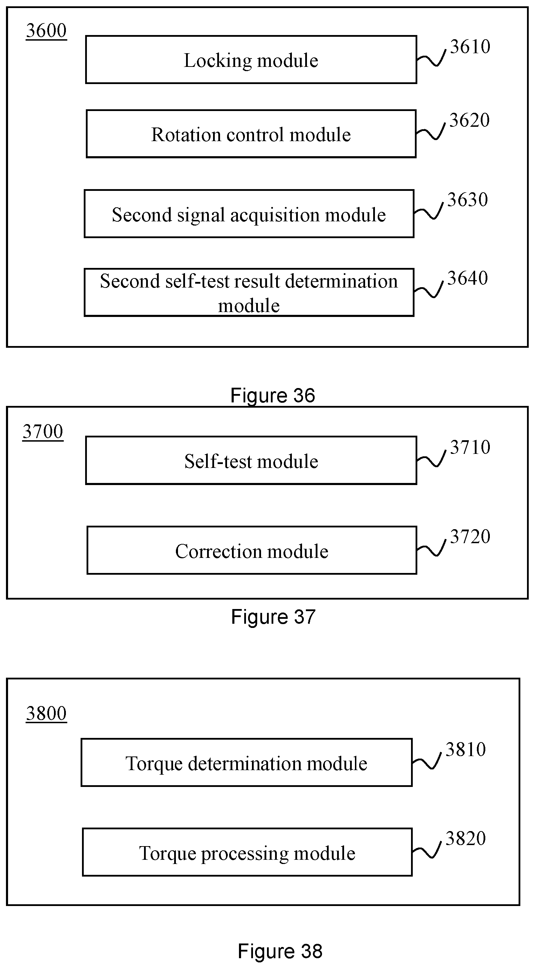

FIG. 36 is an exemplary module diagram of a self-check system for a field emitter according to some embodiments of the present specification. The system is applied to the field emitter shown in some embodiments of the present specification. The self-check system of the emitter can be implemented in the processor 13 in the form of hardware or software. As shown in FIG. 36, the self-check system 3600 includes the following modules:
A locking module 3610, configured to determine a target rotating magnet unit to be self-checked in the field emitter, and lock the angular position of the magnet of the non-target rotating magnet unit;
A rotation control module 3620, configured to control the driving component to drive the magnet of the target rotating magnet unit to rotate at a preset speed;
A second signal acquisition module 3630, configured to acquire a current time-varying magnetic field signal of the magnet; wherein the current time-varying magnetic field signal is obtained by the self-check assembly detecting the magnetic field signal generated by the magnet when rotating;
A second self-check result determination module 3640, configured to determine a signal difference degree between the current time-varying magnetic field signal of the magnet and a reference time-varying magnetic field signal, and determine the self-check result of the rotating magnet unit based on the signal difference degree.

The self-check system 3600 can use the locking module 3610, the rotation control module 3620, the second signal acquisition module 3630, and the second self-check result determination module 3640 to execute the self-check method for the field emitter in the present specification. For details on the self-check method for the field emitter, please refer to the previous description.

FIG. 37 is an exemplary module diagram of an electromagnetic navigation device according to some embodiments of the present specification. The electromagnetic navigation device can be implemented in the processor 13 in the form of hardware or software. As shown in FIG. 37, the electromagnetic navigation device 3700 includes:
Self-check module 3710, configured to: perform a self-check on the field emitter to obtain self-check results for each rotating magnet unit in the field emitter, including: determining a target rotating magnet unit to be self-checked in the field emitter, and locking the angular position of the magnet of a non-target rotating magnet unit; controlling the drive assembly to drive the magnet of the target rotating magnet unit to rotate at a preset rotational speed; acquiring a current time-varying magnetic field signal of the magnet; wherein the current time-varying magnetic field signal is obtained by the self-check assembly detecting the magnetic field signal generated by the magnet during rotation; determining a signal difference between the current time-varying magnetic field signal of the magnet and a reference time-varying magnetic field signal, and determining the self-check result of the rotating magnet unit based on the signal difference.

Correction module 3720, configured to: for any of the rotating magnet units, determine a target magnetic moment strength of the rotating magnet unit based on the self-check result of the rotating magnet unit.

The electromagnetic navigation device 3700 can utilize the self-check module 3710 and the correction module 3720 to execute the electromagnetic navigation method in this specification. For details on the electromagnetic navigation method, please refer to the preceding description.

FIG. 38 is an exemplary module diagram of a control system for a field emitter according to some embodiments of this specification. The system is applied to a field emitter, the field emitter comprising at least one rotating magnet unit, at least one of the rotating magnet units including a drive assembly and a magnet. The control system of the field emitter may be implemented in the processor 13 in the form of hardware or software. The control system 3800 includes the following modules:

Torque determination module 3810, configured to: for at least one of the rotating magnet units in the field emitter, determine an interaction torque exerted on the magnet of the rotating magnet unit by magnets from other rotating magnet units.

Torque processing module 3820, configured to: use the interaction torque exerted on the magnet of the rotating magnet unit as a feed-forward input for its own drive, determine a drive current corresponding to the rotating magnet unit; based on the drive current, control the drive assembly to drive the rotating magnet unit to rotate, generating a time-varying magnetic field.

The control system 3800 of the field emitter can utilize the torque determination module 3810 and the torque processing module 3820 to execute the control method for the field emitter in this specification. For details on the control method for the field emitter, please refer to the preceding description.

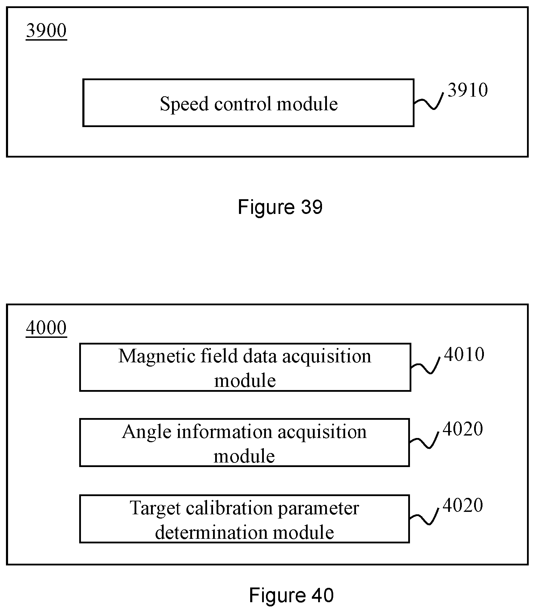

FIG. 39 is an exemplary module diagram of a control system for a field emitter according to some embodiments of this specification. The field emitter comprises at least one rotating magnet unit, at least one of the rotating magnet units including a drive assembly and a magnet. The control system of the field emitter may be implemented in the processor 13 in the form of hardware or software. The control system 3900 includes a rotational speed control module.

Rotational speed control module 3910, configured to: control the magnets of the rotating magnet units having the same initial magnetic moment direction to generate different rotational speeds.

The control system 3900 of the field emitter can utilize the rotational speed control module 3910 to execute the control method for the field emitter in this specification. For details on the control method for the field emitter, please refer to the preceding description.

FIG. 40 is an exemplary module diagram of a self-calibration system for a field emitter according to some embodiments of this specification. The self-calibration system for the field emitter is applied to the field emitter shown in some embodiments of this specification. The self-calibration system of the field emitter may be implemented in the processor 13 in the form of hardware or software. The self-calibration system 4000 includes the following modules:

Magnetic field data acquisition module 4010, configured to acquire measured calibration magnetic field data obtained by the magnetic detection assembly detecting a target calibration magnetic field, wherein the target calibration magnetic field is a calibration magnetic field generated by a target rotating magnet unit, and the target rotating magnet unit is a rotating magnet unit to be calibrated in the field emitter unit group.

Angle information acquisition module 4020, configured to acquire magnet angle information of the target rotating magnet unit.

Target calibration parameter determination module 4030, configured to determine a target calibration parameter of the target rotating magnet unit based on the measured calibration magnetic field data and the magnet angle information.

The self-calibration system 4000 of the field emitter can utilize the magnetic field data acquisition module 4010, the angle information acquisition module 4020, and the target calibration parameter determination module 4030 to execute the self-calibration method for the field emitter in this specification. For details on the self-calibration method for the field emitter, please refer to the preceding description.

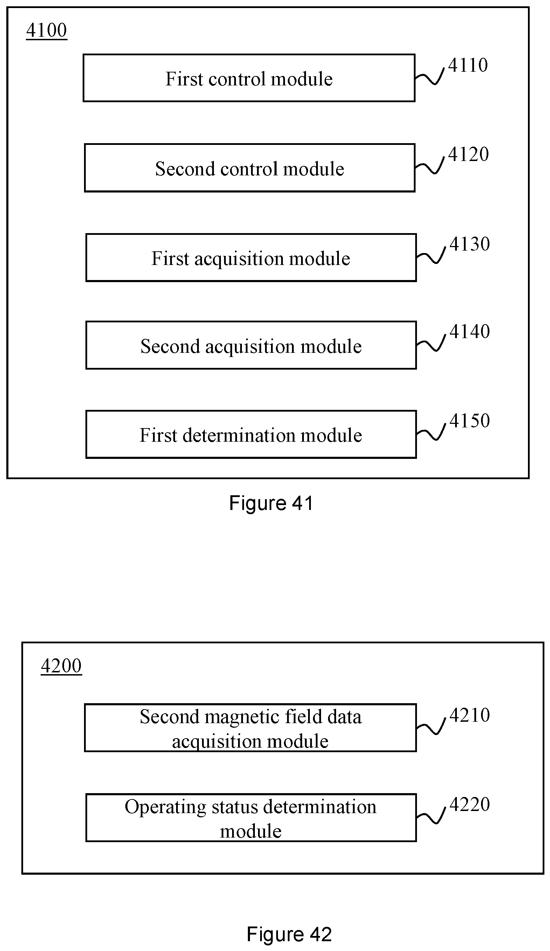

FIG. 41 is an exemplary module diagram of an interference detection system for a field emitter according to some embodiments of this specification. The interference detection system is applied to the field emitter shown in some embodiments of this specification. The interference detection system of the field emitter may be implemented in the processor 13 in the form of hardware or software. The interference detection system 4100 includes the following modules:
First control module 4110, configured to control all rotating magnet units in the field emitter to stop rotating.
Second control module 4120, configured to control the magnetic source assembly to generate the detection magnetic field.

First acquisition module 4130, configured to acquire measured detection magnetic field data obtained by the magnetic detection assembly detecting the detection magnetic field.

Second acquisition module 4140, configured to determine a detection difference between the measured detection magnetic field data and reference detection magnetic field data.

First determination module 4150, configured to determine an interference detection result of the field emitter based on the detection difference.

The interference detection system 4100 of the field emitter can utilize the first control module 4110, the second control module 4120, the first acquisition module 4130, the second acquisition module 4140, and the first determination module 4150 to execute the interference detection method for the field emitter in this specification. For details on the interference detection method for the field emitter, please refer to the preceding description.

FIG. 42 is an exemplary module diagram of an anomaly detection system for a field emitter according to some embodiments of this specification. The anomaly detection system is applied to the field emitter shown in some embodiments of this specification. The anomaly detection system of the field emitter may be implemented in the processor 13 in the form of hardware or software. The anomaly detection system includes the following modules:
Second magnetic field data acquisition module 4210, configured to acquire measured working magnetic field data obtained by the magnetic detection assembly detecting a working magnetic field generated by the field emitter.

Operating state determination module 4220, configured to determine a working difference between the measured working magnetic field data and reference working magnetic field data, and determine an operating state of the field emitter based on the working difference.

The anomaly detection system 4200 of the field emitter can utilize the second magnetic field data acquisition module 4210 and the operating state determination module 4220 to execute the anomaly detection method for the field emitter in this specification. For details on the anomaly detection method for the field emitter, please refer to the preceding description.

The basic concepts have been described above. Obviously, for those skilled in the art, the above detailed disclosure is merely an example and does not constitute a limitation to this specification. Although not explicitly stated herein, those skilled in the art may make various modifications, improvements, and corrections to this specification. Such modifications, improvements, and corrections are suggested in this specification, and therefore such modifications, improvements, and corrections still fall within the spirit and scope of the exemplary embodiments of this specification.

Meanwhile, this specification uses specific terms to describe the embodiments of this specification. For example, "one embodiment," "an embodiment," and/or "some embodiments" refer to a certain feature, structure, or characteristic associated with at least one embodiment of this specification. Therefore, it should be emphasized and noted that references to "an embodiment" or "one embodiment" or "an alternative embodiment" in different places of this specification do not necessarily refer to the same embodiment. Furthermore, certain features, structures, or characteristics of one or more embodiments of this specification may be appropriately combined.

Additionally, unless explicitly stated in the claims, the order of processing elements and sequences, the use of numbers and letters, or other names in this specification are not intended to limit the order of the processes and methods of this specification. Although the above disclosure discusses some currently considered useful embodiments of the invention through various examples, it should be understood that such details are for illustrative purposes only, and the appended claims are not limited to the disclosed embodiments. On the contrary, the claims are intended to cover all modifications and equivalent combinations that fall within the spirit and scope of the embodiments of this specification. For example, although the system components described above may be implemented by hardware devices, they may also be implemented solely through software solutions, such as installing the described system on an existing server or mobile device.

Similarly, it should be noted that, to simplify the disclosure of this specification and thereby aid in understanding one or more inventive embodiments, the foregoing description of the embodiments of this specification sometimes combines various features into a single embodiment, figure, or description thereof. However, this method of disclosure does not imply that the subject matter of this specification requires more features than those recited in the claims. In fact, the features of an embodiment are fewer than all the features of a single embodiment disclosed above.

In some embodiments, numbers describing component quantities or attributes are used. It should be understood that such numbers used in describing embodiments are, in some instances, modified by the terms "about," "approximately," or "substantially." Unless otherwise stated, "about," "approximately," or "substantially" indicates that the stated number allows for a variation of ±20%. Accordingly, in some embodiments, the numerical parameters set forth in the specification and claims are approximations that may vary depending on the desired characteristics of individual embodiments. In some embodiments, numerical parameters should consider the specified significant digits and adopt a general rounding method. Although the numerical ranges and parameters used in some embodiments of this specification to define the breadth of their scope are approximations, in specific embodiments, such numerical values are set as precisely as feasible within the practical range.

Each patent, patent application, patent application publication, and other material, such as articles, books, specifications, publications, documents, etc., cited in this specification is hereby incorporated by reference in its entirety. Except for any prosecution history files that are inconsistent with or conflict with the content of this specification, and except for any documents that limit the broadest scope of the claims of this specification (whether currently or subsequently appended to this specification). It should be noted that if there is any inconsistency or conflict between the descriptions, definitions, and/or usage of terms in the accompanying materials of this specification and the content described herein, the descriptions, definitions, and/or usage of terms in this specification shall prevail.

Finally, it should be understood that the embodiments described in this specification are merely illustrative of the principles of the embodiments of this specification. Other variations may also fall within the scope of this specification. Therefore, by way of example and not limitation, alternative configurations of the embodiments of this specification may be considered consistent with the teachings of this specification. Accordingly, the embodiments of this specification are not limited to the embodiments explicitly introduced and described in this specification.

## Claims

1. A rotating magnet unit, comprising: a drive assembly, a magnet, and a self-check assembly;
the drive assembly is connected to the magnet and is used to drive the magnet to rotate;
the self-check assembly is used to detect a magnetic field signal generated during the rotation of the magnet.

2. The rotating magnet unit according to claim 1, wherein the self-check assembly comprises three ring coils whose normal vectors are pairwise orthogonal;
the three ring coils are respectively located on three sides of the magnet; a normal vector of one of the ring coils coincides with a rotation axis of the magnet.

3. The rotating magnet unit according to claim 1, wherein the self-check assembly comprises a magnetic sensor;
the magnetic sensor is located on a side of the drive assembly away from the magnet.

4. The rotating magnet unit according to any one of claims 1-3, wherein the rotation axis of the magnet:
is not parallel to a magnetic moment direction of the magnet; or,
is not parallel to the magnetic moment direction of the magnet and passes through a center of mass of the magnet; or,
is perpendicular to the magnetic moment direction of the magnet; or,
is perpendicular to the magnetic moment direction of the magnet and passes through the center of mass of the magnet.

5. The rotating magnet unit according to any one of claims 1-4, wherein the drive assembly comprises a motor, a reduction mechanism, and an absolute position encoder;
an output shaft of the motor is connected to the magnet via the reduction mechanism, and the absolute position encoder is used to collect angular position information of the magnet.

6. The rotating magnet unit according to any one of claims 1-5, wherein the drive assembly comprises a transmission device, and a motor drives the magnet to rotate via the transmission device.

7. The rotating magnet unit according to any one of claims 1-6, further comprising: a mounting housing;
the drive assembly, the magnet, and the self-check assembly are all installed inside the mounting housing;
the mounting housing is provided with a functional interface, and the functional interface connects the drive assembly and the self-check assembly.

8. The rotating magnet unit according to any one of claims 1-7, further comprising: a locking assembly;
the locking assembly is used to lock the angular position of the magnet.

9. A field emitter, comprising at least one rotating magnet unit, wherein at least one of the rotating magnet units is the rotating magnet unit according to any one of claims 1-8.

10. An electromagnetic navigation system, comprising a processor, a receiving device, and the field emitter according to claim 9, wherein the receiving device is used to detect a time-varying magnetic field generated by the field emitter, and the processor is used to control the operation of the field emitter and determine a real-time pose of the receiving device in the time-varying magnetic field based on magnetic field detection data from the receiving device.

11. A self-check method for a rotating magnet unit, applied to the rotating magnet unit according to any one of claims 1-8; the self-check method for the rotating magnet unit comprises:
controlling the drive assembly to drive the magnet to rotate at a preset rotational speed;
acquiring a current time-varying magnetic field signal of the magnet; wherein the current time-varying magnetic field signal is obtained by the self-check assembly detecting a magnetic field signal generated when the magnet rotates;
determining a signal difference between the current time-varying magnetic field signal of the magnet and a reference time-varying magnetic field signal, and determining a self-check result of the rotating magnet unit based on the signal difference.

12. The self-check method for a rotating magnet unit according to claim 11, wherein the reference time-varying magnetic field signal is obtained by detecting, via the self-check assembly, a magnetic field signal generated when the magnet rotates at the preset rotational speed in an initial state of the rotating magnet unit.

13. The self-check method for a rotating magnet unit according to claim 11 or 12, wherein determining the signal difference degree between the current time-varying magnetic field signal of the magnet and the reference time-varying magnetic field signal, and determining the self-check result of the rotating magnet unit based on the signal difference degree comprises:
determining a current magnetic field strength and a proportional relationship of each component in the current time-varying magnetic field signal based on the current time-varying magnetic field signal;
determining a strength difference degree between the current magnetic field strength and a reference magnetic field strength, the reference magnetic field strength being determined based on the reference time-varying magnetic field signal;
determining a proportional difference degree between the proportional relationship of each component in the current time-varying magnetic field signal and the proportional relationship of each component in the reference time-varying magnetic field signal;
determining the self-check result of the rotating magnet unit based on the strength difference degree and the proportional difference degree.

14. A self-check system for a rotating magnet unit, applied to the rotating magnet unit according to any one of claims 1-8; the system comprises:
a driving module, configured to control the drive assembly to drive the magnet to rotate at a preset rotational speed;
a first signal acquisition module, configured to acquire a current time-varying magnetic field signal of the magnet; wherein the current time-varying magnetic field signal is obtained by the self-check assembly detecting a magnetic field signal generated when the magnet rotates;
a first self-check result determination module, configured to determine a signal difference between the current time-varying magnetic field signal of the magnet and a reference time-varying magnetic field signal, and determine a self-check result of the rotating magnet unit based on the signal difference.

15. A self-check device for a rotating magnet unit, comprising a processor configured to execute the self-check method for a rotating magnet unit according to any one of claims 11-13.

16. A computer-readable storage medium, storing computer instructions, wherein when a computer reads the computer instructions from the storage medium, the computer executes the self-check method for a rotating magnet unit according to any one of claims 11-13.

17. A self-check method for a field emitter, applied to the field emitter according to claim 9; the self-check method for the field emitter comprises:
determining a target rotating magnet unit to be self-checked in the field emitter, and locking an angular position of a magnet of a non-target rotating magnet unit;
controlling the drive assembly to drive the magnet of the target rotating magnet unit to rotate at a preset rotational speed;
acquiring a current time-varying magnetic field signal of the magnet; wherein the current time-varying magnetic field signal is obtained by the self-check assembly detecting a magnetic field signal generated when the magnet rotates;
determining a signal difference degree between the current time-varying magnetic field signal of the magnet and a reference time-varying magnetic field signal, and determining a self-check result of the rotating magnet unit based on the signal difference degree.

18. A self-check system for a field emitter, applied to the field emitter according to claim 9;
the system comprises:
a locking module, configured to determine a target rotating magnet unit to be self-checked in the field emitter, and lock an angular position of a magnet of a non-target rotating magnet unit;
a rotation control module, configured to control the drive assembly to drive the magnet of the target rotating magnet unit to rotate at a preset rotational speed;
a second signal acquisition module, configured to acquire a current time-varying magnetic field signal of the magnet; wherein the current time-varying magnetic field signal is obtained by the self-check assembly detecting a magnetic field signal generated when the magnet rotates;
A second self-check result determination module, configured to determine a signal difference between a current time-varying magnetic field signal of the magnet and a reference time-varying magnetic field signal, and determine a self-check result of the rotating magnet unit based on the signal difference.

19. A self-check device for a rotating magnet unit, comprising a processor configured to execute the self-check method for a rotating magnet unit according to claim 17.

20. A computer-readable storage medium storing computer instructions, wherein when a computer reads the computer instructions from the storage medium, the computer executes the self-check method for a rotating magnet unit according to claim 17.

21. An electromagnetic navigation method, wherein the electromagnetic navigation method is applied to the electromagnetic navigation system according to claim 10;
The electromagnetic navigation method comprises:
performing a self-check on the field generator to obtain self-check results of each rotating magnet unit in the field generator, comprising:
determining a target rotating magnet unit to be self-checked in the field generator, and locking the angular position of the magnet of a non-target rotating magnet unit;
controlling the drive assembly to drive the magnet of the target rotating magnet unit to rotate at a preset rotational speed;
acquiring a current time-varying magnetic field signal of the magnet; wherein the current time-varying magnetic field signal is obtained by the self-check assembly detecting a magnetic field signal generated by the magnet during rotation;
determining a signal difference between the current time-varying magnetic field signal of the magnet and a reference time-varying magnetic field signal, and determining a self-check result of the rotating magnet unit based on the signal difference;
for any of the rotating magnet units, determining a target magnetic moment strength of the rotating magnet unit based on the self-check result of the rotating magnet unit.

22. The electromagnetic navigation method according to claim 21, further comprising:
acquiring magnetic field data of a time-varying magnetic field generated by the field generator; wherein the magnetic field data of the time-varying magnetic field is obtained by the receiving device detecting the time-varying magnetic field;
determining a real-time pose of the receiving device in the time-varying magnetic field based on the magnetic field data of the time-varying magnetic field and a time-varying characteristic of the time-varying magnetic field.

23. The electromagnetic navigation method according to claim 22, wherein the time-varying characteristic of the time-varying magnetic field comprises a magnetic moment time-varying characteristic of each of the rotating magnet units in the field generator in a field generator coordinate system, the magnetic moment time-varying characteristic comprising a magnetic moment direction time-varying characteristic and a magnetic moment strength.

24. The electromagnetic navigation method according to claim 21 or 22, wherein determining the real-time pose of the receiving device in the time-varying magnetic field based on the magnetic field data of the time-varying magnetic field and the time-varying characteristic of the time-varying magnetic field comprises:
calculating in real time the pose of the receiving device in the time-varying magnetic field based on the magnetic field data of the time-varying magnetic field within a real-time time window and the time-varying characteristic of the time-varying magnetic field; wherein a window width of the real-time time window is determined based on a real-time requirement for calculating the pose of the receiving device.

25. The method according to any one of claims 21-24, further comprising:
acquiring real-time detection results obtained by the self-check assembly of each of the rotating magnet units detecting the time-varying magnetic field generated by the field generator;
determining a result difference between the real-time detection results of the self-check assembly of each of the rotating magnet units and respective corresponding reference detection results;
determining a real-time operating status of the field generator based on the result difference.

26. An electromagnetic navigation device, applied to the electromagnetic navigation system according to claim 10;
The electromagnetic navigation device comprises:
a self-check module, configured to:
perform a self-check on the field generator to obtain self-check results of each rotating magnet unit in the field generator, comprising:
determining a target rotating magnet unit to be self-checked in the field generator, and locking the angular position of the magnet of a non-target rotating magnet unit;
controlling the drive assembly to drive the magnet of the target rotating magnet unit to rotate at a preset rotational speed;
acquiring a current time-varying magnetic field signal of the magnet; wherein the current time-varying magnetic field signal is obtained by the self-check assembly detecting a magnetic field signal generated by the magnet during rotation;
determining a signal difference between the current time-varying magnetic field signal of the magnet and a reference time-varying magnetic field signal, and determining a self-check result of the rotating magnet unit based on the signal difference;
a correction module, configured to, for any of the rotating magnet units, determine a target magnetic moment strength of the rotating magnet unit based on the self-check result of the rotating magnet unit.

27. An electromagnetic navigation device, comprising a processor configured to execute the electromagnetic navigation method according to any one of claims 21-25.

28. A computer-readable storage medium storing computer instructions, wherein when a computer reads the computer instructions from the storage medium, the computer executes the electromagnetic navigation method according to any one of claims 21-25.

29. A control method for a field generator, applied to a field generator, the field generator comprising at least one rotating magnet unit, at least one of the rotating magnet units comprising a drive assembly and a magnet;
The method comprises:
for each of the rotating magnet units in the field generator,
determining an interaction torque exerted on the magnet of the rotating magnet unit by magnets from other rotating magnet units;
using the interaction torque exerted on the magnet of the rotating magnet unit as a feedforward input for its own drive, determining a drive current corresponding to the rotating magnet unit;
controlling, based on the drive current, the drive assembly to drive the rotating magnet unit to rotate, generating a time-varying magnetic field.

30. The method according to claim 29, wherein determining the interaction torque exerted on the magnet of the rotating magnet unit by magnets from other rotating magnet units comprises:
determining one of the rotating magnet units in the field generator as a first target rotating magnet unit;
determining a target magnetic field time-varying characteristic of a combined magnetic field at the magnet of the first target rotating magnet unit, the combined magnetic field being jointly generated by rotating magnet units in the field generator other than the first target rotating magnet unit;
determining a target magnetic moment time-varying characteristic of the magnet of the first target rotating magnet unit;
determining a time-varying characteristic of the interaction torque exerted on the magnet of the first target rotating magnet unit based on the target magnetic field time-varying characteristic and the target magnetic moment time-varying characteristic.

31. A control system for a field generator, applied to a field generator, the field generator comprising at least one rotating magnet unit, at least one of the rotating magnet units comprising a drive assembly and a magnet;
The system comprises:
a torque determination module, configured to, for each of the rotating magnet units in the field generator, determine an interaction torque exerted on the magnet of the rotating magnet unit by magnets from other rotating magnet units;
a torque processing module, configured to:
use the interaction torque exerted on the magnet of the rotating magnet unit as a feedforward input for its own drive, determining a drive current corresponding to the rotating magnet unit;
control, based on the drive current, the drive assembly to drive the rotating magnet unit to rotate, generating a time-varying magnetic field.

32. A control device for a field generator, comprising a processor configured to execute the control method for a field generator according to any one of claims 29-30.

33. A computer-readable storage medium, the storage medium storing computer instructions, when a computer reads the computer instructions in the storage medium, the computer executes the control method for a field emitter according to any one of claims 29-30.

34. A control method for a field emitter, applied to a field emitter, the field emitter comprising at least one rotating magnet unit, at least one of the rotating magnet units including a drive assembly and a magnet;
The method comprises:
Controlling the magnets of the rotating magnet units having the same initial magnetic moment direction to generate different rotational speeds.

35. The method according to claim 34, the method further comprising:
Controlling the magnets of the rotating magnet units having different initial magnetic moment directions to generate the same rotational speed.

36. A control system for a field emitter, applied to a field emitter, the field emitter comprising at least one rotating magnet unit, at least one of the rotating magnet units including a drive assembly and a magnet;
The system comprises a rotational speed control module, configured for:
Controlling the magnets of the rotating magnet units having the same initial magnetic moment direction to generate different rotational speeds.

37. A control device for a field emitter, comprising a processor, the processor being configured to execute the control method for a field emitter according to any one of claims 34-35.

38. A computer-readable storage medium, the storage medium storing computer instructions, when a computer reads the computer instructions in the storage medium, the computer executes the control method for a field emitter according to any one of claims 34-35.

39. A field emitter, the field emitter comprising: a field emission unit group and a magnetic detection assembly;
The field emission unit group comprises at least one rotating magnet unit;
The magnetic detection assembly is used for detecting a calibration magnetic field generated by the rotating magnet unit.

40. The field emitter according to claim 39, the magnetic detection assembly further comprising a circuit board, the magnetic detection assembly comprising a plurality of magnetic sensors;
A plurality of the magnetic sensors are uniformly mounted on the circuit board.

41. The field emitter according to claim 39 or 40, the field emitter further comprising: a mounting housing;
The field emission unit group and the magnetic detection assembly are both mounted inside the mounting housing;
Alternatively, the field emission unit group is mounted inside the mounting housing, and the magnetic detection assembly is mounted outside the mounting housing.

42. The field emitter according to any one of claims 39-41, the field emitter further comprising: a magnetic source assembly;
When the magnetic source assembly is operating, the magnetic detection assembly is further used for detecting a detection magnetic field generated by the magnetic source assembly.

43. The field emitter according to claim 42, the magnetic source assembly comprising a plurality of coils, the plurality of coils being uniformly distributed on the periphery of the field emission unit group.

44. A self-calibration method for a field emitter, the self-calibration method being applied to the field emitter according to any one of claims 39-43; the self-calibration method comprising:
Obtaining measured calibration magnetic field data obtained by the magnetic detection assembly detecting a target calibration magnetic field, wherein the target calibration magnetic field is a calibration magnetic field generated by a target rotating magnet unit, and the target rotating magnet unit is a rotating magnet unit to be calibrated in the field emission unit group;
Obtaining magnet angle information of the target rotating magnet unit;
Determining target calibration parameters of the target rotating magnet unit based on the measured calibration magnetic field data and the magnet angle information.

45. The self-calibration method for a field emitter according to claim 44, the determining target calibration parameters of the target rotating magnet unit based on the measured working magnetic field data and the magnet angle information comprises:
Determining model calibration magnetic field data of the target calibration magnetic field at the magnetic detection assembly based on the magnet angle information, model parameters of the target rotating magnet unit, and the spatial pose of the magnetic detection assembly;
Optimizing the model parameters of the target rotating magnet unit with the optimization objective of minimizing the difference between the measured calibration magnetic field data and the model calibration magnetic field data, to obtain the target calibration parameters of the target rotating magnet unit.

46. The self-calibration method for a field emitter according to claim 45, the minimizing the difference between the measured working magnetic field data and the model calibration magnetic field data as the optimization objective comprises:
Taking minimizing the difference between the modulus value of the measured calibration magnetic field data and the modulus value of the model calibration magnetic field data as the optimization objective.

47. The self-calibration method for a field emitter according to claim 46, the measured calibration magnetic field data comprises a measured calibration magnetic field value sequence, and the model calibration magnetic field data comprises a model calibration magnetic field value sequence;
The minimizing the difference between the measured calibration magnetic field data and the model calibration magnetic field data as the optimization objective comprises:
Determining a sequence mean of the measured calibration magnetic field value sequence, and a sequence mean of the model calibration magnetic field value sequence;
Subtracting the sequence mean of the measured calibration magnetic field value sequence from each magnetic field value in the measured calibration magnetic field value sequence to obtain a first magnetic field value sequence;
Subtracting the sequence mean of the model calibration magnetic field value sequence from each magnetic field value in the model calibration magnetic field value sequence to obtain a second magnetic field value sequence;
Taking minimizing the difference between the first magnetic field value sequence and the second magnetic field value sequence as the optimization objective.

48. A self-calibration system for a field emitter, the system being applied to the field emitter according to any one of claims 44-47; the self-calibration system comprises:
A magnetic field data acquisition module, configured to obtain measured calibration magnetic field data obtained by the magnetic detection assembly detecting a target calibration magnetic field, wherein the target calibration magnetic field is a calibration magnetic field generated by a target rotating magnet unit, and the target rotating magnet unit is a rotating magnet unit to be calibrated in the field emission unit group;
An angle information acquisition module, configured to obtain magnet angle information of the target rotating magnet unit;
A target calibration parameter determination module, configured to determine target calibration parameters of the target rotating magnet unit based on the measured calibration magnetic field data and the magnet angle information.

49. A self-calibration device for a field emitter, comprising a processor, the processor being configured to execute the self-calibration method for a field emitter according to any one of claims 44-47.

50. A computer-readable storage medium, the storage medium storing computer instructions, when a computer reads the computer instructions in the storage medium, the computer executes the self-calibration method for a field emitter according to any one of claims 44-47.

51. A field emitter, the field emitter comprising: a field emission unit group and a magnetic source assembly;
The field emission unit group comprises at least one rotating magnet unit;
When the field emission unit group stops working, the magnetic source assembly generates a detection magnetic field for performing interference detection on the field emitter.

52. The field emitter according to claim 51, the magnetic source assembly comprising a plurality of coils, the plurality of coils being uniformly distributed on the periphery of the field emission unit group.

53. An interference detection method for a field emitter, the interference detection method being applied to the field emitter according to any one of claims 42-43 or 51-52; the interference detection method comprises:
Controlling all rotating magnet units in the field emitter to stop rotating;
Controlling the magnetic source assembly to generate the detection magnetic field;
Acquiring measured detection magnetic field data obtained by the magnetic detection assembly detecting the detection magnetic field;
Determining a detection difference between the measured detection magnetic field data and reference detection magnetic field data;
Determining an interference detection result of the field emitter according to the detection difference.

54. The interference detection method for a field emitter according to claim 53, wherein the magnetic source assembly comprises a plurality of coils, the plurality of coils being uniformly distributed on the periphery of the field emission unit group;
The step of acquiring the measured detection magnetic field data obtained by the magnetic sensor detecting the detection magnetic field comprises:
Acquiring target measured detection magnetic field data obtained by the magnetic detection assembly detecting a target detection magnetic field; wherein the target detection magnetic field is a detection magnetic field generated by a target coil in the magnetic source assembly;
The step of determining the detection difference between the measured detection magnetic field data and the reference detection magnetic field data, and determining the interference detection result of the field emitter according to the detection difference comprises:
Determining a target detection difference between the target measured detection magnetic field data and target reference detection magnetic field data, and determining an interference detection result of the field emitter in a direction corresponding to the target coil according to the target detection difference.

55. An interference detection system for a field emitter, applied to the field emitter according to any one of claims 42-43 or 51-52; the interference detection system comprises:
a first control module, configured to control all rotating magnet units in the field emitter to stop rotating;
a second control module, configured to control the magnetic source assembly to generate the detection magnetic field;
a first acquisition module, configured to acquire measured detection magnetic field data obtained by the magnetic detection assembly detecting the detection magnetic field;
a second acquisition module, configured to determine a detection difference between the measured detection magnetic field data and reference detection magnetic field data;
a first determination module, configured to determine an interference detection result of the field emitter according to the detection difference.

56. An interference detection device for a field emitter, comprising a processor configured to execute the interference detection method for a field emitter according to any one of claims 53-54.

57. A computer-readable storage medium, storing computer instructions, wherein when a computer reads the computer instructions in the storage medium, the computer executes the interference detection method for a field emitter according to any one of claims 53-54.

58. An anomaly detection method for a field emitter, applied to the field emitter according to any one of claims 39-43; the anomaly detection method comprises:
Acquiring measured working magnetic field data obtained by the magnetic detection assembly detecting a working magnetic field generated by the field emitter;
Determining a working difference between the measured working magnetic field data and reference working magnetic field data, and determining an operating state of the field emitter according to the working difference.

59. The anomaly detection method for a field emitter according to claim 58, wherein the field emitter further comprises: a magnetic source assembly;
The step of determining the operating state of the field emitter according to the working difference comprises:
In response to determining that the working difference is less than or equal to a working difference threshold, determining that the field emitter is in a normal operating state;
In response to determining that the working difference is greater than the working difference threshold,
controlling all rotating magnet units in the field emitter to stop rotating;
controlling the magnetic source assembly to generate a detection magnetic field;
acquiring measured detection magnetic field data obtained by the magnetic detection assembly detecting the detection magnetic field;
determining a detection difference between the measured detection magnetic field data and reference detection magnetic field data;
determining an interference detection result of the field emitter according to the detection difference.

60. The anomaly detection method for a field emitter according to claim 59, further comprising:
After performing the interference detection on the field emitter,
in response to determining that the detection difference is greater than a detection difference threshold, determining that the field emitter is in an abnormal operating state;
in response to determining that the detection difference is less than or equal to the detection difference threshold,
acquiring measured calibration magnetic field data obtained by the magnetic detection assembly detecting a target calibration magnetic field, wherein the target calibration magnetic field is a calibration magnetic field generated by a target rotating magnet unit, and the target rotating magnet unit is a rotating magnet unit to be calibrated in the field emission unit group;
acquiring magnet angle information of the target rotating magnet unit;
determining a target calibration parameter of the target rotating magnet unit according to the measured calibration magnetic field data and the magnet angle information.

61. The anomaly detection method for a field emitter according to claim 60, the method further comprising:
After performing the self-calibration on the field emitter,
determining a calibration difference between the target calibration parameter and an initial calibration parameter;
in response to determining that the calibration difference is greater than a calibration difference threshold, determining that the field emitter is in an abnormal operating state;
in response to determining that the calibration difference is less than or equal to the calibration difference threshold,
determining that the field emitter is in a normal operating state;
updating a target parameter of the field emitter to the target calibration parameter.

62. An electromagnetic navigation system, comprising a processor, a receiving device, and the field emitter according to any one of claims 39-43, wherein the receiving device is configured to detect a time-varying magnetic field generated by the field emitter, and the processor is configured to control the operation of the field emitter and determine a real-time pose of the receiving device in the time-varying magnetic field according to magnetic field detection data of the receiving device.

63. An anomaly detection system for a field emitter, applied to the field emitter according to any one of claims 58-61; the anomaly detection system comprises:
a second magnetic field data acquisition module, configured to acquire measured working magnetic field data obtained by the magnetic detection assembly detecting a working magnetic field generated by the field emitter;
an operating state determination module, configured to determine a working difference between the measured working magnetic field data and reference working magnetic field data, and determine an operating state of the field emitter according to the working difference.

64. An anomaly detection device for a field emitter, comprising a processor configured to execute the anomaly detection method for a field emitter according to any one of claims 58-61.

65. A computer-readable storage medium, storing computer instructions, wherein when a computer reads the computer instructions in the storage medium, the computer executes the anomaly detection method for a field emitter according to any one of claims 58-61.

66. A field emitter, comprising at least one rotating magnet unit, for each of the rotating magnet units, a reference axis is defined in the rotating magnet unit, the rotating magnet unit comprises a magnet capable of rotating about the reference axis, and a magnetic moment direction of the magnet is not parallel to the reference axis.

67. The field emitter according to claim 66, wherein at least one of the rotating magnet units comprises a self-check assembly for detecting a magnetic field signal generated during the rotation of the magnet.

68. The field emitter according to any one of claims 9, 39, 51, 66 or 67, wherein the field emitter comprises a plurality of rotating magnet units, the plurality of rotating magnet units comprising a first rotating magnet unit and a second rotating magnet unit;
the first rotating magnet unit comprises a first motor and a first magnet, the second rotating magnet unit comprises a second motor and a second magnet, the first motor is used to drive the first magnet to rotate, and the second motor is used to drive the second magnet to rotate; or,
the first magnet unit comprises a first motor and a first magnet, the second magnet unit comprises a first transmission assembly and a second magnet, the first motor is used to drive the first magnet to rotate, and the first motor is also used to drive the second magnet to rotate by driving the first transmission assembly.

69. The field emitter according to any one of claims 66-68, wherein for each of the rotating magnet units, a reference axis is defined in the rotating magnet unit, the rotating magnet unit comprises a magnet capable of rotating about the reference axis, and a magnetic moment direction of the magnet is perpendicular to the reference axis;
wherein, when the field emitter comprises a plurality of rotating magnet units:
the reference axes of at least two of the plurality of rotating magnet units are perpendicular to each other; or,
the number of the plurality of rotating magnet units is four, the reference axes of the four rotating magnet units are in a same plane, and the reference axes of any two adjacent rotating magnet units among the four rotating magnet units are perpendicular to each other; or,
the number of the plurality of rotating magnet units is three, and the reference axes of the three rotating magnet units are pairwise perpendicular.

70. The field emitter according to any one of claims 66-69, wherein the magnets are installed at ends of any two of the plurality of rotating magnet units that are far away from each other.

71. The field emitter according to any one of claims 66-70, further comprising a mounting body, the mounting body being provided with a plurality of mounting positions, the plurality of mounting positions being used for mounting the at least one rotating magnet unit.
